# EUROPEAN PATENT APPLICATION

(11) **EP 2 666 480 A2**
(43) Date of publication of application: **27.11.2013**
(21) Application number: 13173241.4
(22) Date of filing: 10.04.2007
(51) Int. Cl.: A61K 39/395, C07K 16/24

(54) **Uses and compositions for treatment of Crohn's desease**

(30) Priority: 10.04.2006 US 790909 P; 22.05.2006 US 802616 P; 22.05.2006 US 802858 P; 30.05.2006 US 809770 P; 20.06.2006 US 815489 P; 06.10.2006 US 849967 P; 01.03.2007 US 904626 P; 01.03.2007 US 904637 P; 14.03.2007 US 918174 P
(62) Divisional of application: 07755288.3
(71) Applicant: Abbott Biotechnology Ltd, HM 11 Hamilton (BM)
(72) Inventor: Pollack, Paul F., West Orange, NJ New Jersey 07052 (US); Hoffman, Rebecca, S., Wilmette, IL Illinois 60091 (US); Renz, Cheryl, Kildeer, IL Illinois 60047 (US)
(74) Representative: Modiano, Micaela Nadia

(57) **Abstract**

The invention provides methods, uses and compositions for the treatment of Crohn's disease. The invention describes methods and uses for treating Crohn's disease, wherein a TNFcc inhibitor, such as a human TNF oc antibody, or antigen-binding portion thereof, is used to induce and maintain remission of Crohn's disease in a subject. Also described are methods for determining the efficacy of a TNFa inhibitor for treatment of Crohn's disease in a subject.

SEQUENCE LISTING

<110> Abbott Biotechnology Ltd.

<120> USES AND COMPOSITIONS FOR TREATMENT OF CROHN\222S DISEASE

<130> 117813/28321

<140> EP07755288.3
<141> 2008-11-10

<150> 60/790909
<151> 2006-04-10

<150> 60/809770
<151> 2006-05-30

<150> 60/815489
<151> 2006-06-20

<150> 60/802858
<151> 2006-05-22

<150> 60/904637
<151> 2007-03-01

<150> 60/802616
<151> 2006-05-22

<150> 60/849967
<151> 2006-10-06

<150> 60/904626
<151> 2007-03-01

<150> 60/918174
<151> 2007-03-14

<160> 37

<170> FastSEQ for Windows Version 4.0

<210> 1
<211> 107
<212> PRT
<213> Artificial Sequence

<220>
<223> adalimumab light chain variable region

<400> 1

<210> 2
<211> 121
<212> PRT
<213> Artificial Sequence

<220>
<223> adalimumab heavy chain variable region

<400> 2

<210> 3
<211> 9
<212> PRT
<213> Artificial Sequence

<220>
<223> adalimumab light chain variable region CDR3

<221> VARIANT
<222> 9
<223> Xaa = Thr or Ala

<400> 3
Gln Arg Tyr Asn Arg Ala Pro Tyr Xaa
 1               5

<210> 4
<211> 12
<212> PRT
<213> Artificial Sequence

<220>
<223> adalimumab heavy chain variable region CDR3

<221> VARIANT
<222> 12
<223> Xaa = Tyr or Asn

<400> 4
Val Ser Tyr Leu Ser Thr Ala Ser Ser Leu Asp Xaa
 1               5                  10

<210> 5
<211> 7

<212> PRT
<213> Artificial Sequence

<220>
<223> adalimumab light chain variable region CDR2

<400> 5
Ala Ala Ser Thr Leu Gln Ser
1               5

<210> 6
<211> 17
<212> PRT
<213> Artificial Sequence

<220>
<223> adalimumab heavy chain variable region CDR2

<400> 6
Ala Ile Thr Trp Asn Ser Gly His Ile Asp Tyr Ala Asp Ser Val Glu
1               5                   10                  15
Gly

<210> 7
<211> 11
<212> PRT
<213> Artificial Sequence

<220>
<223> adalimumab light chain variable region CDR1

<400> 7
Arg Ala Ser Gln Gly Ile Arg Asn Tyr Leu Ala
1               5                   10

<210> 8
<211> 5
<212> PRT
<213> Artificial Sequence

<220>
<223> adalimumab heavy chain variable region CDR1

<400> 8
Asp Tyr Ala Met His
1               5

<210> 9
<211> 107
<212> PRT
<213> Artificial Sequence

<220>
<223> 2SD4 light chain variable region

<400> 9
Asp Ile Gln Met Thr Gln Ser Pro Ser Ser Leu Ser Ala Ser Ile Gly
1               5                   10                  15
Asp Arg Val Thr Ile Thr Cys Arg Ala Ser Gln Gly Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Thr Leu Gln Ser Gly Val Pro Ser Arg Phe Ser Gly

50                  55                  60
Ser Gly Ser Gly Thr Asp Phe Thr Leu Thr Ile Ser Ser Leu Gln Pro
65                  70                  75                  80

Glu Asp Val Ala Thr Tyr Tyr Cys Gln Lys Tyr Asn Ser Ala Pro Tyr
                85                  90                  95
Ala Phe Gly Gln Gly Thr Lys Val Glu Ile Lys
            100                 105

<210> 10
<211> 121
<212> PRT
<213> Artificial Sequence

<220>
<223> 2SD4 heavy chain variable region

<400> 10
Gln Val Gln Leu Val Glu Ser Gly Gly Gly Leu Val Gln Pro Gly Arg
 1               5                  10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Asp Trp Val
        35                  40                  45
Ser Ala Ile Thr Trp Asn Ser Gly His Ile Asp Tyr Ala Asp Ser Val
    50                  55                  60
Glu Gly Arg Phe Ala Val Ser Arg Asp Asn Ala Lys Asn Ala Leu Tyr
65                  70                  75                  80
Leu Gln Met Asn Ser Leu Arg Pro Glu Asp Thr Ala Val Tyr Tyr Cys
                85                  90                  95
Thr Lys Ala Ser Tyr Leu Ser Thr Ser Ser Ser Leu Asp Asn Trp Gly
            100                 105                 110
Gln Gly Thr Leu Val Thr Val Ser Ser
        115                 120

<210> 11
<211> 9
<212> PRT
<213> Artificial Sequence

<220>
<223> 2SD4 light chain variable region CDR3

<400> 11
Gln Lys Tyr Asn Ser Ala Pro Tyr Ala
 1               5

<210> 12
<211> 9
<212> PRT
<213> Artificial Sequence

<220>
<223> EP B12  light chain variable region CDR3

<400> 12
Gln Lys Tyr Asn Arg Ala Pro Tyr Ala
 1               5

<210> 13
<211> 9
<212> PRT
<213> Artificial Sequence

<220>
<223> VL10E4 light chain variable region CDR3

<400> 13
Gln Lys Tyr Gln Arg Ala Pro Tyr Thr
 1               5

<210> 14
<211> 9
<212> PRT
<213> Artificial Sequence

<220>
<223> VL100A9 light chain variable region CDR3

<400> 14
Gln Lys Tyr Ser Ser Ala Pro Tyr Thr
 1               5

<210> 15
<211> 9
<212> PRT
<213> Artificial Sequence

<220>
<223> VLL100D2 light chain variable region CDR3

<400> 15

Gln Lys Tyr Asn Ser Ala Pro Tyr Thr
 1               5

<210> 16
<211> 9
<212> PRT
<213> Artificial Sequence

<220>
<223> VLL0F4 light chain variable region CDR3

<400> 16
Gln Lys Tyr Asn Arg Ala Pro Tyr Thr
 1               5

<210> 17
<211> 9
<212> PRT
<213> Artificial Sequence

<220>
<223> LOE5 light chain variable region CDR3



<400> 17
Gln Lys Tyr Asn Ser Ala Pro Tyr Tyr
 1               5

<210> 18
<211> 9
<212> PRT
<213> Artificial Sequence

<220>
<223> VLLOG7 light chain variable region CDR3

<400> 18
Gln Lys Tyr Asn Ser Ala Pro Tyr Asn
1               5

<210> 19
<211> 9
<212> PRT
<213> Artificial Sequence

<220>
<223> VLLOG9 light chain variable region CDR3

<400> 19
Gln Lys Tyr Thr Ser Ala Pro Tyr Thr
1               5

<210> 20
<211> 9
<212> PRT
<213> Artificial Sequence

<220>
<223> VLLOH1 light chain variable region CDR3

<400> 20
Gln Lys Tyr Asn Arg Ala Pro Tyr Asn
1               5

<210> 21
<211> 9
<212> PRT
<213> Artificial Sequence

<220>
<223> VLLOH10 light chain variable region CDR3

<400> 21
Gln Lys Tyr Asn Ser Ala Ala Tyr Ser
1               5

<210> 22
<211> 9
<212> PRT
<213> Artificial Sequence

<220>
<223> VL1B7 light chain variable region CDR3

<400> 22
Gln Gln Tyr Asn Ser Ala Pro Asp Thr
1               5

<210> 23
<211> 9
<212> PRT
<213> Artificial Sequence

<220>
<223> VL1C1 light chain variable region CDR3

<400> 23
Gln Lys Tyr Asn Ser Asp Pro Tyr Thr
1               5

<210> 24
<211> 9
<212> PRT
<213> Artificial Sequence

<220>
<223> VL0.1F4 light chain variable region CDR3

<400> 24
Gln Lys Tyr Ile Ser Ala Pro Tyr Thr
1               5

<210> 25
<211> 9
<212> PRT
<213> Artificial Sequence

<220>
<223> VL0.1H8 light chain variable region CDR3

<400> 25
Gln Lys Tyr Asn Arg Pro Pro Tyr Thr
1               5

<210> 26
<211> 9
<212> PRT
<213> Artificial Sequence

<220>
<223> LOE7.A light chain variable region CDR3

<400> 26
Gln Arg Tyr Asn Arg Ala Pro Tyr Ala
1               5

<210> 27
<211> 12
<212> PRT
<213> Artificial Sequence

<220>
<223> 2SD4 heavy chain variable region CDR3

<400> 27
Ala Ser Tyr Leu Ser Thr Ser Ser Ser Leu Asp Asn
1               5                   10

<210> 28
<211> 12
<212> PRT
<213> Artificial Sequence

<220>
<223> VH1B11 heavy chain variable region CDR3

<400> 28
Ala Ser Tyr Leu Ser Thr Ser Ser Ser Leu Asp Lys

1                   5                   10

<210> 29
<211> 12
<212> PRT
<213> Artificial Sequence

<220>
<223> VH1D8 heavy chain variable region CDR3

<400> 29
Ala Ser Tyr Leu Ser Thr Ser Ser Ser Leu Asp Tyr
1                   5                   10

<210> 30
<211> 12
<212> PRT
<213> Artificial Sequence

<220>
<223> VH1A11 heavy chain variable region CDR3

<400> 30
Ala Ser Tyr Leu Ser Thr Ser Ser Ser Leu Asp Asp
1                   5                   10

<210> 31
<211> 12
<212> PRT
<213> Artificial Sequence


<220>
<223> VH1B12 heavy chain variable region CDR3

<400> 31
Ala Ser Tyr Leu Ser Thr Ser Phe Ser Leu Asp Tyr
1                   5                   10

<210> 32
<211> 12
<212> PRT
<213> Artificial Sequence

<220>
<223> VH1E4 heavy chain variable region CDR3

<400> 32
Ala Ser Tyr Leu Ser Thr Ser Ser Ser Leu His Tyr
1                   5                   10

<210> 33
<211> 12
<212> PRT
<213> Artificial Sequence

<220>
<223> VH1F6 heavy chain variable region CDR3

<400> 33
Ala Ser Phe Leu Ser Thr Ser Ser Ser Leu Glu Tyr
1                   5                   10

<210> 34
<211> 12
<212> PRT
<213> Artificial Sequence

<220>
<223> 3C-H2 heavy chain variable region CDR3

<400> 34
Ala Ser Tyr Leu Ser Thr Ala Ser Ser Leu Glu Tyr
1               5                   10

<210> 35
<211> 12
<212> PRT
<213> Artificial Sequence

<220>
<223> VH1-D2.N heavy chain variable region CDR3

<400> 35
Val Ser Tyr Leu Ser Thr Ala Ser Ser Leu Asp Asn
1               5                   10

<210> 36
<211> 321
<212> DNA
<213> Artificial Sequence

<220>
<223> adalimumab light chain variable region

<400> 36
gacatccaga tgacccagtc tccatcctcc ctgtctgcat ctgtagggga cagagtcacc 60
atcacttgtc gggcaagtca gggcatcaga aattacttag cctggtatca gcaaaaacca 120
gggaaagccc ctaagctcct gatctatgct gcatccactt tgcaatcagg ggtcccatct 180
cggttcagtg gcagtggatc tgggacagat ttcactctca ccatcagcag cctacagcct 240
gaagatgttg caacttatta ctgtcaaagg tataaccgtg caccgtatac ttttggccag 300
gggaccaagg tggaaatcaa a                                           321

<210> 37
<211> 363
<212> DNA
<213> Artificial Sequence

<220>
<223> adalimumab heavy chain variable region

<400> 37
gaggtgcagc tggtggagtc tgggggaggc ttggtacagc ccggcaggtc cctgagactc 60
tcctgtgcgg cctctggatt cacctttgat gattatgcca tgcactgggt ccggcaagct 120
ccagggaagg gcctggaatg ggtctcagct atcacttgga atagtggtca catagactat 180
gcggactctg tggagggccg attcaccatc tccagagaca acgccaagaa ctccctgtat 240
ctgcaaatga acagtctgag agctgaggat acggccgtat attactgtgc gaaagtctcg 300
taccttagca ccgcgtcctc ccttgactat tggggccaag gtaccctggt caccgtctcg 360
agt                                                               363

## Description

### RELATED APPLICATIONS

This application claims the benefit of priority to U.S. provisional patent application number 60/790909 filed on April 10, 2006; U.S. provisional patent application number 60/809770 filed on May 30, 2006; U.S. provisional patent application number 60/815489 filed on June 20, 2006; U.S. provisional patent application number 60/802858 filed on May 22, 2006; U.S. provisional patent application number 60/904637 filed on March 1, 2007; U.S. provisional patent application number 60/802616 filed on May 22, 2006; U.S. provisional patent application number 60/849967 filed on October 6, 2006; U.S. provisional patent application number 60/904626 filed on March 1, 2007; and U.S. provisional patent application number 60/918174 filed on March 14, 2007. The contents of all the above-mentioned priority applications are hereby incorporated by reference in their entirety.

### BACKGROUND OF THE INVENTION

Crohn's disease is a T-helper Type 1 (Th 1) disease, which has a characteristic immune response pattern that includes an increased production of interleukin-12, tumour necrosis factor (TNF), and interferon γ (Romagnani. Inflamm Bowel Dis 1999;5:285-94). Increased production of TNF by macrophages in patients with Crohn's disease (CD) results in elevated concentrations of TNF in the stool, blood, and mucosa (Murch et al. Gut 1991;32:913-7; Braegger et al. Lancet 1992;339:89-91; Murch et al. Gut 1993;34:1705-9). Tumor necrosis factor (TNF) is an important cytokine in the pathogenesis of Crohn's disease (CD), with elevated concentrations playing a role in pathologic inflammation (Papadakis et al. Gastroenterology 2000;119:1148-1157; Van Deventer Gut 1997;40:443-448). There is no cure for Crohn's disease, and long-term, effective treatment options are limited. For patients afflicted with Crohn's disease, the disease can have a devastating impact on their lifestyle, as common symptoms of Crohn's disease include diarrhea, cramping, abdominal pain, fever, and even rectal bleeding. Crohn's disease and complications associated with it often results in the patient requiring surgery, often more than once.

### SUMMARY OF THE INVENTION

There remains a need for an effective and safe treatment option for patients suffering from Crohn's disease and Crohn's related disorders. The instant invention provides improved methods and compositions for treating Crohn's disease, including methods of inducing remission of Crohn's disease and maintaining remission. The invention further provides a means for treating certain subpopulations of patients who have Crohn's disease, including patients who have failed therapy or lost responsiveness to treatment with TNFα inhibitors. The invention further provides a means by which the efficacy of a TNFα inhibitor for the treatment of Crohn's disease can be determined, The invention also includes methods for treating certain types of Crohn's disease, e.g., early Crohn's disease, and Crohn's-related disorders, such as complications associated with Crohn's disease such as fistulas. Kits and labels which provide information pertaining to the methods, uses, and compositions of the invention are also described herein. Each of the examples described herein describes methods which can be used to determine whether a TNFα inhibitor is effective for treating the given disorder.

The invention provides for the use of a TNFα inhibitor in the manufacture of a medicament for the treatment of early Crohn's disease in a subject who has early Crohn's disease. The invention also provides a method of treating early Crohn's disease in a subject comprising administering to the subject a TNFα inhibitor, such that early Crohn's disease is treated. In one embodiment, the subject has had Crohn's disease for less than 2 years.

The invention also provides for a method of inducing and maintaining remission of Crohn's disease in a subject comprising administering an initial loading dose of a TNFα inhibitor to the subject at week 0, administering a second dose of the TNFα inhibitor to the subject, wherein the second dose is about half the dose amount of the loading dose, and administering at least one a maintenance dose to the subject, wherein the maintenance dose is about half the dose amount of the second dose, such that remission of Crohn's disease is induced and maintained. In one embodiment, the initial the initial dose is given in its entirety on one day or is divided over 2 days. In a further embodiment, the the second dose is administered to the subject about two weeks after the first dose. In another embodiment, the maintenance dose is administered to the subject about two weeks after the second dose. In one embodiment, the maintenance dose is administered on a biweekly dosing regimen.

The invention also includes a method of determining the efficacy of a TNFα inhibitor for maintaining remission of Crohn's disease in a subject comprising determining a Crohn's Disease Activity Index (CDAI) score of a patient population having Crohn's disease and who was administered the TNFα inhibitor, wherein a CDAI score of less than 150 maintained in at least about 49% of the patient population indicates that the TNFα inhibitor is an effective TNFα inhibitor for the treatment of Crohn's disease in a subject. In one embodiment, the method further comprising administering the effective TNFα inhibitor to a subject to maintain remission of Crohn's disease, In another embodiment, a CDAI score of less than 150 maintained in at least about 50% of the patient population indicates that the TNFα inhibitor is an effective TNFα inhibitor for the treatment of Crohn's disease in a subject. In another further embodiment, a CDAI score of less than 150 maintained in at least about 60% of the patient population indicates that the TNFα inhibitor is an effective TNFα inhibitor for the treatment of Crohn's disease in a subject. In another further embodiment, a CDAI score of less than 150 maintained in at least about 70% of the patient population indicates that the TNFα inhibitor is an effective TNFα inhibitor for the treatment of Crohn's disease in a subject. In another further embodiment, a CDAI score of less than 150 maintained in at least about 80% of the patient population indicates that the TNFα inhibitor is an effective TNFα inhibitor for the treatment of Crohn's disease in a subject. In another further embodiment, a CDAI score of less than 150 maintained in at least about 90% of the patient population indicates that the TNFα inhibitor is an effective TNFα inhibitor for the treatment of Crohn's disease in a subject. In another further embodiment, a CDAI score of less than 150 maintained in at least about 94% of the patient population indicates that the TNFα inhibitor is an effective TNFα inhibitor for the treatment of Crohn's disease in a subject.

The invention also provides for a method of maintaining remission of Crohn's disease in a subject comprising administering an effective TNFα inhibitor to the subject such that remission of Crohn's disease is maintained, wherein the effective TNFα inhibitor was previously identified as maintaining a CDAI score of less than 150 in at least about 49% ofa patient population having Crohn's disease. In one embodiment, the effective TNFα inhibitor was previously identified as maintaining a CDAI score of less than 150 in at least about 50% of a patient population having Crohn's disease. In another embodiment, the effective TNFα inhibitor was previously identified as maintaining a CDAI score of less than 150 in at least about 60% of a patient population having Crohn's disease. In another embodiment, the effective TNFα inhibitor was previously identified as maintaining a CDAI score of less than 150 in at least about 70% of a patient population having Crohn's disease. In another embodiment, the effective TNFα inhibitor was previously identified as maintaining a CDAI score of less than 150 in at least about 80% of a patient population having Crohn's disease. In another embodiment, the effective TNFα inhibitor was previously identified as maintaining a CDAI score of less than 150 in at least about 90% of a patient population having Crohn's disease. In another embodiment, the effective TNFα inhibitor was previously identified as maintaining a CDAI score of less than 150 in at least about 94% of a patient population having Crohn's disease.

The invention also includes a method of determining the efficacy of a TNFα inhibitor for achieving a clinical response in Crohn's disease in a subject comprising determining a Crohn's Disease Activity Index (CDAI) score of a patient population having Crohn's disease and who was administered the TNFα inhibitor, wherein a decrease of at least 100 in the CDAI score of at least about 47% of the patient population indicates that the TNFα inhibitor is an effective TNFα inhibitor for achieving a clinical response in Crohn's disease in a subject. In one embodiment, the invention further comprises administering the effective TNFα inhibitor to a subject to achieve a clinical response in Crohn's disease. In one embodiment, a decrease of at least 100 in the CDAI score of at least about 50% of the patient population indicates that the TNFα inhibitor is an effective TNFα inhibitor for achieving a clinical response in Crohn's disease in a subject. In another embodiment, a decrease of at least 100 in the CDAI score of at least about 60% of the patient population indicates that the TNFα inhibitor is an effective TNFα inhibitor for achieving a clinical response in Crohn's disease in a subject. In another embodiment, a decrease of at least 100 in the CDAI score of at least about 70% of the patient population indicates that the TNFα inhibitor is an effective TNFα inhibitor for achieving a clinical response in Crohn's disease in a subject. In another embodiment, a decrease of at least 100 in the CDAI score of at least about 80% of the patient population indicates that the TNFα inhibitor is an effective TNFα inhibitor for achieving a clinical response in Crohn's disease in a subject. In another embodiment, a decrease of at least 100 in the CDAI score of at least about 90% of the patient population indicates that the TNFα inhibitor is an effective TNFα inhibitor for achieving a clinical response in Crohn's disease in a subject.

The invention further provides a method of achieving a clinical response in Crohn's disease in a subject comprising administering an effective TNFα inhibitor to the subject such that a clinical response in Crohn's disease is achieved, wherein the effective TNFα inhibitor was previously identified as decreasing a CDAI score by at least 100 in at least about 47% of a patient population having Crohn's disease. In one embodiment, the effective TNFα inhibitor was previously identified as decreasing a CDAI score by at least 100 in at least about 50% of a patient population having Crohn's disease. In another embodiment, the effective TNFα inhibitor was previously identified as decreasing a CDAI score by at least 100 in at least about 60% of a patient population having Crohn's disease. In another embodiment, the effective TNFα inhibitor was previously identified as decreasing a CDAI score by at least 100 in at least about 70% of a patient population having Crohn's disease. In another embodiment, the effective TNFα inhibitor was previously identified as decreasing a CDAI score by at least 100 in at least about 80% of a patient population having Crohn's disease. In another embodiment, the effective TNFα inhibitor was previously identified as decreasing a CDAI score by at least 100 in at least about 90% of a patient population having Crohn's disease.

The invention also pertains to a method of determining the efficacy of a human TNFα antibody, or antigen-binding portion thereof, for achieving a clinical response in Crohn's disease in a subject comprising determining a Crohn's Disease Activity Index (CDAI) score of a patient population having Crohn's disease and who was administered the human TNFα antibody, or antigen-binding portion thereof, wherein a decrease of at least 70 in the CDAI score of at least about 43% of the patient population indicates that the human TNFα antibody, or antigen-binding portion thereof, is an effective human TNFα antibody, or antigen-binding portion thereof, for achieving a clinical response in Crohn's disease in a subject. In one embodiment, the method further comprises administering the effective human TNFα antibody, or antigen-binding portion thereof, to a subject. In one embodiment, a decrease of at least 70 in the CDAI score of at least about 50% of the patient population indicates that the human TNFα antibody, or antigen-binding portion thereof, is an effective human TNFα antibody, or antigen-binding portion thereof, for achieving a clinical response in Crohn's disease in a subject. In another embodiment, a decrease of at least 70 in the CDAI score of at least about 60% of the patient population indicates that the human TNFα antibody, or antigen-binding portion thereof, is an effective human TNFα antibody, or antigen-binding portion thereof, for achieving a clinical response in Crohn's disease in a subject. In another embodiment, wherein a decrease of at least 70 in the CDAI score of at least about 70% of the patient population indicates that the human TNFα antibody, or antigen-binding portion thereof, is an effective human TNFα antibody, or antigen-binding portion thereof, for achieving a clinical response in Crohn's disease in a subject. In another embodiment, a decrease of at least 70 in the CDAI score of at least about 80% of the patient population indicates that the human TNFα antibody, or antigen-binding portion thereof, is an effective human TNFα antibody, or antigen-binding portion thereof, for achieving a clinical response in Crohn's disease in a subject. In another embodiment, a decrease of at least 70 in the CDAI score of at least about 90% of the patient population indicates that the human TNFα antibody, or antigen-binding portion thereof, is an effective human TNFα antibody, or antigen-binding portion thereof, for achieving a clinical response in Crohn's disease in a subject.

The invention also pertains to a method of determining the efficacy of a TNFα inhibitor to maintain remission of Crohn's disease in a subject comprising determining an Inflammatory Bowel Disease Questionnaire (IBDQ) score of a patient population having Crohn's disease who was administered the TNFα inhibitor, wherein an IBDQ score greater than 170 in at least about 74% of the patient population indicates that the TNFα inhibitor is an effective TNFα inhibitor for maintaining remission of Crohn's disease in a subject. In one embodiment, the method further comprises administering the effective TNFα inhibitor to a subject having Crohn's disease. In another embodiment, a IBDQ score greater than 170 in at least about 80% of the patient population indicates that the TNFα inhibitor is an effective TNFα inhibitor for maintaining remission of Crohn's disease in a subject.

The invention includes a method of maintaining remission of Crohn's disease in a subject comprising administering an effective amount of a TNFα inhibitor to the subject, such that remission of Crohn's disease is maintained, wherein the effective amount of the TNFα inhibitor was previously identified as maintaining an IBDQ score greater than 170 in at least about 74% of a patient population having Crohn's disease. In one embodiment, the effective amount of the TNFα inhibitor was previously identified as maintaining an IBDQ score greater than 170 in at least about 80% of a patient population having Crohn's disease.

The invention also includes a method of maintaining remission of Crohn's disease in a subject who has achieved remission of Crohn's disease comprising administering a maintenance dose of the TNFα inhibitor to the subject, wherein the maintenance dose provides a mean serum trough level of about 7 µg/mL of the TNFα inhibitor.

The invention further provides a method of inducing and maintaining remission of Crohn's disease in a subject in need thereof comprising administering a loading dose of a TNFα inhibitor to the subject, wherein the loading dose provides a mean serum TNFα inhibitor trough level of about 12 µg/mL, and administering a maintenance dose of the TNFα inhibitor to the subject to maintain remission of Crohn's disease, wherein the maintenance dose provides a mean serum trough level of about 7 µg/mL of the TNFα inhibitor.

The invention pertains to a method of achieving a clinical response in Crohn's disease in a subject comprising administering an effective human TNFα antibody, or antigen-binding portion thereof, to the subject such that a clinical response in Crohn's disease is achieved, wherein the effective human TNFα antibody, or antigen-binding portion thereof, was previously identified as decreasing a CDAI score by at least 70 in at least about 43% of a patient population having Crohn's disease. In one embodiment, the effective human TNFα antibody, or antigen-binding portion thereof, was previously identified as decreasing a CDAI score by at least 70 in at least about 50% of a patient population having Crohn's disease. In another embodiment, the effective human TNFα antibody, or antigen-binding portion thereof, was previously identified as decreasing a CDAI score by at least 70 in at least about 60% of a patient population having Crohn's disease. In another embodiment, the effective human TNFα antibody, or antigen-binding portion thereof, was previously identified as decreasing a CDAI score by at least 70 in at least about 70% of a patient population having Crohn's disease. In another embodiment, the effective human TNFα antibody, or antigen-binding portion thereof, was previously identified as decreasing a CDAI score by at least 70 in at least about 80% of a patient population having Crohn's disease. In another embodiment, the effective human TNFα antibody, or antigen-binding portion thereof, was previously identified as decreasing a CDAI score by at least 70 in at least about 90% of a patient population having Crohn's disease.

The invention also pertains to the use of a human TNFα antibody, or antigen binding portion thereof, in the manufacture of a medicament for maintaining remission of a Crohn's-related fistula in a subject.

The invention pertains to a method of maintaining remission of a Crohn's-related fistula in a subject comprising administering a human TNFα antibody, or antigen binding portion thereof, to the subject, such that remission of the Crohn's-related fistula is maintained.

The invention pertains to the use of a human TNFα antibody, or antigen-binding portion thereof, in the manufacture of a medicament for maintaining remission of Crohn's disease in a subject who has achieved remission. In one embodiment, the medicament is for administration to the subject on a maintenance dose regimen. In a further embodiment, the remission of Crohn's disease is a CDAI of < 150.

The invention pertains to a method of maintaining remission of Crohn's disease in a subject who has achieved remission of Crohn's disease comprising administering a human TNFα antibody, or an antigen-binding portion thereof, to the subject, such that remission of Crohn's disease is maintained. In one embodiment, a Crohn's Disease Activity Index (CDAI) score of less than 150 is maintained in the subject. In another embodiment, the human TNFα antibody, or an antigen-binding portion thereof, is administered to the subject on a maintenance dose regimen. In still another embodiment, the method further comprises decreasing steroid use in the subject. In still a further embodiment, the remission of Crohn's disease is a CDAI of < 150.

The invention also pertains to a method of inducing and maintaining remission of Crohn's disease in a subject comprising administering an initial loading dose of a human TNFα antibody or antigen-binding portion thereof, to the subject at week 0, administering a second dose of the human TNFα antibody or antigen-binding portion thereof, to the subject, wherein the second dose is about half the dose amount of the loading dose, and administering at least one maintenance dose of the human TNFα antibody or antigen-binding portion thereof, to the subject, wherein the maintenance dose is about half the dose amount of the second dose, such that remission of Crohn's disease is induced and maintained. In one embodiment, the initial dose is given in its entirety on one day or is divided over 2 days. In another embodiment, the second dose is administered to the subject about two weeks after the first dose. In one embodiment, the maintenance dose is administered to the subject about two weeks after the second dose. In another embodiment, the maintenance dose is administered on a biweekly dosing regimen.

The invention includes an article of manufacture comprising a TNFα inhibitor and a package insert, wherein the package insert indicates the recommended TNFα inhibitor dose regimen for adult patients with Crohn's disease is 160 mg at week 0, 80 mg at week 2, followed by 40 mg every other week beginning at week 4, In one embodiment, the the package insert indicates that the week 0 dose can be administered as four injections in one day or as two injections per day for two consecutive days.

The invention also includes an article of manufacture comprising a TNFα inhibitor and a package insert, wherein the package insert indicates that in patients with Crohn's disease who have been administered the TNFα inhibitor, the loading dose on week 0 followed by a second dose on week 2 achieves serum adalimumab trough concentrations of approximately 12 µg/mL.

The invention includes an article of manufacture comprising a TNFα inhibitor and a package insert, wherein the package insert indicates that in patients with Crohn's disease who have been administered the TNFα inhibitor, the mean steady-state trough levels of approximately 7 µg/mL were observed in Crohn's disease patients who received a maintenance dose of the TNFα inhibitor every other week.

In one embodiment, the TNFα inhibitor is a TNFα antibody, or an antigen-binding portion thereof. In a further embodiment, wherein the TNFα antibody, or an antigen-binding portion thereof, is human. In another embodiment, the human TNFα antibody, or an antigen-binding portion thereof, dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less. In another embodiment, the human TNFα antibody, or an antigen-binding portion thereof, comprises a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and comprises a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11. In another embodiment, the human TNFα antibody, or an antigen-binding portion thereof, is adalimumab.

The invention pertains to an article of manufacture which comprising adalimumab and a package insert, wherein the package insert indicates that the adalimumab may be used to treat Crohn's disease in patients who have had an inadequate response to conventional therapy and/or who have lost response to or are intolerant to infliximab.

In one embodiment of the methods, uses and compositions of the invention, the TNFα inhibitor is a TNFα antibody, or antigen-binding portion thereof, or a TNFα fusion protein.

In one embodiment, the TNFα fusion protein is etanercept.

In another embodiment, the TNFα antibody, or antigen-binding portion thereof, is an antibody selected from the group consisting of a humanized antibody, a chimeric antibody, a human antibody, and a multivalent antibody.

In one embodiment, the TNFα antibody, or antigen-binding portion thereof, is infliximab or golimumab. In another embodiment, the human TNFα antibody, or an antigen-binding portion thereof, dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less.

In one embodiment, the human TNFα antibody, or an antigen-binding portion thereof, has the following characteristics:
a) dissociates from human TNFα with a K_{off} rate constant of 1 x 10⁻³ s⁻¹ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

In one embodiment, the human TNFα antibody, or an antigen-binding portion thereof, comprises a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and comprises a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11.

In still another embodiment, the human TNFα antibody, or an antigen-binding portion thereof, comprises a light chain variable region (LCVR) comprising the amino acid sequence of SEQ ID NO: 1 and a heavy chain variable region (HCVR) comprising the amino acid sequence of SEQ ID NO: 2.

In one embodiment, the human TNFα antibody, or an antigen-binding portion thereof, is adalimumab.

In one embodiment, the human TNFα antibody, or an antigen-binding portion thereof, was administered to the patient population on a maintenance therapy comprising a biweekly dosing regimen. In another embodiment, the human TNFα antibody, or an antigen-binding portion thereof, was administered in a dose of about 40 mg.

The invention also provides a method of monitoring the effectiveness of a TNFα inhibitor for the treatment of Crohn's disease comprising administering the TNFα inhibitor to a preselected patient population having Crohn's disease; and determining the effectiveness of the TNFα inhibitor using a mean baseline Crohn's Disease Activity Index (CDAI) score of the patient population and a mean CDAI score following administration of the TNFα inhibitor, wherein a Δ100 CDAI in at least about 60% of the patient population indicates that the TNFα inhibitor is effective for the treatment of Crohn's disease.

The invention includes a method of monitoring the effectiveness of a TNFα inhibitor for the treatment of Crohn's disease comprising administering the TNFα inhibitor to a preselected patient population having Crohn's disease; and determining the effectiveness of the TNFα inhibitor by using a mean baseline Crohn's Disease Activity Index (CDAI) score of the patient population and a mean CDAI score following administration of the TNFα inhibitor, wherein a CDAI<150 achieved in at least about 40% of the patient population indicates that the TNFα inhibitor is effective for the treatment of Crohn's disease. In one embodiment, the patient population comprises patients on concomitant immunosuppressant (IMM) treatment. In another embodiment, the patient population comprises patients not on concomitant IMM treatment. In one embodiment, the TNFα inhibitor has already been administered to the pre-selected patient population.

The invention provides a method of testing the efficacy of a TNFα inhibitor to induce and maintain remission of Crohn's disease comprising administering the TNFα inhibitor to a preselected patient population having Crohn's disease; and determining the efficacy of the TNFα inhibitor by using a mean baseline Inflammatory Bowel Disease Questionnaire (IBDQ) score of the patient population and a mean IBDQ score following administration of the TNFα inhibitor, wherein an IBDQ > 170 achieved in at least about 74% of the patient population indicates that the TNFα inhibitor is efficacious for inducing and maintaining remission of Crohn's disease. In one embodiment, the TNFα inhibitor is administered weekly. In another embodiment, the TNFα inhibitor is administered every other week. In one embodiment, the TNFα inhibitor has already been administered to the pre-selected patient population.

The invention further provides a package comprising a TNFα inhibitor and a label, *e.g.*, in a position which is visible to prospective purchasers, comprising a printed statement which informs subjects, e.g., purchasers, that the median apparent clearance (CL/F) of the TNFα inhibitor ranges from about 13.2 to about 15.0 mL/hr. In one embodiment of the invention, the statement further informs prospective purchasers that concomitant therapy with either immunosuppressant 6 mercaptopurine or azathioprine has slightly lower or no impact on TNFα inhibitor CL/F.

The invention provides a method of maintaining remission of an intestinal disorder in a subject who has achieved clinical remission of the intestinal disorder comprising administering to the subject a maintenance dose regimen of a TNFα inhibitor such that remission of the intestinal disorder is maintained. In one embodiment, the intestinal disorder is Crohn's disease, including, moderate to severe Crohn's disease. In one embodiment, prior to the maintenance dose regimen the subject achieved a clinical response comprising a CDAI decrease of at least about 70 points.

The invention also includes a method for maintaining clinical remission of Crohn's disease in a subject who has achieved clinical remission of Crohn's disease comprising administering a maintenance dose of a TNFα antibody, or an antigen-binding portion thereof, such that clinical remission of Crohn's is maintained.

The invention provides a method for decreasing steroid use and maintaining clinical remission of Crohn's disease in a subject who has achieved clinical remission of Crohn's disease comprising administering a maintenance dose of a TNFα antibody, or an antigen-binding portion thereof, such that steroid use is decreased and clinical remission of Crohn's disease is maintained. In one embodiment, the clinical remission of Crohn's disease is a CDAI decrease of at least about 70 points. In another embodiment, the clinical remission of Crohn's disease is a CDAI of < 150.

The invention also provides a method of completely healing a draining fistula in a subject comprising administering a maintenance dose of a TNFα antibody, or antigen-binding portion thereof, to the subject, such that the draining fistula is completely healed. In one embodiment, the subject has Crohn's disease.

The invention includes a method of treating Crohn's disease in a subject comprising subcutaneously administering to the subject a TNFα inhibitor wherein the apparent clearance (CL/F) of the TNFα inhibitor in the subject is about 14.9 mL/hr.

In one embodiment of the invention, the subject has been treated previously with an induction dose of the TNFα inhibitor.

The invention provides an article of manufacture comprising a packaging material; a TNFα antibody, or antigen-binding portion thereof; and a label or package insert contained within the packaging material indicating that in studies of the TNFα antibody, or antigen-binding portion thereof, for the treatment of Crohn's disease the most common adverse events (AEs) were infections.

The invention also includes an article of manufacture comprising a packaging material; a TNFα antibody, or antigen-binding portion thereof; and a label or package insert contained within the packaging material indicating that administration of the maintenance dose of the TNFα antibody, or antigen-binding portion thereof, for the treatment of Crohn's disease is about half of the induction dose. In one embodiment, the package insert further contains instructions for biweekly administration of the maintenance dose. In one embodiment, the package insert further contains instructions for weekly administration of the maintenance dose.

The invention provides a method of treating a subject having Crohn's disease who has become non-responsive or intolerant to another TNF antagonist. In one embodiment, the TNF antagonist is an anti-TNF antibody, *e.g.,* infliximab.

The invention also provides a method of inducing remission in a non-responder subject having Crohn's disease comprising administering adalimumab. In one embodiment, the invention provides a method for improving the IBDQ score of a non-responder subject comprising administering adalimumab to the subject. In one embodiment, the invention provides a method for improving the CDAI score of a non-responder subject comprising administering adalimumab to the subject. In one embodiment, adalimumab is administered to the subject in a multiple variable dose, *e.g.*, 160 mg induction dose followed by an 80 mg dose.

The invention provides compositions for use in treating Crohn's disease with a TNFα inhibitor, *e.g.*, a TNFα antibody. In particular, the invention provides a printed material, such as a label or packaging insert, which is used to inform a reader, e.g., including a prospective purchaser and/or a subject who will be administering the TNFα inhibitor for treatment, about the TNFα inhibitor, *e.g.,* a TNFα antibody such as adalimumab. The label may contain important information regarding adverse events, methods of administering, pharmacokinetic and pharmacodynamic information, clinical trial information, etc.

The invention provides a package comprising a TNFα inhibitor and a label, in a position which is visible to prospective purchasers, comprising a printed statement which informs prospective purchasers that the median apparent clearance (CL/F) of the TNFα inhibitor ranges from about 13.2 to about 15.4 mL/hr.

In one embodiment, the printed statement further informs prospective purchasers that concomitant therapy with either immunosuppressant 6 mercaptopurine or azathioprine has slightly lower or no impact on TNFα inhibitor CL/F.

In one embodiment, the label or package insert of the invention indicates that the TNFα inhibitor, e.g., TNFα antibody, *e.g.,* adalimumab, may be used to treat Crohn's disease in patients who have had an inadequate response to conventional therapy and/or who have lost response to or are intolerant to infliximab.

In a further embodiment, the label or package insert of the invention indicates that extra TNFα in your body can attack normal healthy body tissues and cause inflammation especially in the tissues in your bones, cartilage, joints and digestive tract.

In still another embodiment, the label or package insert of the invention indicates that the TNFα inhibitor helps reduce the signs and symptoms of immune diseases, including rheumatoid and psoriatic arthritis (pain and swollen joints), ankylosing spondylitis (morning stiffness and back pain), and Crohn's disease (abdominal pain and diarrhea).

The invention also includes a label or package insert which indicates dose and administration information for the TNFα inhibitor. In one embodiment, the label or package insert of the invention indicates the TNFα inhibitor for the treatment of Crohn's disease. In a further embodiment, the label or package insert indicates that the initiation of therapy includes a 160 mg dose at week 0 and 80 mg at week 2. In still another embodiment, the label or package insert indicates that the maintenance dosing for the treatment of Crohn's disease with the TNFα inhibitor, *e.g.*, a TNFα antibody such as adalimumab, is 40 mg every other week. In one embodiment, the week 0 dose may be administered as 4 injections in one day or divided over 2 days. In a further embodiment, the label or package insert indicates that some patients with Crohn's disease may derive additional benefit by increasing frequency to 40 mg every week.

The invention also includes a label or package insert which indicates that the TNFα inhibitor is indicated for treatment of moderately to severely active Crohn's disease in adult patients who have had an inadequate response to conventional therapy. In another embodiment, the label or package insert of the invention indicates the TNFα inhibitor is also indicated for treatment in adult patients with moderately to severely active Crohn's disease who have lost response to or are intolerant to infliximab.

The invention also includes a label or package insert which indicates that the TNFα inhibitor administered by subcutaneous injection.

The invention also includes a label or package insert which indicates the recommended TNFα inhibitor dose regimen for adult patients with Crohn's disease is 160 mg at week 0 (dose can be administered as four injections in one day or as two injections per day for two consecutive days), 80 mg at week 2, followed by 40 mg every other week beginning at week 4. In one embodiment, the label or package insert of the invention indicates that aminosalicylates, corticosteroids, and/or immunomodulatory agents (e.g., 6-mercaptopurine and azathioprine) may be continued during treatment with the TNFα inhibitor, *e.g.,* a TNFα antibody such as adalimumab. In an additional embodiment, the label or package insert of the invention indicates that some patients may derive additional benefit from increasing the dosing frequency of the TNFα inhibitor from 40 mg every other week to 40 mg every week.

The invention also includes a label or package insert which indicates warnings and precautions regarding the use of the TNFα inhibitor. In one embodiment, the information provided in the label or package insert describes malignancies. In another embodiment, the label or package insert of the invention may indicate during the controlled portions of TNFα inhibitor trials in patients with rheumatoid arthritis, psoriatic arthritis, ankylosing spondylitis, and Crohn's disease, malignancies, other than lymphoma and non-melanoma skin cancer, were observed at a rate (95% confidence interval) of 0.6 (0.3, 1.0)/100 patient-years among 2887 adalimumab-treated patients versus a rate of 0.4 (0.2, 1.1)/100 patient-years among 1570 control patients (median duration of treatment of 5.7 months for adalimumab-treated patients and 5.5 months for control-treated patients). In another embodiment, the label of the invention indicates that the size of the control group and limited duration of the controlled portions of studies precludes the ability to draw firm conclusions. In one embodiment, the label indicates that in the controlled and uncontrolled open-label portions of the clinical trials of the TNFα inhibitor, the more frequently observed malignancies, other than lymphoma and non-melanoma skin cancer, were breast, colon, prostate, lung and melanoma. In one embodiment, the label indicates that these malignancies in TNFα inhibitor treated and control-treated patients were similar in type and number to what would be expected in the general population. In a further embodiment, the label indicates that during the controlled portions of the TNFα inhibitor rheumatoid arthritis, psoriatic arthritis, ankylosing spondylitis, and Crohn's disease trials, the rate (95% confidence interval) of non-melanoma skin cancers was 0.8 (0.47, 1.24)/100 patient-years among adalimumab - treated patients 0.2 (0.05, 0.82)/100 patient-years among control patients. In one embodiment, the label indicates that the potential role of TNF blocking therapy in the development of malignancies is not known. In one embodiment, the label indicates that in the controlled portions of clinical trials of all the TNF-blocking agents, more cases of lymphoma have been observed among patients receiving TNF blockers compared to control patients. In one embodiment, the label indicates that in controlled trials in patients with rheumatoid arthritis, psoriatic arthritis, ankylosing spondylitis, and Crohn's disease, 2 lymphomas were observed among 2887 HUMIRA®-treated patients versus 1 among 1570 control patients. In another embodiment, the label of the invention indicates that in combining the controlled and uncontrolled open-label portions of these clinical trials with a median duration of approximately 2 years, including 4843 patients and over 13,000 patient-years of therapy, the observed rate of lymphomas is approximately 0.12/100 patient-years, and that this is approximately 3.5-fold higher than expected in the general population.

The label of the invention may also contain information regarding the use of the TNFα inhibitor in clinical studies for Crohn's disease. In one embodiment, the label of the invention describes the studies described herein as Example 1, either as a whole or in portion. In one embodiment, the label of the invention indicates that the TNFα inhibitor has been studied in over 1400 patients with Crohn's disease in four placebo-controlled and two open-label extension studies. In a further embodiment, the label of the invention indicates that the safety profile for patients with Crohn's disease treated with the TNFα inhibitor was similar to the safety profile seen in patients with rheumatoid arthritis.

The label of the invention may also contain information regarding the pharmacodynamics of the TNFα inhibitor. In one embodiment, the label of the invention indicates that after treatment with the TNFα inhibitor, a rapid decrease in levels of acute phase reactants of inflammation (C-reactive protein (CRP) and erythrocyte sedimentation rate (ESR) and serum cytokines (IL-6) was observed compared to baseline in patients with rheumatoid arthritis. In one embodiment, the label of the invention indicates that a rapid decrease in CRP levels was also observed in patients with Crohn's disease. The label may further indicate serum levels of matrix metalloproteinases (MMP-1 and MMP-3) that produce tissue remodeling responsible for cartilage destruction were also decreased after TNFα inhibitor administration.

The label of the invention may also contain information regarding the pharmacokinetics of the TNFα inhibitor. In one embodiment, the label of the invention indicates that in patients with Crohn's disease, the loading dose of 160 mg TNFα inhibitor on week 0 followed by 80 mg TNFα inhibitor on week 2 achieves serum adalimumab trough concentrations of approximately 12 µg/mL. The label of the invention may also indicate mean steady-state trough levels of approximately 7 µg/mL were observed in Crohn's disease patients who received a maintenance dose of 40 mg adalimumab every other week

The label of the invention may also contain information regarding the drug interactions of the TNFα inhibitor, with other drugs. In one embodiment, the label indicates that methotrexate (MTX) reduced adalimumab apparent clearance after single and multiple dosing by 29% and 44% respectively, in patients with rheumatoid arthritis.

In one embodiment, the TNFα inhibitor is selected from the group consisting of a TNFα antibody, or an antigen-binding portion thereof; a TNF fusion protein; and a recombinant TNF binding protein.

In one embodiment, the TNF fusion protein is etanercept.

In one embodiment, the TNFα antibody, or antigen-binding portion thereof, is an antibody selected from the group consisting of a humanized antibody, a chimeric antibody, a human antibody, and a multivalent antibody.

In one embodiment, the TNFα antibody, or antigen-binding portion thereof, is infliximab or golimumab.

In one embodiment, the human antibody, or an antigen-binding portion thereof, dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less.

In another embodiment, the human antibody, or an antigen-binding portion thereof, has the following characteristics:
a) dissociates from human TNFα with a K_{off} rate constant of 1 x 10⁻³ s⁻¹ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

In still another embodiment, the human antibody, or an antigen-binding portion thereof, comprises a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and comprises a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11.

In one embodiment, the human antibody, or an antigen-binding portion thereof, comprises a light chain variable region (LCVR) comprising the amino acid sequence of SEQ ID NO: 1 and a heavy chain variable region (HCVR) comprising the amino acid sequence of SEQ ID NO: 2.

In one embodiment, the human antibody, or an antigen-binding portion thereof, is adalimumab.

In one embodiment, the maintenance dose regimen comprises biweekly administration of a maintenance dose of the TNFα antibody, or antigen-binding portion thereof, to the subject. In one embodiment, the maintenance dose of the TNFα antibody, or antigen-binding portion thereof, comprises about 40 mg.

In one embodiment, the maintenance dose regimen comprises weekly administration of a maintenance dose of the TNFα antibody, or antigen-binding portion thereof, to the subject. In one embodiment, the maintenance dose of the TNFα antibody, or antigen-binding portion thereof, comprises about 40 mg.

In one embodiment, the TNFα antibody, or antigen-binding portion thereof, is administered in combination with an additional therapeutic agent.

In one embodiment, the maintenance dose comprises about 40 mg.

In one embodiment, the anti-TNFα antibody, or antigen-binding portion thereof, is selected from the group consisting of a 40 mg dose, an 80 mg does, and a 160 mg dose.

### BRIEF DESCRIPTION OF THE FIGURES

*Figure 1* shows the Study F study overview.
*Figure 2* describes the study design described in the example 3.
*Figure 3* graphically depicts the clinical responses to adalimumab induction at week 4.
*Figure 4* shows a graph which shows the patient population at week 4 according to response or non-response.
*Figure 5* shows the percentage of patients in the randomized responder population who maintained clinical remission (CDAI < 150).
*Figure 6* graphically depicts over time the percentage of patients in the randomized responder population who maintained clinical remission (CDAI < 150) from Study R.
*Figure 7* graphically depicts the maintenance of healing of draining fistulas at week 26 and at weeks 26 and 56 for all randomized patients.
*Figure 8* shows the patient disposition.
*Figure 9* shows the mean (SD) serum adalimumab concentration in patients with Crohn's disease from Study E.
*Figure 10* shows the mean (SD) serum adalimumab concentration in patients with Crohn's disease from StudyF.
*Figure 11* shows the Study F randomized cohort, wherein clinical remission is CDAI < 150.
*Figure 12* shows the days in clinical remission for patients who achieved clinical remission in study 3.
*Figure 13* shows the mean CDAI score at each visit.
*Figure 14* shows the mean C-reactive protein concentration at weeks 0 and 4. A 70-point response and a 100-point response were defined as a decrease from baseline in the CDAI score of at least 70 and 100 points, respectively. Remission was defined as a decrease of the CDAI score to less than 150 points. Statistically significant differences between the treatment groups are shown. CDAI, Crohn's Disease Activity Index.

### DETAILED DESCRIPTION OF THE INVENTION

### I. Definitions

The term "human TNFα" (abbreviated herein as hTNFα, or simply hTNF), as used herein, is intended to refer to a human cytokine that exists as a 17 kD secreted form and a 26 kD membrane associated form, the biologically active form of which is composed of a trimer of noncovalently bound 17 kD molecules. The structure of hTNFα is described further in, for example, Pennica, D., et al. (1984) Nature 312:724-729; Davis, J.M., et al. (1987) Biochemistry 26:1322-1326; and Jones, E.Y., et al. (1989) Nature 338:225-228. The term human TNFα is intended to include recombinant human TNFα (rhTNFα), which can be prepared by standard recombinant expression methods or purchased commercially (R & D Systems, Catalog No. 210-TA, Minneapolis, MN). TNFα is also referred to as TNF.

The term "TNFα inhibitor" includes agents which interfere with TNFα activity. The term also includes each of the anti-TNFα human antibodies and antibody portions described herein as well as those described in U.S. Patent Nos. 6,090,382; 6,258,562; 6,509,015, and in U.S. Patent Application Serial Nos. 09/801185 and 10/302356. In one embodiment, the TNFα inhibitor used in the invention is an anti-TNFα antibody, or a fragment thereof, including infliximab (Remicade^{®}, Johnson and Johnson; described in U.S. Patent No. 5,656,272, incorporated by reference herein), CDP571 (a humanized monoclonal anti-TNF-alpha IgG4 antibody), CDP 870 (a humanized monoclonal anti-TNF-alpha antibody fragment), an anti-TNF dAb (Peptech), CNTO 148 (golimumab; Medarex and Centocor, see WO 02/12502), and adalimumab (HUMIRA®^{®} Abbott Laboratories, a human anti-TNF mAb, described in US 6,090,382 as D2E7). Additional TNF antibodies which may be used in the invention are described in U. S. Patent Nos. 6,593,458; 6,498,237; 6,451,983; and 6,448,380, each of which is incorporated by reference herein. In another embodiment, the TNFα inhibitor is a TNF fusion protein, e.g., etanercept (Enbrel^{®}, Amgen; described in WO 91/03553 and WO 09/406476, incorporated by reference herein). In another embodiment, the TNFα inhibitor is a recombinant TNF binding protein (r-TBP-I) (Serono).

The term "antibody", as used herein, is intended to refer to immunoglobulin molecules comprised of four polypeptide chains, two heavy (H) chains and two light (L) chains inter-connected by disulfide bonds. Each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The antibodies of the invention are described in further detail in U.S. Patent Nos. 6,090,382; 6,258,562; and 6,509,015, each of which is incorporated herein by reference in its entirety.

The term "antigen-binding portion" or "antigen-binding fragment" of an antibody (or simply "antibody portion"), as used herein, refers to one or more fragments of an antibody that retain the ability to specifically bind to an antigen (*e.g*., hTNFα). It has been shown that the antigen-binding function of an antibody can be performed by fragments of a full-length antibody. Binding fragments include Fab, Fab', F(ab')₂, Fabc, Fv, single chains, and single-chain antibodies. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al. (1989) Nature 341:544-546 ), which consists of a VH domain; and (vi) an isolated complementarity determining region (CDR), Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.,* Bird et al. (1988) Science 242:423-426; and Huston et al. (1988) Proc. Natl. Acad. Sci. USA 85:5879-5883) . Such single chain antibodies are also intended to be encompassed within the term "antigen-binding portion" of an antibody. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see *e.g.,* Holliger et al. (1993) Proc. Natl. Acad Sci. USA 90:6444-6448; Poljak et al. (1994) Structure 2:1121-1123). The antibody portions of the invention are described in further detail in U.S. Patent Nos. 6,090,382, 6,258,562, 6,509,015, each of which is incorporated herein by reference in its entirety.

Still further, an antibody or antigen-binding portion thereof may be part of a larger immunoadhesion molecules, formed by covalent or noncovalent association of the antibody or antibody portion with one or more other proteins or peptides. Examples of such immunoadhesion molecules include use of the streptavidin core region to make a tetranleric scFv molecule (Kipriyanov, S.M., et al. (1995) Human Antibodies and Hybridomas 6:93-101) and use of a cysteine residue, a marker peptide and a C-terminal polyhistidine tag to make bivalent and biotinylated scFv molecules (Kipriyanov, S.M., et al. (1994) Mol. Immunol. 31:1047-1058). Antibody portions, such as Fab and F(ab')₂ fragments, can be prepared from whole antibodies using conventional techniques, such as papain or pepsin digestion, respectively, of whole antibodies. Moreover, antibodies, antibody portions and immunoadhesion molecules can be obtained using standard recombinant DNA techniques, as described herein.

A "conservative amino acid substitution", as used herein, is one in which one amino acid residue is replaced with another amino acid residue having a similar side chain. Families of amino acid residues having similar side chains have been defined in the art, including basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g.,* threonine, valine, isoleucine) and aromatic side chains (*e.g.,* tyrosine, phenylalanine, tryptophan, histidine).

"Chimeric antibodies" refers to antibodies wherein one portion of each of the amino acid sequences of heavy and light chains is homologous to corresponding sequences in antibodies derived from a particular species or belonging to a particular class, while the remaining segment of the chains is homologous to corresponding sequences from another species. In one embodiment, the invention features a chimeric antibody or antigen-binding fragment, in which the variable regions of both light and heavy chains mimics the variable regions of antibodies derived from one species of mammals, while the constant portions are homologous to the sequences in antibodies derived from another species. In a preferred embodiment of the invention, chimeric antibodies are made by grafting CDRs from a mouse antibody onto the framework regions of a human antibody.

"Humanized antibodies" refer to antibodies which comprise at least one chain comprising variable region framework residues substantially from a human antibody chain (referred to as the acceptor immunoglobulin or antibody) and at least one complementarity determining region (CDR) substantially from a non-human-antibody (e.g., mouse). In addition to the grafting of the CDRs, humanized antibodies typically undergo further alterations in order to improve affinity and/or immmunogenicity.

The term "multivalent antibody" refers to an antibody comprising more than one antigen recognition site. For example, a "bivalent" antibody has two antigen recognition sites, whereas a "tetravalent" antibody has four antigen recognition sites. The terms "monospecific", "bispecific", "trispecific", "tetraspecific", etc: refer to the number of different antigen recognition site specificities (as opposed to the number of antigen recognition sites) present in a multivalent antibody. For example, a "monospecific" antibody's antigen recognition sites all bind the same epitope. A "bispecific" or "dual specific" antibody has at least one antigen recognition site that binds a first epitope and at least one antigen recognition site that binds a second epitope that is different from the first epitope. A "multivalent monospecific" antibody has multiple antigen recognition sites that all bind the same epitope. A "multivalent bispecific" antibody has multiple antigen recognition sites, some number of which bind a first epitope and some number of which bind a second epitope that is different from the first epitope

The term "human antibody", as used herein, is intended to include antibodies having variable and constant regions derived from human germline immunoglobulin sequences. The human antibodies of the invention may include amino acid residues not encoded by human germline immunoglobulin sequences (*e.g.,* mutations introduced by random or site-specific mutagenesis *in vitro* or by somatic mutation *in vivo),* for example in the CDRs and in particular CDR3. However, the term "human antibody", as used herein, is not intended to include antibodies in which CDR sequences derived from the germline of another mammalian species, such as a mouse, have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (*e.g.,* a mouse) that is transgenic for human immunoglobulin genes (see *e.g.,* Taylor et al. (1992) Nucl. Acids Res. 20:6287) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.*

Such chimeric, humanized, human, and dual specific antibodies can be produced by recombinant DNA techniques known in the art, for example using methods described in PCT International Application No. PCT/US86/02269; European Patent Application No. 184,187; European Patent Application No. 171,496; European Patent Application No. 173,494; PCT International Publication No, WO 86/01533; U.S. Pat. No. 4,816,567; European Patent Application No. 125,023; Better et al. (1988) Science 240:1041-1043; Liu et al. (1987) Proc. Natl. Acad. Sci. USA 84:3439-3443; Liu et al. (1987) J. Immunol. 139:3521-3526; Sun et al. (1987) Proc. Natl. Acad. Sci. USA 84:214-218; Nishimura ef al. (1987) Cancer Res. 47:999-1005; Wood et al. (1985) Nature 314:446-449; Shaw et al. (1988) J. Natl. Cancer Inst. 80:1553-1559); Morrison (1985) Science 229:1202-1207; Oi et al. (1986) Bio Techniques 4:214; U.S. Pat. No. 5,225,539; Jones et al. (1986) Nature 321:552-525; Verhoeyan et al. (1988) Science 239:1534; and Beidler et al. (1988) J. Immunol. 141:4053-4060, Queen et al., Proc. Natl. Acad. Sci. USA 86:10029-10033 (1989), US 5,530,101, US 5,585,089, US 5,693,761, US 5,693,762, Selick et al., WO 90/07861, and Winter, US 5,225,539.

An "isolated antibody", as used herein, is intended to refer to an antibody that is substantially free of other antibodies having different antigenic specificities (*e.g.*, an isolated antibody that specifically binds hTNFα is substantially free of antibodies that specitically-bind antigens other than hTNFα). An isolated antibody that specifically binds hTNFα may, however, have cross-reactivity to other antigens, such as TNFα molecules from other species. Moreover, an isolated antibody may be substantially free of other cellular material and/or chemicals.

A "neutralizing antibody", as used herein (or an "antibody that neutralized hTNFα activity"), is intended to refer to an antibody whose binding to hTNFα results in inhibition of the biological activity of hTNFα. This inhibition of the biological activity of hTNFα can be assessed by measuring one or more indicators of hTNFα biological activity, such as hTNFα-induced cytotoxicity (either *in vitro* or *in vivo*), hTNFα-induced cellular activation and TNFα binding to hTNFα receptors. These indicators of hTNFα biological activity can be assessed by one or more of several standard *in vitro* or *in vivo* assays known in the art (see U.S. Patent No. 6,090,382). Preferably, the ability of an antibody to neutralize hTNFα activity is assessed by inhibition of hTNFα-induced cytotoxicity of L929 cells. As an additional or alternative parameter of hTNFα activity, the ability of an antibody to inhibit hTNFα-induced expression of ELAM-1 on HUVEC, as a measure of hTNFα-induced cellular activation, can be assessed.

The term "surface plasmon resonance", as used herein, refers to an optical phenomenon that allows for the analysis of real-time biospecific interactions by detection of alterations in protein concentrations within a biosensor matrix, for example using the BIAcore system (Pharmacia Biosensor AB, Uppsala, Sweden and Piscataway, NJ). For further descriptions, see Example 1 of U.S. Patent 6,258,562 and Jönsson et al, (1993) Ann. Biol. Clin. 51:19; Jönsson et al. (1991) Biotechniques 11:620-627; Johnsson et al. (1995) J. Mol. Recognit. 8:125; and Johnnson et al. (1991) Anal.Biochem.198:268.

The term "K_{off}", as used herein, is intended to refer to the off rate constant for dissociation of an antibody from the antibody/antigen complex.

The term "K_{d}", as used herein, is intended to refer to the dissociation constant of a particular antibody-antigen interaction.

The term "IC₅₀" as used herein, is intended to refer to the concentration of the inhibitor required to inhibit the biological endpoint of interest, *e.g.,* neutralize cytotoxicity activity.

The term "dose," as used herein, refers to an amount of TNFα inhibitor which is administered to a subject.

The term "dosing", as used herein, refers to the administration of a substance (*e.g.,* an anti-TNFα antibody) to achieve a therapeutic objective (e.g., treatment of Crohn's disease).

A "dosing regimen" describes a treatment schedule for a TNFα inhibitor, *e.g.*, a treatment schedule over a prolonged period of time and/or throughout the course of treatment, *e.g.* administering a first dose of a TNFα inhibitor at week 0 followed by a second dose of a TNFα inhibitor on a biweekly dosing regimen.

The term "multiple-variable dose" includes different doses of a TNFα inhibitor which are administered to a subject for therapeutic treatment. "Multiple-variable dose regimen" or "multiple-variable dose therapy" describes a treatment schedule which is based on administering different amounts of TNFα inhibitor at various time points throughout the course of treatment. Multiple-variable dose regimens are described in PCT application no. PCT/US05/12007 and US 20060009385, which is incorporated by reference herein.

The term "maintenance therapy" or "maintenance dosing regime" refers to a treatment schedule for a subject or patient diagnosed with a disorder/disease, *e.g.,* Crohn's disease, to enable them to maintain their health in a given state, *e.g,* remission. Generally, the first goal of treatment of Crohn's is to induce remission in the subject in need thereof. The next challenge is to keep the subject in remission. Maintenance doses may be used in a maintenance therapy for maintaining remission in a subject who has achieved remission of a disease or who has reached a state of the disease which is advantageous, *e.g.* reduction in symptoms. In one embodiment, a maintenance therapy of the invention is used for a subject or patient diagnosed with a disorder/disease, *e.g.,* Crohn's disease, to enable them to maintain their health in a state which is completely free of symptoms associated with the disease. In one embodiment, a maintenance therapy of the invention is used for a subject or patient diagnosed with a disorder/disease, *e.g.,* Crohn's disease, to enable them to maintain their health in a state which is substantially free of symptoms associated with the disease. In one embodiment, a maintenance therapy of the invention is used for a subject or patient diagnosed with a disorder/disease, *e.g.,* Crohn's disease, to enable them to maintain their health in a state where there is a significant reduction in symptoms associated with the disease. In one embodiment, maintenance therapy is used for a subject having Crohn's disease to maintain a CDAI score of less than 150 for the subject.

The term "induction dose" or "loading dose," used interchangeably herein, refers to the first dose of TNFα inhibitor which is initially used to induce remission of Crohn's disease. Often, the loading dose is larger in comparison to the subsequent maintenance or treatment dose. The induction dose can be a single dose or, alternatively, a set of doses. In one embodiment, an induction dose is subsequently followed by administration of smaller doses of TNFα inhibitor, *e.g.,* the treatment or maintenance dose. The induction dose is administered during the induction or loading phase of therapy. In one embodiment of the invention, the induction dose is at least twice the given amount of the treatment dose.

The term "treatment phase" or "maintenance phase", as used herein, refers to a period of treatment comprising administration of a TNFα inhibitor to a subject in order to maintain a desired therapeutic effect, *i.e.*, maintaining remission of Crohn's disease.

The term "maintenance dose" or "treatment dose" is the amount of TNFα inhibitor taken by a subject to maintain or continue a desired therapeutic effect. A maintenance dose can be a single dose or, alternatively, a set of doses. A maintenance dose is administered during the treatment or maintenance phase of therapy. In one embodiment, amaintenance dose(s) is smaller than the induction dose(s) and can be equal to each other when administered in succession. In one embodiment, the invention provides a maintenance dose of 40 mg of adalimumab administered subcutaneously to a subject who is in remission, every other week, or biweekly. In one embodiment, the maintenance dose is administered every other week beginning at week 4 of treatment.

The terms "biweekly dosing regimen", "biweekly dosing", and "biweekly administration", as used herein, refer to the time course of administering a substance *(e.g.,* an anti-TNFα antibody) to a subject to achieve a therapeutic objective, *e.g*, throughout the course of treatment. The biweekly dosing regimen is not intended to include a weekly dosing regimen. Preferably, the substance is administered every 9-19 days, more preferably, every 11-17 days, even more preferably, every 13-15 days, and most preferably, every 14 days. In one embodiment, the biweekly dosing regimen is initiated in a subject at week 0 of treatment. In another embodiment, a maintenance dose is administered on a biweekly dosing regimen. In one embodiment, both the loading and maintenance doses are administered according to a biweekly dosing regimen. In one embodiment, biweekly dosing includes a dosing regimen wherein doses of a TNPα inhibitor are administered to a subject every other week beginning at week 0. In one embodiment, biweekly dosing includes a dosing regimen where doses of a TNFα inhibitor are administered to a subject every other week consecutively for a given time period, *e.g.,* 4 weeks, 8 weeks, 16, weeks, 24 weeks, 26 weeks, 32 weeks, 36 weeks, 42 weeks, 48 weeks, 52 weeks, 56 weeks, etc. Biweekly dosing methods are also described in US 20030235585, incorporated by reference herein.

The term "combination" as in the phrase "a first agent in combination with a second agent" includes co-administration of a first agent and a second agent, which for example may be dissolved or intermixed in the same pharmaceutically acceptable carrier, or administration of a first agent, followed by the second agent, or administration of the second agent, followed by the first agent. The present invention, therefore, includes methods of combination therapeutic treatment and combination pharmaceutical compositions.

The term "concomitant" as in the phrase "concomitant therapeutic treatment" includes administering an agent in the presence of a second agent. A concomitant therapeutic treatment method includes methods in which the first, second, third, or additional agents are co-administered. A concomitant therapeutic treatment method also includes methods in which the first or additional agents are administered in the presence of a second or additional agents, wherein the second or additional agents, for example, may have been previously administered. A concomitant therapeutic treatment method may be executed step-wise by different actors. For example, one actor may administer to a subject a first agent and a second actor may to administer to the subject a second agent, and the administering steps may be executed at the same time, or nearly the same time, or at distant times, so long as the first agent (and additional agents) are after administration in the presence of the second agent (and additional agents). The actor and the subject may be the same entity (*e.g.,* human).

The term "combination therapy", as used herein, refers to the administration of two or more therapeutic substances, *e.g.,* an anti-TNFα antibody and another drug. The other drug(s) may be administered concomitant with, prior to, or following the administration of an anti-TNFα antibody.

The term "treatment, as used within the context of the present invention, is meant to include therapeutic treatment, as well as prophylactic or suppressive measures, for the treatment of Crohn's disease. For example, the term treatment may include administration of a TNFα inhibitor prior to or following the onset of Crohn's disease thereby preventing or removing signs of the disease or disorder. As another example, administration of a TNFα inhibitor after clinical manifestation of Crohn's disease to combat the symptoms and/or complications and disorders associated with Crohn's disease comprises "treatment" of the disease. Further, administration of the agent after onset and after clinical symptoms and/or complications have developed where administration affects clinical parameters of the disease or disorder and perhaps amelioration of the disease, comprises "treatment" of the Crohn's disease. In one embodiment, treatment of Crohn's disease in a subject comprises inducing and maintaining remission of Crohn's disease in a subject. In another embodiment, treatment ofCrohn's disease in a subject comprises maintaining remission of Crohn's disease in a subject.

Those "in need of treatment" include mammals, such as humans, already having Crohn's disease, including those in which the disease or disorder is to be prevented.

Various aspects of the invention are described in further detail herein.

The invention provides improved uses and compositions for treating Crohn's disease with a TNFα inhibitor, *e.g.,* a human TNFα antibody, or an antigen-binding portion thereof. Compositions and articles of manufacture, including kits, relating to the methods and uses for treating Crohn's disease are also contemplated as part of the invention.

### II. TNF Inhibitors

A TNFα inhibitor which is used in the methods and compositions of the invention includes any agent which interferes with TNFα activity. In a preferred embodiment, the TNFα inhibitor can neutralize TNFα activity, particularly detrimental TNFα activity which is associated with Crohn's disease, and related complications and symptoms.

In one embodiment, the TNFα inhibitor used in the invention is an TNFα antibody (also referred to herein as a TNFα antibody), or an antigen-binding fragment thereof, including chimeric, humanized, and human antibodies. Examples of TNFα antibodies which may be used in the invention include, but not limited to, infliximab (Remicade^{®}, Johnson and Johnson; described in U.S. Patent No. 5,656,272, incorporated by reference herein), CDP571 (a humanized monoclonal anti-TNF-alpha IgG4 antibody), CDP 870 (a humanized monoclonal anti-TNF-alpha antibody fragment), an anti-TNF dAb (Peptech), CNTO 148 (golimumab; Medarex and Centocor, see WO 02/12502), and adalimumab (HUMIRA^{®} Abbott Laboratories, a human anti-TNF mAb, described in US 6,090,382 as D2E7). Additional TNF antibodies which may be used in the invention are described in U.S. Patent Nos. 6,593,458; 6,498,237; 6,451,983; and 6,448,380, each of which is incorporated by reference herein.

Other examples of TNFα inhibitors which may be used in the methods and compositions of the invention include etanercept (Enbrel, described in WO 91/03553 and WO 09/406476), soluble TNF receptor Type I, a pegylated soluble TNF receptor Type I (PEGs TNF-R1), p55TNFR1gG (Lenercept), and recombinant TNF binding protein (r-TBP-I) (Serono).

In one embodiment, the term "TNFα inhibitor" excludes infliximab. In one embodiment, the term "TNFα inhibitor" excludes adalimumab. In another embodiment, the term "TNFα inhibitor" excludes adalimumab and infliximab.

In one embodiment, the term "TNFα inhibitor" excludes etanercept, and, optionally, adalimumab, infliximab, and adalimumab and infliximab.

In one embodiment, the term "TNFα antibody" excludes infliximab. In one embodiment, the term "TNFα antibody" excludes adalimumab. In another embodiment, the term "TNFα antibody" excludes adalimumab and infliximab.

In one embodiment, the invention features uses and composition for treating or determining the efficacy of a TNFα inhibitor for the treatment of Crohn's disease, wherein the TNFα antibody is an isolated human antibody, or antigen-binding portion thereof, that binds to human TNFα with high affinity and a low off rate, and also has a high neutralizing capacity. Preferably, the human antibodies used in the invention are recombinant, neutralizing human anti-hTNFα antibodies. The most preferred recombinant, neutralizing antibody of the invention is referred to herein as D2E7, also referred to as HUMIRA^{®} or adalimumab (the amino acid sequence of the D2E7 VL region is shown in SEQ ID NO: 1; the amino acid sequence of the D2E7 VH region is shown in SEQ ID NO: 2). The properties of D2E7 (adalimumab / HUMIRA^{®}) have been described in Salfeld et al., U.S. Patent Nos. 6,090,382, 6,258,562, and 6,509,015, which are each incorporated by reference herein. The methods of the invention may also be performed using chimeric and humanized murine anti-hTNFα antibodies which have undergone clinical testing for treatment of rheumatoid arthritis (see *e.g.,* Elliott, M.J., et al. (1994) Lancet 344:1125-1127; Elliot, M.J., et al. (1994) Lancet 344:1105-1110; Rankin, E.C., et al. (1995) Br. J. Rheumatol, 34:334-342).

In one embodiment, the method of the invention includes determining the efficacy of D2E7 antibodies and antibody portions, D2E7-related antibodies and antibody portions, or other human antibodies and antibody portions with equivalent properties to D2E7, such as high affinity binding to hTNFα with low dissociation kinetics and high neutralizing capacity, for the treatment of Crohn's disease. In one embodiment, the invention provides treatment with an isolated human antibody, or an antigen-binding portion thereof, that dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less. More preferably, the isolated human antibody, or antigen-binding portion thereof, dissociates from human TNFα with a K_{off} of 5 x 10⁻⁴ s⁻¹ or less, or even more preferably, with a K_{off} of 1 x 10⁻⁴ s⁻¹ or less. More preferably, the isolated human antibody, or antigen-binding portion thereof, neutralizes human TNFα cytotoxicity in a standard *in vitro* L929 assay with an IC₅₀ of 1 x 10⁻⁸ M or less, even more preferably with an IC₅₀ of 1 x 10⁻⁹ M or less and still more preferably with an IC₅₀ of 1 x 10⁻¹⁰ M or less. In a Preferred embodiment, the antibody is an isolated human recombinant antibody, or an antigen-binding portion thereof.

It is well known in the art that antibody heavy and light chain CDR3 domains play an important role in the binding specificity/affinity of an antibody for an antigen. Accordingly, in another aspect, the invention pertains to treating Crohn's disease by administering human antibodies that have slow dissociation kinetics for association with hTNFα and that have light and heavy chain CDR3 domains that structurally are identical to or related to those of D2E7. Position 9 of the D2E7 VL CDR3 can be occupied by Ala or Thr without substantially affecting the K_{off}. Accordingly, a consensus motif for the D2E7 VL CDR3 comprises the amino acid sequence: Q-R-Y-N-R-A-P-Y-(T/A) (SEQ ID NO: 3). Additionally, position 12 of the D2E7 VH CDR3 can be occupied by Tyr or Asn, without substantially affecting the K_{off}. Accordingly, a consensus motif for the D2E7 VH CDR3 comprises the amino acid sequence: V-S-Y-L-S-T-A-S-S-L-D-(Y/N) (SEQ ID NO: 4). Moreover, as demonstrated in Example 2 of U.S. Patent No. 6,090,382, the CDR3 domain of the D2E7 heavy and light chains is amenable to substitution with a single alanine residue (at position 1, 4, 5, 7 or 8 within the VL CDR3 or at position 2, 3, 4, 5, 6, 8, 9, 10 or 11 within the VH CDR3) without substantially affecting the K_{off}. Still further, the skilled artisan will appreciate that, given the amenability of the D2E7 VL and VH CDR3 domains to substitutions by alanine, substitution of other amino acids within the CDR3 domains may be possible while still retaining the low off rate constant of the antibody, in particular substitutions with conservative amino acids. Preferably, no more than one to five conservative amino acid substitutions are made within the D2E7 VL and/or VH CDR3 domains. More preferably, no more than one to three conservative amino acid substitutions are made within the D2E7 VL and/or VH CDR3 domains. Additionally, conservative amino acid substitutions should not be made at amino acid positions critical for binding to hTNFα. Positions 2 and 5 of the D2E7 VL CDR3 and positions 1 and 7 of the D2E7 VH CDR3 appear to be critical for interaction with hTNFα and thus, conservative amino acid substitutions preferably are not made at these positions (although an alanine substitution at position 5 of the D2E7 VL CDR3 is acceptable, as described above) (see U.S. Patent No. d,090,382).

Accordingly, in another embodiment, the antibody or antigen-binding portion thereof preferably contains the following characteristics:
a) dissociates from human TNFα with a K_{off} rate constant of 1 x 10⁻³ s⁻¹ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

More preferably, the antibody, or antigen-binding portion thereof, dissociates from human TNFα with a K_{off} of 5 x 10⁻⁴ s⁻¹ or less. Even more preferably, the antibody, or antigen-binding portion thereof, dissociates from human TNFα with a K_{off} of 1 x 10⁻⁴ s⁻¹ or less.

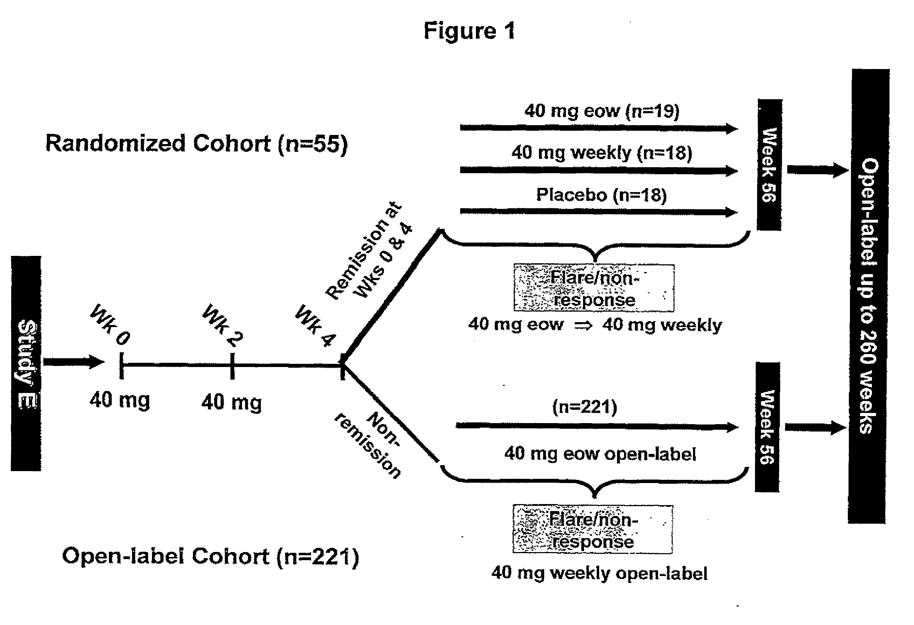

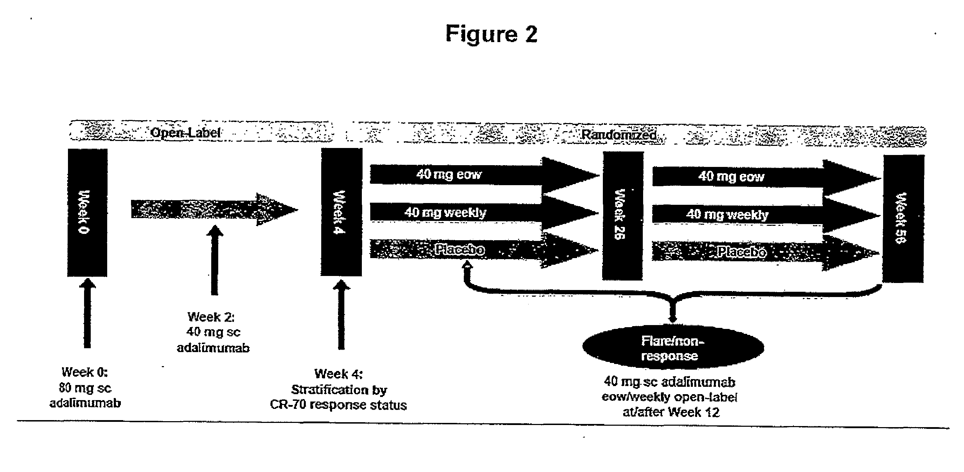

In yet another embodiment, the antibody or antigen-binding portion thereof preferably contains a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and with a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11. Preferably, the LCVR further has a CDR2 domain comprising the amino acid sequence of SEQ ID NO: 5 *(i.e.,* the D2E7 VL CDR2) and the HCVR further has a CDR2 domain comprising the amino acid sequence of SEQ ID NO: 6 (*i.e*., the D2E7 VH CDR2). Even more preferably, the LCVR further has CDR1 domain comprising the amino acid sequence of SEQ ID NO: 7 (*i.e*., the D2E7 VL CDR1) and the HCVR has a CDR1 domain comprising the amino acid sequence of SEQ ID NO: 8 (i.e., the D2E7 VH CDR1). The framework regions for VL preferably are from the V_{κ}I human germline family, more preferably from the A20 human germline Vk gene and most preferably from the D2E7 VL framework sequences shown in Figures 1A and 1B of U.S. Patent No. 6,090,382. The framework regions for VH preferably are from the V_{H}3 human germline family, more preferably from the DP-31 human germline VH gene and most preferably from the D2E7 VH framework sequences shown in Figures 2A and 2B of U.S. Patent No. 6,090,382.

Accordingly, in another embodiment, the antibody or antigen-binding portion thereof preferably contains a light chain variable region (LCVR) comprising the amino acid sequence of SEQ ID NO: 1 (*i.e*., the D2E7 VL) and a heavy chain variable region (HCVR) comprising the amino acid sequence of SEQ ID NO: 2 (*i.e*., the D2E7 VH). In certain embodiments, the antibody comprises a heavy chain constant region, such as an IgG1, IgG2, IgG3, IgG4, IgA, IgE, IgM or IgD constant region. Preferably, the heavy chain constant region is an IgG1 heavy chain constant region or an IgG4 heavy chain constant region. Furthermore, the antibody can comprise a light chain constant region, either a kappa light chain constant region or a lambda light chain constant region. Preferably, the antibody comprises a kappa light chain constant region. Alternatively, the antibody portion can be, for example, a Fab fragment or a single chain Fv fragment.

In still other embodiments, the invention includes uses of an isolated human antibody, or an antigen-binding portions thereof, containing D2E7-related VL and VH CDR3 domains. For example, antibodies, or antigen-binding portions thereof, with a light chain variable region (LCVR) having a CDR3 domain comprising an amino acid sequence selected from the group consisting of SEQ ID NO: 3, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25 and SEQ ID NO: 26 or with a heavy chain variable region (HCVR) having a CDR3 domain comprising an amino acid sequence selected from the group consisting of SEQ ID NO: 4, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34 and SEQ ID NO: 35.

The TNFα antibody used in the methods and compositions of the invention may be modified for improved treatment of Crohn's disease. In some embodiments, the TNFα antibody or antigen binding fragments thereof, is chemically modified to provide a desired effect. For example, pegylation of antibodies and antibody fragments of the invention may be carried out by any of the pegylation reactions known in the art, as described, for example, in the following references: Focus on Growth Factors 3:4-10 (1992); EP 0 154 316; and EP 0 401 384 (each of which is incorporated by reference herein in its entirety). Preferably, the pegylation is carried out via an acylation reaction or an alkylation reaction with a reactive polyethylene glycol molecule (or an analogous reactive water-soluble polymer). A preferred water-soluble polymer for pegylation of the antibodies and antibody fragments of the invention is polyethylene glycol (PEG). As used herein, "polyethylene glycol" is meant to encompass any of the forms of PEG that have been used to derivatize other proteins, such as mono (Cl-ClO) alkoxy- or aryloxy-polyethylene glycol.

Methods for preparing pegylated antibodies and antibody fragments of the invention will generally comprise the steps of (a) reacting the antibody or antibody fragment with polyethylene glycol, such as a reactive ester or aldehyde derivative of PEG, under conditions whereby the antibody or antibody fragment becomes attached to one or more PEG groups, and (b) obtaining the reaction products. It will be apparent to one of ordinary skill in the art to select the optimal reaction conditions or the acylation reactions based on known parameters and the desired result.

Pegylated antibodies and antibody fragments may generally be used to treat Crohn's disease by administration of the TNFα antibodies and antibody fragments described herein, Generally the pegylated antibodies and antibody fragments have increased half-life, as compared to the nonpegylated antibodies and antibody fragments. The pegylated antibodies and antibody fragments may be employed alone, together, or in combination with other pharmaceutical compositions.

In yet another embodiment of the invention, TNFα antibodies or fragments thereof can be altered wherein the constant region of the antibody is modified to reduce at least one constant region-mediated biological effector function relative to an unmodified antibody. To modify an antibody of the invention such that it exhibits reduced binding to the Fc receptor, the immunoglobulin constant region segment of the antibody can be mutated at particular regions necessary for Fc receptor (FcR) interactions (see *e.g.,* Canfield, S.M. and S.L. Morrison (1991) J. Exp. Med 173:1483-1491; and Lund, J. et al. (1991) J. of Iminunol. 147:2657-2662). Reduction in FcR binding ability of the antibody may also reduce other effector functions which rely on FcR interactions, such as opsonization and phagocytosis and antigen-dependent cellular cytotoxicity.

An antibody or antibody portion used in the methods of the invention can be derivatized or linked to another functional molecule (e.g., another peptide or protein). Accordingly, the antibodies and antibody portions of the invention are intended to include derivatized and otherwise modified forms of the human anti-hTNFα antibodies described herein, including immunoadhesion molecules. For example, an antibody or antibody portion of the invention can be functionally linked (by chemical coupling, genetic fusion, noncovalent association or otherwise) to one or more other molecular entities, such as another antibody (*e.g.,* a bispecific antibody or a diabody), a detectable agent, a cytotoxic agent, a pharmaceutical agent, and/or a protein or peptide that can mediate associate of the antibody or antibody portion with another molecule (such as a streptavidin core region or a polyhistidine tag).

One type of derivatized antibody is produced by crosslinking two or more antibodies (of the same type or of different types, e.g., to create bispecific antibodies). Suitable crosslinkers include those that are heterobifunctional, having two distinctly reactive groups separated by an appropriate spacer (*e.g.*, m-maleimidobenzoyl-N-hydroxysuccinimide ester) or homobifunctional (*e.g.,* disuccinimidyl suberate). Such linkers are available from Pierce Chemical Company, Rockford, IL.

Useful detectable agents with which an antibody or antibody portion of the invention may be derivatized include fluorescent compounds. Exemplary fluorescent detectable agents include fluorescein, fluorescein isothiocyanate, rhodamine, 5-dimethylamine-1-napthalenesulfonyl chloride, phycoerythrin and the like. An antibody may also be derivatized with detectable enzymes, such as alkaline phosphatase, horseradish peroxidase, glucose oxidase and the Like. When an antibody is derivatized with a detectable enzyme, it is detected by adding additional reagents that the enzyme uses to produce a detectable reaction product. For example, when the detectable agent horseradish peroxidase is present, the addition of hydrogen peroxide and diaminobenzidine leads to a colored reaction product, which is detectable. An antibody may also be derivatized with biotin, and detected through indirect measurement of avidin or streptavidin binding.

An antibody, or antibody portion, used in the methods and compositions of the invention, can be prepared by recombinant expression of immunoglobulin light and heavy chain genes in a host cell. To express an antibody recombinantly, a host cell is transfected with one or more recombinant expression vectors carrying DNA fragments encoding the immunoglobulin light and heavy chains of the antibody such that the light and heavy chains are expressed in the host cell and, preferably, secreted into the medium in which the host cells are cultured, from which medium the antibodies can be recovered. Standard recombinant DNA methodologies are used to obtain antibody heavy and light chain genes, incorporate these genes into recombinant expression vectors and introduce the vectors into host cells, such as those described in Sambrook, Fritsch and Maniatis (eds), Molecular Cloning; A Laboratory Manual, Second Edition, Cold Spring Harbor, N.Y., (1989), Ausubel, F.M. et al. (eds.) Current Protocols in Molecular Biology, Greene Publishing Associates, (1989) and in U.S. Patent No. 4,816,397 by Boss et al.

To express adalimumab (D2E7) or an adalimumab (D2E7)-related antibody, DNA fragments encoding the light and heavy chain variable regions are first obtained. These DNAs can be obtained by amplification and modification of germline light and heavy chain variable sequences using the polymerase chain reaction (PCR). Germline DNA sequences for human heavy and light chain variable region genes are known in the art (see *e.g.,* the "Vbase" human germline sequence database; see also Kabat, E.A., et al. (1991) Sequences of Proteins of Immunological Interest, Fifth Edition, U.S. Department of Health and Human Services, NIH Publication No. 91-3242; Tomlinson, I.M., et al. (1992) "The Repertoire of Human Germline VH Sequences Reveals about Fifty Groups of VH Segments with Different Hypervariable Loops" J. Mol. Biol. 227:776-798; and Cox, J.P.L. et al. (1994) "A Directory of Human Germ-line V78 Segments Reveals a Strong Bias in their Usage" Eur. J. Immunol. 24:827-836; the contents of each of which are expressly incorporated herein by reference). To obtain a DNA fragment encoding the heavy chain variable region of D2B7, or a D2E7-related antibody, a member of the V_{H}3 family of human germline VH genes is amplified by standard PCR. Most preferably, the DP-31 VH germline sequence is amplified. To obtain a DNA fragment encoding the light chain variable region of D2E7, or a D2E7-related antibody, a member of the V_{κ}I family of human germline VL genes is amplified by standard PCR. Most preferably, the A20 VL germline sequence is amplified. PCR primers suitable for use in amplifying the DP-31 germline VH and A20 germline VL sequences can be designed based on the nucleotide sequences disclosed in the references cited *supra*, using standard methods.

Once the germline VH and VL fragments are obtained, these sequences can be mutated to encode the D2E7 or D2E7-related amino acid sequences disclosed herein. The amino acid sequences encoded by the germline VH and VL DNA sequences are first compared to the D2E7 or D2E7-related VH and VL amino acid sequences to identify amino acid residues in the D2E7 or D2E7-related sequence that differ from germline. Then, the appropriate nucleotides of the germline DNA sequences are mutated such that the mutated germline sequence encodes the D2E7 or D2E7-related amino acid sequence, using the genetic code to determine which nucleotide changes should be made. Mutagenesis of the germline sequences is carried out by standard methods, such as PCR-mediated mutagenesis (in which the mutated nucleotides are incorporated into the PCR primers such that the PCR product contains the mutations) or site-directed mutagenesis.

Moreover, it should be noted that if the "germline" sequences obtained by PCR amplification encode amino acid differences in the framework regions from the true germline configuration (*i.e*., differences in the amplified sequence as compared to the true germline sequence, for example as a result of somatic mutation), it may be desireable to change these amino acid differences back to the true germline sequences (*i.e*., "backmutation" of framework residues to the germline configuration).

Once DNA fragments encoding D2E7 or D2E7-related VH and VL segments are obtained (by amplification and mutagenesis of germline VH and VL genes, as described above), these DNA fragments can be further manipulated by standard recombinant DNA techniques, for example to convert the variable region genes to full-length antibody chain genes, to Fab fragment genes or to a scFv gene. In these manipulations, a VL- or VH-encoding DNA fragment is operatively linked to another DNA fragment encoding another protein, such as an antibody constant region or a flexible linker. The term "operatively linked", as used in this context, is intended to mean that the two DNA fragments are joined such that the amino acid sequences encoded by the two DNA fragments remain in-frame.

The isolated DNA encoding the VH region can be converted to a full-length heavy chain gene by operatively linking the VH-encoding DNA to another DNA molecule encoding heavy chain constant regions (CH1, CH2 and CH3). The sequences of human heavy chain constant region genes are known in the art (see *e.g*., Kabat, E.A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an IgG1, IgG2, IgG3, IgG4, IgA, IgE, IgM or IgD constant region, but most preferably is an IgG1 or IgG4 constant region. For a Fab fragment heavy chain gene, the VH-encoding DNA can be operatively linked to another DNA molecule encoding only the heavy chain CH1 constant region.

The isolated DNA encoding the VL region can be converted to a full-length light chain gene (as well as a Fab light chain gene) by operatively linking the VL-encoding DNA to another DNA molecule encoding the light chain constant region, CL. The sequences of human light chain constant region genes are known in the art (see e.g., Kabat, E.A., et al. (1991) Sequences of Proteins of Immuno/ogical Interest, Fifth Edition, U.S. Department of Health and Human Services, NIH Publication No. 91-3242) and DNA fragments encompassing these regions can be obtained by standard PCR amplification. The light chain constant region can be a kappa or lambda constant region, but most preferably is a kappa constant region.

To create a scFv gene, the VH- and VL-encoding DNA fragments are operatively linked to another fragment encoding a flexible linker, *e.g.,* encoding the amino acid sequence (Gly₄-Ser)₃, such that the VH and VL sequences can be expressed as a contiguous single-chain protein, with the VL and VH regions joined by the flexible linker (see *e.g.,* Bird et al. (1988) Science 242:423-426; Huston et al. (1988) Proc. Natl. Acad. Sci. USA 85:5879-5883; McCafferty et al., Nature (1990) 348:552-554).

To express the antibodies, or antibody portions used in the invention, DNAs encoding partial or full-length light and heavy chains, obtained as described above, are inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vector or, more typically, both genes are inserted into the same expression vector. The antibody genes are inserted into the expression vector by standard methods (*e.g.,* ligation of complementary restriction sites on the antibody gene fragment and vector, or blunt end ligation if no restriction sites are present). Prior to insertion of the D2E7 or D2E7-related light or heavy chain sequences, the expression vector may already carry antibody constant region sequences. For example, one approach to converting the D2E7 or D2E7-related VH and VL sequences to full-length antibody genes is to insert them into expression vectors already encoding heavy chain constant and light chain constant regions, respectively, such that the VH segment is operatively linked to the CH segment(s) within the vector and the VL segment is operatively linked to the CL segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (*i.e.,* a signal peptide from a non-immunoglobulin protein).

In addition to the antibody chain genes, the recombinant expression vectors of the invention carry regulatory sequences that control the expression of the antibody chain genes in a host cell. The term "regulatory sequence" is intended to include promoters, enhancers and other expression control elements *(e.g.,* polyadenylation signals) that control the transcription or translation of the antibody chain genes. Such regulatory sequences are described, for example, in Goeddel; Gene Expression Technology: Methods in Enzymology 185, Academic Press, San Diego, CA (1990). It will be appreciated by those skilled in the art that the design of the expression vector, including the selection of regulatory sequences may depend on such factors as the choice of the host cell to be transformed, the level of expression of protein desired, *etc.* Preferred regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, *(e.g.,* the adenovirus major late promoter (AdMLP)) and polyoma. For further description of viral regulatory elements, and sequences thereof, see *e.g*., U.S. Patent No. 5,168,062 by Stinski, U.S. Patent No. 4,510,245 by Bell et al. and U.S. Patent No. 4,968,615 by Schaffner et al.

In addition to the antibody chain genes and regulatory sequences, the recombinant expression vectors used in the invention may carry additional sequences, such as sequences that regulate replication of the vector in host cells (e.g., origins of replication) and selectable marker genes. The selectable marker gene facilitates selection of host cells into which the vector has been introduced (see *e.g.,* U.S. Patents Nos. 4,399,216, 4,634,665 and 5,179,017, all by Axel *et al.*)*.* For example, typically the selectable marker gene confers resistance to drugs, such as G418, hygromycin or methotrexate, on a host cell into which the vector has been introduced. Preferred selectable marker genes include the dihydrofolate reductase (DHFR) gene (for use in dhfr⁻ host cells with methotrexate selection/amplification) and the neo gene (for G418 selection).

For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, *e.g.,* electroporation, calcium-phosphate precipitation, DEAE-dextran transfection and the like. Although it is theoretically possible to express the antibodies of the invention in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active antibody. Prokaryotic expression of antibody genes has been reported to be ineffective for production of high yields of active antibody (Boss, M.A. and Wood, C. R. (1985) Immunology Today 6:12-13).

Preferred mammalian host cells for expressing the recombinant antibodies of the invention include Chinese Hamster Ovary (CHO cells) (including dhfr- CHO cells, described in Urlaub and Chasin, (1980) Proc. Natl. Acad. Sci. USA 77:4216-4220, used with a DHFR selectable marker, *e.g.,* as described in R.J. Kaufman and P.A. Sharp (1982) Mol. Biol. 159:601-621), NSO myeloma cells, COS cells and SP2 cells. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

Host cells can also be used to produce portions of intact antibodies, such as Fab fragments or scFv molecules. It is understood that variations on the above procedure are within the scope of the present invention. For example, it may be desirable to transfect a host cell with DNA encoding either the light chain or the heavy chain (but not both) of an antibody of this invention. Recombinant DNA technology may also be used to remove some or all of the DNA encoding either or both of the light and heavy chains that is not necessary for binding to hTNFα. The molecules expressed from such truncated DNA molecules are also encompassed by the antibodies of the invention. In addition, bifunctional antibodies may be produced in which one heavy and one light chain are an antibody of the invention and the other heavy and light chain are specific for an antigen other than hTNFα by crosslinking an antibody of the invention to a second antibody by standard chemical crosslinking methods.

In a preferred system for recombinant expression of an antibody, or antigen-binding portion thereof, of the invention, a recombinant expression vector encoding both the antibody heavy chain and the antibody light chain is introduced into dhfr-CHO cells by calcium phosphate-mediated transfection. Within the recombinant expression vector, the antibody heavy and light chain genes are each operatively linked to CMV enhancer/AdMLP promoter regulatory elements to drive high levels of transcription of the genes. The recombinant expression vector also carries a DHFR gene, which allows for selection of CHO cells that have been transfected with the vector using methotrexate selection/amplification. The selected transformant host cells are culture to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium.

In view of the foregoing, nucleic acid, vector and host cell compositions that can be used for recombinant expression of the antibodies and antibody portions used in the invention include nucleic acids, and vectors comprising said nucleic acids, comprising the human TNFα antibody adalimumab (D2E7). The nucleotide sequence encoding the D2E7 light chain variable region is shown in SEQ ID NO: 36. The CDR1 domain of the LCVR encompasses nucleotides 70-102, the CDR2 domain encompasses nucleotides 148-168 and the CDR3 domain encompasses nucleotides 265-291. The nucleotide sequence encoding the D2E7 heavy chain variable region is shown in SEQ ID NO: 37. The CDR1 domain of the HCVR encompasses nucleotides 91-105, the CDR2 domain encompasses nucleotides 148-198 and the CDR3 domain encompasses nucleotides 295-330. It will be appreciated by the skilled artisan that nucleotide sequences encoding D2E7-related antibodies, or portions thereof *(e.g.,* a CDR domain, such as a CDR3 domain), can be derived from the nucleotide sequences encoding the D2E7 LCVR and HCVR using the genetic code and standard molecular biology techniques.

Recombinant human antibodies of the invention in addition to D2E7 or an antigen binding portion thereof, or D2E7-related antibodies disclosed herein can be isolated by screening of a recombinant combinatorial antibody library, preferably a scFv phage display library, prepared using human VL and VH cDNAs prepared from mRNA derived from human lymphocytes. Methodologies for preparing and screening such libraries are known in the art. In addition to commercially available kits for generating phage display libraries (*e.g*., the Pharmacia Recombinant Phage Antibody System, catalog no. 27-9400-01; and the Stratagene *SurfZAP*™ phage display kit, catalog no. 240612), examples of methods and reagents particularly amenable for use in generating and screening antibody display libraries can be found in, for example, Ladner et al. U.S. Patent No. 5,223,409; Kang et al. PCT Publication No. WO 92/18619; Dower et al. PCT Publication No. WO 91/17271; Winter et al. PCT Publication No. WO 92/20791; Markland et al. PCT Publication No. WO 92/15679; Breitling et al. PCT Publication No. WO 93/01288; McCafferty et al. PCT Publication No. WO 92/01047; Garrard et al. PCT Publication No. WO 92/09690; Fuchs et al. (1991) Bio/Technology 9:1370-1372; Hay et al. (1992) Hum Antibod Hybridomas 3:81-65; Huse et al. (1989) Science 246:1275-1281; McCafferty et al., Nature (1990) 348:552-554; Griffiths et al. (1993) EMBO J 12:725-734; Hawkins et al, (1992) J Mol Biol 226:889-896; Clackson et al. (1991) Nature 352:624-628; Gram et al. (1992) PNAS 89:3576-3580; Garrard et al. (1991) Bio/Technology 9:1373-1377; Hoogenboom et al. (1991) Nuc Acid Res 19:4133-4137; and Barbas et al. (1991) PNAS 88:7978-7982.

In a preferred embodiment, to isolate human antibodies with high affinity and a low off rate constant for hTNFα, a murine anti-hTNFα antibody having high affinity and a low off rate constant for hTNFα (*e.g*., MAK 195, the hybridoma for which has deposit number ECACC 87 050801) is first used to select human heavy and light chain sequences having similar binding activity toward hTNFα, using the epitope imprinting methods described in Hoogenboom et al., PCT Publication No. WO 93/06213. The antibody libraries used in this method are preferably scFv libraries prepared and screened as described in McCafferty et al., PCT Publication No. WO 92/01047, McCafferty et al., Nature (1990) 348:552-554; and Griffiths et al., (1993) EMBO J 12:725-734. The scFv antibody libraries preferably are screened using recombinant human TNFα as the antigen.

Once initial human VL and VH segments are selected, "mix and match" experiments, in which different pairs of the initially selected VL and VH segments are screened for hTNFα, binding, are performed to select preferred VL/VH pair combinations. Additionally, to further improve the affinity and/or lower the off rate constant for hTNFα binding, the VL and VH segments of the preferred VL/VH pair(s) can be randomly mutated, preferably within the CDR3 region of VH and/or VL, in a process analogous to the *in vivo* somatic mutation process responsible for affinity maturation of antibodies during a natural immune response. This *in vitro* affinity maturation can be accomplished by amplifying VH and VL regions using PCR primers complimentary to the VH CDR3 or VL CDR3, respectively, which primers have been "spiked" with a random mixture of the four nucleotide bases at certain positions such that the resultant PCR products encode VH and VL segments into which random mutations have been introduced into the VH and/or VL CDR3 regions. These randomly mutated VH and VL segments can be rescreened for binding to hTNFα and sequences that exhibit high affinity and a low off rate for hTNFα binding can be selected.

Following screening and isolation of an anti-hTNFα antibody of the invention from a recombinant immunoglobulin display library, nucleic acid encoding the selected antibody can be recovered from the display package (e.g., from the phage genome) and subcloned into other expression vectors by standard recombinant DNA techniques. If desired, the nucleic acid can be further manipulated to create other antibody forms of the invention (*e.g*., linked to nucleic acid encoding additional immunoglobulin domains, such as additional constant regions). To express a recombinant human antibody isolated by screening of a combinatorial library, the DNA encoding the antibody is cloned into a recombinant expression vector and introduced into a mammalian host cells, as described in further detail in above.

Methods of isolating human neutralizing antibodies with high affinity and a low off rate constant for hTNFα are described in U.S. Patent Nos. 6,090,382, 6,258,562, and 6,509,015, each of which is incorporated by reference herein.

Antibodies, antibody-portions, and other TNFα inhibitors for use in the methods of the invention, can be incorporated into pharmaceutical compositions suitable for administration to a subject. Typically, the pharmaceutical composition comprises an antibody, antibody portion, or other TNFα inhibitor, and a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. In many cases, it is preferable to include isotonic agents, for example, sugars, polyalcohols such as mannitol, sorbitol, or sodium chloride in the composition. Pharmaceutically acceptable carriers may further comprise minor amounts of auxiliary substances such as wetting or emulsifying agents, preservatives or buffers, which enhance the shelf life or effectiveness of the antibody, antibody portion, or other TNFα inhibitor.

The compositions for use in the methods and compositions of the invention may be in a variety of forms. These include, for example, liquid, semi-solid and solid dosage forms, such as liquid solutions (*e.g*., injectable and infusible solutions), dispersions or suspensions, tablets, pills, powders, liposomes and suppositories. The preferred form depends on the intended mode of administration and therapeutic application. Typical preferred compositions are in the form of injectable or infusible solutions, such as compositions similar to those used for passive immunization of humans with other antibodies or other TNFα inhibitors. The preferred mode of administration is parenteral *(e.g.,* intravenous, subcutaneous, intraperitoneal, intramuscular). In a preferred embodiment, the antibody or other TNFα inhibitor is administered by intravenous infusion or injection. In another preferred embodiment, the antibody or other TNFα inhibitor is administered by intramuscular or subcutaneous injection.

Therapeutic compositions typically must be sterile and stable under the conditions of manufacture and storage. The composition can be formulated as a solution, microemulsion, dispersion, liposome, or other ordered structure suitable to high drug concentration. Sterile injectable solutions can be prepared by incorporating the active compound (*i.e*., antibody, antibody portion, or other TNFα inhibitor) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

In one embodiment, the invention includes pharmaceutical compositions comprising an effective TNFα inhibitor and a pharmaceutically acceptable carrier, wherein the effective TNFα inhibitor may be used to treat Crohn's disease.

In one embodiment, the antibody or antibody portion for use in the methods of the invention is incorporated into a pharmaceutical formulation as described in PCT/IB03/04502 and U.S. Appln. No. 20040033228, incorporated by reference herein. This formulation includes a concentration 50 mg/ml of the antibody D2E7 (adalimumab), wherein one pre-filled syringe contains 40 mg of antibody for subcutaneous injection.

The antibodies, antibody-portions, and other TNFα inhibitors of the present invention can be administered by a variety of methods known in the art, although for many therapeutic applications, the preferred route/mode of administration is parenteral, e.g., subcutaneous injection. In another embodiment, administration is via intravenous injection or infusion.

As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, *e.g*., Sustained and Controlled Release Drug Delivery Systems, Robinson, ed., Dekker, Inc., New York, 1978.

In one embodiment, the TNFα antibodies and inhibitors used in the invention are delivered to a subject subcutaneously. In one embodiment, the subject administers the TNFα inhibitor, including, but not limited to, TNFα antibody, or antigen-binding portion thereof, to himself/herself.

The TNFα antibodies and inhibitors used in the invention may also be administered in the form of protein crystal formulations which include a combination of protein crystals encapsulated within a polymeric carrier to form coated particles. The coated particles of the protein crystal formulation may have a spherical morphology and be microspheres of up to 500 micro meters in diameter or they may have some other morphology and be microparticulates. The enhanced concentration of protein crystals allows the antibody of the invention to be delivered subcutaneously. In one embodiment, the TNFα antibodies of the invention are delivered via a protein delivery system, wherein one or more of a protein crystal formulation or composition, is administered to a subject with a TNFα-related disorder. Compositions and methods of preparing stabilized formulations of whole antibody crystals or antibody fragment crystals are also described in WO 02/072636, which is incorporated by reference herein. In one embodiment, a formulation comprising the crystallized antibody fragments described in PCT/IB03/04502 and U.S. Appln. No. 20040033228, incorporated by reference herein, are used to treat rheumatoid arthritis using the treatment methods of the invention.

In certain embodiments, an antibody, antibody portion, or other TNFα inhibitor of the invention may be orally administered, for example, with an inert diluent or an assimilable edible carrier. The compound (and other ingredients, if desired) may also be enclosed in a hard or soft shell gelatin capsule, compressed into tablets, or incorporated directly into the subject's diet. For oral therapeutic administration, the compounds may be incorporated with excipients and used in the form of ingestible tablets, buccal tablets, troches, capsules, elixirs, suspensions, syrups, wafers, and the like. To administer a compound of the invention by other than parenteral administration, it may be necessary to coat the compound with, or co-administer the compound with, a material to prevent its inactivation.

Supplementary active compounds can also be incorporated into the compositions. In certain embodiments, an antibody or antibody portion for use in the methods of the invention is coformulated with and/or coadministered with one or more additional therapeutic agents, including an Crohn's disease inhibitor or antagonist. For example, an anti-hTNFα antibody or antibody portion of the invention may be coformulated and/or coadministered with one or more additional antibodies that bind other targets associated with TNFα related disorders (*e.g.*, antibodies that bind other cytokines or that bind cell surface molecules), one or more cytokines, soluble TNFα receptor (see e.g., PCT Publication No. WO 94/06476) and/or one or more chemical agents that inhibit TNFα production or activity (such as cyclohexane-ylidene derivatives as described in PCT Publication No. WO 931/19751) or any combination thereof. Furthermore, one or more antibodies of the invention may be used in combination with two or more of the foregoing therapeutic agents. Such combination therapies may advantageously utilize lower dosages of the administered therapeutic agents, thus avoiding possible side effects, complications or low level of response by the patient associated with the various monotherapies.

The pharmaceutical compositions of the invention may include a "therapeutically effective amount" or a "prophylactically effective amount" of an antibody or antibody portion of the invention. A "therapeutically effective amount" refers to an amount effective, at dosages and for periods of time necessary, to achieve the desired therapeutic result. A therapeutically effective amount of the antibody, antibody portion, or other TNFα inhibitor may vary according to factors such as the disease state, age, sex, and weight of the individual, and the ability of the antibody, antibody portion, other TNFα inhibitor to elicit a desired response in the individual. A therapeutically effective amount is also one in which any toxic or detrimental effects of the antibody, antibody portion, or other TNFα inhibitor are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

Additional description regarding methods and uses of the invention comprising administration of a TNFα inhibitor are described in Part III of this specification.

The invention also pertains to packaged pharmaceutical compositions or kits for administering the anti-TNF antibodies of the invention for the treatment of Crohn's disease. In one embodiment of the invention, the kit comprises a TNFα inhibitor, such as an antibody and instructions for administration of the TNFα inhibitor for treatment of Crohn's disease. The instructions may describe how, *e.g*., subcutaneously, and when, *e.g.,* at week 0, week 2, week 4, etc., the different doses of TNFα inhibitor shall be administered to a subject for treatment.

Another aspect of the invention pertains to kits containing a pharmaceutical composition comprising a TNFα inhibitor, such as an antibody, and a pharmaceutically acceptable carrier and one or more pharmaceutical compositions each comprising an additional therapeutic agent useful for treating Crohn's disease, and a pharmaceutically acceptable carrier. Alternatively, the kit comprises a single pharmaceutical composition comprising an anti-TNFα antibody, one or more drugs useful for treating Crohn's disease, and a pharmaceutically acceptable carrier. The instructions may describe how, e.g., subcutaneously, and when, *e.g.,* at week 0, week 2, week 4, etc., the different doses of TNFα inhibitor and/or the additional therapeutic agent shall be administered to a subject for treatment.

The kit may contain instructions for dosing of the pharmaceutical compositions for the treatment of Crohn's disease. Additional description regarding articles of manufacture of the invention are described in subsection III.

The package or kit alternatively can contain the TNFα inhibitor and it can be promoted for use, either within the package or through accompanying information, for the uses or treatment of the disorders described herein. The packaged pharmaceuticals or kits further can include a second agent (as described herein) packaged with or copromoted with instructions for using the second agent with a first agent (as described herein).

### III. Uses and Compositions for Treating Crohn's Disease

TNFα is an important cytokine in the pathogenesis of Crohn's disease (CD), with elevated concentrations of TNFα playing a role in pathologic inflammation. A challenge to the treatment of Crohn's disease is maintaining remission in a subject once remission is achieved. Thus, one of the goals in treating Crohn's disease is to control active disease, e.g., to induce and maintain remission in patients / subjects. The methods and uses described herein provide a means of not only inducing remission, but also maintaining remission of Crohn's disease. In one embodiment, the invention provides a method for maintaining remission of Crohn's disease in a subject having Crohn's disease.

Remission of Crohn's disease may be determined according to standard clinical definitions. For example, clinical remission of Crohn's may be defined as a Crohn's Disease Activity Index (CDAI) score of less than 150. The CDAI score is derived as a weighted sum of eight different Crohn's disease-related subjective and objective assessments: extra-intestinal manifestations of Crohn's disease, abdominal mass, use of antidiarrheal drugs, body weight, hematocrit, total number of liquid or soft stools, abdominal pain/cramps, and general well-being. In one embodiment, remission of Crohn's disease is defined as a CDAI of < 150. Other measures of improvements in the disease state of a subject having Crohn's disease include clinical responses, such as a decrease in the CDAI score of at least about 70 points or a decrease in the CDAI score of at least about 100.

In one embodiment, the invention provides a method for maintaining remission of Crohn's disease in a subject who has achieved remission of Crohn's disease comprising administering a human TNFα antibody, or an antigen-binding portion thereof, to the subject, such that remission of Crohn's disease is maintained. In one embodiment, the invention describes a use of a human TNFα antibody, or antigen-binding portion thereof, in the manufacture of a medicament for maintaining remission of Crohn's disease in a subject who has achieved remission. The medicament may be for administration to the subject on a maintenance dose regimen. In one embodiment, remission of Crohn's disease is determined by a Crohn's Disease Activity Index (CDAI) score of less than 150 being maintained in the subject. The TNFα antibody, or an antigen-binding portion thereof, may be administered to the subject on a maintenance dose regimen. In one embodiment, the method and use of a human TNFα antibody, or an antigen-binding portion thereof, for maintaining remission of Crohn's disease, further comprises a method of decreasing steroid use in the subject.

In one embodiment, maintenance of remission of Crohn's disease is achieved by administering a human TNFα antibody, or an antigen-binding portion thereof, to a subject having Crohn's disease, wherein the human TNFα antibody, or an antigen-binding portion thereof, is administered on a maintenance therapy comprising a biweekly dosing regimen. In one embodiment, the human TNFα antibody, or an antigen-binding portion thereof, is administered in a dose of about 40 mg. In one embodiment, the human TNFα antibody, or an antigen-binding portion thereof, is adalimumab.

Methods of treatment described herein may include administration of a TNFα inhibitor to a subject to achieve a therapeutic goal, *e.g.,* induction and/or remission of Crohn's disease, decrease in CDAI score, maintenance of a level of CDAI score, and/or improvement in IBDQ score. Also included in the scope of the invention are uses of a TNFα inhibitor in the manufacture of a medicament to achieve a therapeutic goal, *e.g*., induction and/or remission, of Crohn's disease, decrease in CDAI score, maintenance of a level of CDAI score, and/or improvement in IBDQ score. Thus, where methods are described herein, it is also intended to be part of this invention that the use of the TNFα inhibitor in the manufacture of a medicament for the purpose of the method is also considered within the scope of the invention. Likewise, where a use of a TNFα inhibitor in the manufacture of a medicament for the purpose of achieving a therapeutic goal is described, methods of treatment resulting in the therapeutic goal are also intended to be part of the invention.

While steroids may work effectively, steroids are not effective in preventing flare-ups and thus are rarely used as a maintenance medication in Crohn's disease. Steroids also have many potentially serious side effects-such as elevated blood sugar, high blood pressure, cataracts, osteoporosis (even leading to bone fractures), among others. The risk of adverse effects increases with the duration of the treatment using steroids. The invention provides a means for phasing steroid use out while using a TNFα inhibitor to maintain remission of Crohn's disease. In one embodiment, use of the TNFα inhibitor, including a TNFα antibody, or an antigen-binding portion thereof, results in maintaining remission of Crohn's disease and decreasing steroid use in the subject. In another embodiment, steroid use by a subject having Crohn's is diminished by administering a TNFα inhibitor, *e.g.,* a human TNFα antibody, or an antigen-binding portion thereof, to the subject.

The invention also provides pharmacokinetic parameters which have been identified as providing a therapeutic benefit to a subject having Crohn's disease. Certain mean steady-state trough levels of a TNFα inhibitor have be identified as corresponding to therapeutic benefits for subject having Crohn's disease, including, but not limited to, maintenance of Crohn's disease. The term "trough level" refers to the serum TNFα inhibitor concentration at a time after delivery of a previous dose and immediately prior to delivery of the next subsequent dose of drug in a series of doses. Generally, the trough serum concentration is a minimum sustained efficacious drug concentration in the series of drug administrations. Also, the trough serum concentration is frequently targeted as a minimum serum concentration for efficacy because it represents the serum concentration at which another dose of drug is to be administered as part of the treatment regimen.

In one embodiment, the invention provides a method of inducing remission of Crohn's disease in a subject in need thereof comprising administering a loading dose of a TNFα inhibitor, *e.g.,* human TNFα antibody, or antigen-binding portion thereof, to the subject, wherein the loading dose provides a mean serum TNFα inhibitor trough level of about 12 µg/mL. Once remission has been achieved, *e.g.,* CDAI score of less than 150 achieved, a maintenance dose(s) of the TNFα inhibitor, e.g., human TNFα antibody, or antigen-binding portion thereof, may be administered to the subject in order to maintain remission of Crohn's disease, wherein the maintenance dose provides a mean serum trough level of about 7 µg/mL of the TNFα inhibitor.

In one embodiment, the invention provides a method of maintaining remission of Crohn's disease in a subject who has achieved remission of Crohn's disease comprising administering a maintenance dose of the TNFα inhibitor, *e.g*., human TNFα antibody, or antigen-binding portion thereof, to the subject, wherein the maintenance dose provides a mean serum trough level of about 7 µg/mL of the TNFα inhibitor.

The invention also provides a method of treating Crohn's-related disorders, comprising administering a TNFα inhibitor to a subject. The TNFα inhibitors used in the present invention may be administered by a variety of methods known in the art, although for many therapeutic applications, the preferred route/mode of administration is parenteral, including intravenous or subcutaneous injection. In one embodiment, the invention provides a method of treating fistulas associated with Crohn's disease.

In one embodiment, induction and remission of Crohn's disease is achieved using multiple variable dosing methods of treatment. Examples of such multiple variable dosing regimens are described in PCT appln. no. PCT/US05/12007, incorporated by reference herein.

In one embodiment, maintenance of remission of Crohn's disease is achieved by administering a TNFα inhibitor to a subject in accordance with a biweekly dosing regimen. Biweekly dosing regimens can be used to treat disorders in which TNFα activity is detrimental, and are further described in US Appln. No. 10/163657 (US 20030235585), incorporated by reference herein.

In one embodiment, the invention provides a method of inducing and maintaining remission of Crohn's disease in a subject comprising administering an initial loading dose of a TNFα inhibitor to the subject at week 0. In one embodiment, the initial dose is given in its entirety on one day or is divided over 2 days. In one embodiment, the initial dose is administered subcutaneously. Following administration of the initial loading dose, a second dose of the TNFα inhibitor may be administered to the subject, wherein the second dose is about half the dose amount of the initial loading dose. In one embodiment, the second dose is administered to the subject about two weeks after the first dose. In one embodiment, the second dose is administered subcutaneously. Subsequent doses may be administered following the second dose in order to achieve remission of the subject. Such additional doses may, in one embodiment of the invention, comprise half the dose amount of the second dose. Once remission is achieved, at least one a maintenance dose of the TNFα inhibitor is administered to the subject in order to maintain remission of Crohn's disease. In one embodiment, the maintenance dose is about half the dose amount of the second dose. In one embodiment, the maintenance dose is administered to the subject about two weeks after the second dose. In one embodiment, the maintenance therapy for administering the TNFα inhibitor comprises a biweekly dosing regimen. In one embodiment, the maintenance dose is administered subcutaneously.

In one embodiment, the invention provides a method of inducing and maintaining remission of Crohn's disease in a subject comprising administering an initial loading dose of a human TNFα antibody, or antigen-binding portion thereof, *e.g.,* adalimumab, to the subject at week 0. The initial dose may be given in its entirety on one day or may be divided over 2 days. In one embodiment, the initial dose of the human TNFα antibody, or antigen-binding portion thereof, comprises 160 mg. In one embodiment, the initial dose is administered subcutaneously. Following administration of the initial loading dose, a second dose of the human TNFα antibody, or antigen-binding portion thereof, *e.g.,* adalimumab, is administered to the subject, wherein the second dose is about half the dose amount of the loading dose. In one embodiment, the second dose comprises 80 mg of the human TNFα antibody, or antigen-binding portion thereof. In one embodiment, the second dose is administered to the subject about two weeks after the first dose. In one embodiment, the second dose is administered subcutaneously. In order to maintain remission of Crohn's disease once it is achieved, at least one maintenance dose of the human TNFα antibody, or antigen-binding portion thereof, *e.g.,* adalimumab, is administered to the subject. In one embodiment, the maintenance dose is about half the dose amount of the second dose. In one embodiment, the maintenance dose of the human TNFα antibody, or antigen-binding portion thereof, comprises 40 mg. In one embodiment, the maintenance therapy for administering the human TNFα antibody, or antigen-binding portion thereof, comprises a biweekly dosing regimen. In one embodiment, the maintenance dose is administered subcutaneously.

Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic or prophylactic effect to be achieved, and (b) the limitations inherent in the art of compounding such an active compound for the treatment of sensitivity in individuals.

Dosage regimens described herein may be adjusted to provide the optimum desired response, e.g., maintaining remission of Crohn's disease, in consideration of the teachings herein. It is to be noted that dosage values may vary with the type and severity of Crohn's disease. It is to be further understood that for any particular subject, specific dosage regimens may be adjusted over time according to the teachings of the specification and the individual need and the professional judgment of the person administering or supervising the administration of the compositions, and that dosage amounts and ranges set forth herein are exemplary only and are not intended to limit the scope or practice of the claimed invention.

Examples of other methods and uses of TNFα inhibitors for the treatment of Crohn's disease are also described in 60/801584, incorporated herein.

### Crohn's-related disorders

In addition, the invention provides methods and compositions for treating disorders often associated with Crohn's disease, *i.e.,* Crohn's-related disorders. The term "Crohn's disease-related disorder," is used to describe disorders and complications associated with Crohn's disease. Examples of Crohn's-related disorders include fistulas in the bladder, vagina, and skin; bowel obstructions; abscesses; nutritional deficiencies; complications from corticosteroid use; inflammation of the joints; erythem nodosum; pyoderma gangrenosum; and lesions of the eye. Other disorders commonly associated with Crohn's disease include Crohn's-related arthralgias, fistulizing Crohn's, indeterminant colitis, and pouchitis.

In one embodiment, the invention provides a method of maintaining remission of a Crohn's-related fistula in a subject comprising administering a TNFα inhibitor to the subject, such that remission of the Crohn's-related fistula is maintained. In one embodiment, the invention provides use of a TNFα inhibitor in the manufacture of a medicament for maintaining remission of a Crohn's-related fistula in a subject.

### Subpopulations

The invention provides uses and methods for treating certain subpopulations of Crohn's patients with a TNFα inhibitor.

In one embodiment, the invention provides a method of treating early Crohn's disease in a subject comprising administering to the subject a TNFα inhibitor, such that early Crohn's disease is treated. Subjects having early Crohn's disease may be administered a TNFα inhibitor such that early Crohn's disease is treated and advancement of the disease is prevented. The invention also provides use of a TNFα inhibitor in the manufacture of a medicament for the treatment of early Crohn's disease in a subject who has early Crohn's disease. In one embodiment, early Crohn's is defined as a disease duration of less than 2 years.

The invention also provides a method for treating a subpopulation of Crohn's patients who are intolerant to or have lost response to a first TNFα inhibitor, *e.g.,* infliximab, for the treatment of Crohn's. Clinical trials have demonstrated the efficacy of infliximab, a chimeric monoclonal antibody to TNF, for induction and maintenance therapy of patients with moderate to severe CD, including those with draining fistulas (Hanauer et al. Lancet 2002;359:1541-1549; Present et al. N Engl J Med 1999,340:1398-1405; Rutgeerts et al. Gastroenterology 1999;117:761-769; Sands et al. N Engl J Med 2004;3 50:876-885; and Targan et al. N Engl J Med 1997;337:1029-1035). Infusions of infliximab, especially when given episodically, may result in the development of antibodies to infliximab, however, which in turn may lead to infusion reactions, loss of efficacy, and delayed hypersensitivity reactions (Baert et al. N Engl J Med 2003;348:601-608; Cheifetz et al. Am J Gastroenterol 2003;98:1315-1324; Farrell et al. Gastroenterology 2003;124:917-924; Hanauer et al. Gastroenterology 1999;116:A731; and Hanauer et al. Clin Gastroenterol Hepatol 2004;2:542-553). In certain instances, some patients who are administered a TNFα inhibitor for the treatment of Crohn's disease and respond to said treatment, may eventually lose their response to the first TNFα inhibitor. In other patient populations, intolerance to a certain TNFα inhibitor may be marked from the initial administration of the TNFα inhibitor. In one embodiment, the invention provides use of a TNFα inhibitor in the manufacture of a medicament for inducing remission of Crohn's disease in a subject who has lost response to or is intolerant to a different TNFα inhibitor. In one embodiment, the TNFα inhibitor which the subject has lost response to or is intolerant to is infliximab.

In one embodiment, the invention also provides methods and compositions for use in a subject who has not previously been administered infliximab. Thus, in one embodiment, the methods and compositions of the invention are directed to a subpopulation of Crohn's patients who have not previously received infliximab.

In one embodiment, the invention provides an article of manufacture comprising adalimumab and a package insert, wherein the package insert indicates that adalimumab may be used to treat Crohn's disease in patients who have had an inadequate response to conventional therapy and/or who have lost response to or are intolerant to infliximab.

### Articles of Manufacture

The invention also provides a packaged pharmaceutical composition wherein the TNFα inhibitor, *e.g.,* TNFα antibody, is packaged within a kit or an article of manufacture. The kit or article of manufacture of the invention contains materials useful for the treatment, including induction and/or remission, prevention and/or diagnosis of Crohn's disease. The kit or article of manufacture comprises a container and a label or package insert or printed material on or associated with the container which provides information regarding use of the TNFα inhibitor, *e.g*., a TNFα antibody, for the treatment of Crohn's disease.

A kit or an article of manufacture refers to a packaged product comprising components with which to administer a TNFα inhibitor for treatment of a Crohn's disease. The kit preferably comprises a box or container that holds the components of the kit. The box or container is affixed with a label or a Food and Drug Administration approved label, including a protocol for administering the TNFα inhibitor. The box or container holds components of the invention which are preferably contained within plastic, polyethylene, polypropylene, ethylene, or propylene vessels. The vessels can be capped-tubes or bottles. The kit can also include instructions for administering the TNFα antibody of the invention. In one embodiment the kit of the invention includes the formulation comprising the human antibody adalimumab (or D2E7), as described in PCT/IB03/04502 and U.S. Appln. No. 10/222140, incorporated by reference herein.

The term "package insert" is used to refer to instructions customarily included in commercial packages of therapeutic products, that contain information about the indications, usage, dosage, administration, contraindications and/or warnings concerning the use of such therapeutic products.

In one embodiment, the article of manufacture of the invention comprises (a) a first container with a composition contained therein, wherein the composition comprises a

TNFα antibody; and (b) a package insert indicating that the TNFα antibody may be used for reducing signs and symptoms and inducing and maintaining remission of Crohn's disease. In a preferred embodiment, the label or package insert indicates that the TNFα inhibitor, *e.g.,* a TNFα antibody, is used for inducing and maintaining remission Crohn's disease.

Suitable containers for the TNFα inhibitor, *e.g.,* a TNFα antibody, include, for example, bottles, vials, syringes, pens, etc. The containers may be formed from a variety of materials such as glass or plastic. The container holds a composition which is by itself or when combined with another composition effective for treating, preventing and/or diagnosing the condition and may have a sterile access port.

In one embodiment, the article of manufacture comprises a TNFα inhibitor, e.g., a TNFα antibody, and a label which indicates to a subject who will be administering the TNFα inhibitor about using the TNFα inhibitor for the treatment of Crohn's disease. The label may be anywhere within or on the article of manufacture. In one embodiment, the article of manufacture comprises a container, such as a box, which comprises the TNFα inhibitor and a package insert or label providing information pertaining to use of the TNFα inhibitor for the treatment of Crohn's disease. In another embodiment, the information is printed on a label which is on the outside of the article of manufacture, in a position which is visible to prospective purchasers.

In one embodiment, the package insert of the invention informs a reader, including a subject, e.g., a purchaser, who will be administering the TNFα inhibitor for treatment, that the TNFα inhibitor, e.g., a TNFα antibody such as adalimumab, is an indicated treatment of Crohn's disease, including of moderately to severely active disease in adult patients.

In one embodiment, the package insert describes certain patient populations who may respond favorably to the TNFα inhibitor within the article of manufacture. For example, the package insert may indicate that the TNFα antibody, *e.g.,* adalimumab, may be used to treat Crohn's disease in patients who have had an inadequate response to conventional therapy and/or who have lost response to or are intolerant to infliximab. In another embodiment, the label of the invention indicates that adalimumab is indicated for treatment of moderately to severely active Crohn's disease in adult patients who have had an inadequate response to conventional therapy. In another embodiment, the label of the invention indicates that the TNFα inhibitor, e.g., a TNFα antibody such as adalimumab, is also indicated for treatment in adult patients with moderately to severely active Crohn's disease who have lost response to or are intolerant to infliximab.

In one embodiment, the package insert of the invention describes certain therapeutic benefits of the TNFα antibody, e.g., adalimumab, including specific symptoms of Crohn's disease which may be reduced by using the TNFα antibody, *e.g.,* adalimumab. It should be noted that the package insert may also contain information pertaining to other disorders which are treatable using the TNFα antibody, *e.g.,* adalimumab. Information described herein which is provided in a package insert and pertains to other disorders, *i.e*., diseases other than Crohn's disease, is also included within the scope of the invention. The package insert of the invention may indicate that extra TNFα in your body can attack normal healthy body tissues and cause inflammation especially in the tissues in your bones, cartilage, joints and digestive tract. The package insert of the invention may also indicate that adalimumab helps reduce the signs and symptoms of immune diseases, including rheumatoid and psoriatic arthritis (pain and swollen joints), ankylosing spondylitis (morning stiffness and back pain), and Crohn's disease (abdominal pain and diarrhea).

In another embodiment, the package insert of the invention describes the dose and administration of adalimumab, for the treatment of Crohn's disease. The label may indicate that the initiation of therapy includes a 160 mg dose at week 0 and 80 mg at week 2. The label may also indicate that the maintenance dosing for the treatment of Crohn's disease with adalimumab is 40 mg every other week. In one embodiment, the package insert indicates that the week 0 dose may be administered as 4 injections in one day or divided over 2 days. The label may also indicate that some patients with Crohn's disease may derive additional benefit by increasing frequency to 40 mg every week. In another embodiment, the package insert of the invention indicates that adalimumab is administered by subcutaneous injection.

In another embodiment, the label of the invention indicates that the recommended TNFα inhibitor, *e.g.,* a TNFα antibody such as adalimumab, dose regimen for adult patients with Crohn's disease is 160 mg at week 0 (dose can be administered as four injections in one day or as two injections per day for two consecutive days), 80 mg at week 2, followed by 40 mg every other week beginning at week 4. The label of the invention may also indicate that some patients may derive additional benefit from increasing the dosing frequency of the TNFα inhibitor, e.g., a TNFα antibody such as adalimumab from 40 mg every other week to 40 mg every week.

The package insert of the invention may also provide information to subjects who will be receiving adalimumab regarding combination uses for both safety and efficacy purposes. In another embodiment, the label of the invention indicates that aminosalicylates, corticosteroids, and/or immunomodulatory agents (e.g., 6-mercaptopurine and azathioprine) may be continued during treatment with the TNFα inhibitor, *e.g.,* a TNFα antibody, including adalimumab. In one embodiment, the invention provides an article of manufacture comprising a packaging material; a TNFα antibody, or antigen-binding portion thereof; and a label or package insert contained within the packaging material indicating that aminosalicylates, corticosteroids, and/or immunomodulatory agent, e.g., 6-mercaptopurine and azathioprine, may be continued during treatment with the TNFα antibody, or antigen-binding portion thereof

The package insert of the invention may contain warnings and precautions regarding the use of the TNFα inhibitor, *e.g.,* a TNFα antibody such as adalimumab. In one embodiment, the information provided in the label describes malignancies. The label of the invention may indicate that during the controlled portions of TNFα antibody, such as adalimumab, trials in patients with rheumatoid arthritis, psoriatic arthritis, ankylosing spondylitis, and Crohn's disease, malignancies, other than lymphoma and non-melanoma skin cancer, were observed at a rate (95% confidence interval) of 0.6 (0.3, 1.0)/100 patient-years among 2887 adalimumab-treated patients versus a rate of 0.4 (0.2, 1.1)/100 patient-years among 1570 control patients (median duration of treatment of 5.7 months for adalimumab-treated patients and 5.5 months for control-treated patients). The label may also indicate that the size of the control group and limited duration of the controlled portions of studies precludes the ability to draw firm conclusions. In one embodiment, the label indicates that in the controlled and uncontrolled open-label portions of the clinical trials of adalimumab, the more frequently observed malignancies, other than lymphoma and non-melanoma skin cancer, were breast, colon, prostate, lung and melanoma. In one embodiment, the label indicates that these malignancies in adalimumab treated and control-treated patients were similar in type and number to what would be expected in the general population. The label may further indicate that during the controlled portions of adalimumab rheumatoid arthritis, psoriatic arthritis, ankylosing spondylitis, and Crohn's disease trials, the rate (95% confidence interval) of non-melanoma skin cancers was 0.8 (0.47, 1.24)/100 patient-years among adalimumab -treated patients 0.2 (0.05, 0.82)/100 patient-years among control patients. In one embodiment, the label indicates that the potential role of TNF blocking therapy in the development of malignancies is not known. In one embodiment, the label indicates that in the controlled portions of clinical trials of all the TNF-blocking agents, more cases of lymphoma have been observed among patients receiving TNF blockers compared to control patients. In one embodiment, the label indicates that in controlled trials in patients with rheumatoid arthritis, psoriatic arthritis, ankylosing spondylitis, and Crohn's disease, 2 lymphomas were observed among 2887 HUMIRA®-treated patients versus 1 among 1570 control patients. In one embodiment, the label of the invention indicates that in combining the controlled and uncontrolled open-label portions of these clinical trials with a median duration of approximately 2 years, including 4843 patients and over 13,000 patient-years of therapy, the observed rate of lymphomas is approximately 0.12/100 patient-years, and that this is approximately 3.5-fold higher than expected in the general population.

The label of the invention may contain information regarding the use of the TNFα inhibitor, *e.g.,* a TNFα antibody such as adalimumab, in clinical studies for Crohn's disease. In one embodiment, the label of the invention describes the studies described herein as Example 1, either as a whole or in portion. The label of the invention may also indicate that adalimumab has been studied in over 1400 patients with Crohn's disease in four placebo-controlled and two open-label extension studies. The label of the invention may also indicate that the safety profile for patients with Crohn's disease treated with HUMIRA® was similar to the safety profile seen in patients with rheumatoid arthritis.

The label of the invention may contain information regarding the pharmacodynamics of the TNFα inhibitor, e.g., a TNFα antibody such as adalimumab. In one embodiment, the label of the invention indicates that after treatment with adalimumab, a rapid decrease in levels of acute phase reactants of inflammation (C-reactive protein (CRP) and erythrocyte sedimentation rate (ESR) and serum cytokines (IL-6) was observed compared to baseline in patients with rheumatoid arthritis. In one embodiment, the label of the invention indicates that a rapid decrease in CRP levels was also observed in patients with Crohn's disease. The label may further indicate that serum levels of matrix metalloproteinases (MMP-1 and MMP-3) that produce tissue remodeling responsible for cartilage destruction were also decreased after adalimumab administration.

The label of the invention may also contain information regarding the pharmacokinetics of the TNFα inhibitor, e.g., a TNFα antibody such as adalimumab. In one embodiment, the label of the invention indicates that in patients with Crohn's disease, the loading dose of 160 mg adalimumab on week 0 followed by 80 mg adalimumab on week 2 achieves serum adalimumab trough concentrations of approximately 12 µg/mL. The label of the invention may also indicate that mean steady-state trough levels of approximately 7 µg/mL were observed in Crohn's disease patients who received a maintenance dose of 40 mg adalimumab every other week

In one embodiment, the invention provides an article of manufacture comprising a TNFα inhibitor and a package insert, wherein the package insert indicates that in patients with Crohn's disease who have been administered the TNFα inhibitor, the loading dose on week 0 followed by a second dose on week 2 achieves serum adalimumab trough concentrations of approximately 12 µg/mL.

In one embodiment, an article of manufacture comprising a TNFα inhibitor and a package insert, wherein the package insert indicates that in patients with Crohn's disease who have been administered the TNFα inhibitor, the mean steady-state trough levels of approximately 7 µg/mL were observed in Crohn's disease patients who received a maintenance dose of the TNFα inhibitor every other week.

The label of the invention may also contain information regarding drug interactions of the TNFα inhibitor, e.g., a TNFα antibody such as adalimumab, with other drugs. In one embodiment, the label indicates that methotrexate (MTX) reduced adalimumab apparent clearance after single and multiple dosing by 29% and 44% respectively, in patients with rheumatoid arthritis.

In one embodiment of the invention, the kit comprises a TNFα inhibitor, such as an antibody, an second pharmaceutical composition comprising an additional therapeutic agent, and instructions for administration of both agents for the treatment of Crohn's disease. The instructions may describe how, *e.g.,* subcutaneously, and when, e.g., at week 0, week 2, and biweekly thereafter, doses of TNFα antibody and/or the additional therapeutic agent shall be administered to a subject for treatment.

Another aspect of the invention pertains to kits containing a pharmaceutical composition comprising an anti-TNFα antibody and a pharmaceutically acceptable carrier and one or more additional pharmaceutical compositions each comprising a drug useful for treating a TNFα related disorder and a pharmaceutically acceptable carrier. Alternatively, the kit comprises a single pharmaceutical composition comprising an anti-TNFα antibody, one or more drugs useful for treating a TNFα related disorder and a pharmaceutically acceptable carrier. The kits further contain instructions for dosing of the pharmaceutical compositions for the treatment of a TNFα related disorder.

The package or kit alternatively may contain the TNFα inhibitor and it may be promoted for use, either within the package or through accompanying information, for the uses or treatment of the disorders described herein. The packaged pharmaceuticals or kits further can include a second agent (as described herein) packaged with or copromoted with instructions for using the second agent with a first agent (as described herein).

### Additional therapeutic agents

TNFα inhibitors, including TNFα antibodies, or antigen binding portions thereof, may be used in the methods, uses, and compositions of the invention either alone or in combination with an additional therapeutic agent. It should be understood that the TNFα inhibitors can be used alone or in combination with an additional agent, e.g., a therapeutic agent, said additional agent being selected by the skilled artisan for its intended purpose. For example, the additional agent can be a therapeutic agent art-recognized as being useful to treat the disease or condition being treated by the TNFα inhibitors. The additional agent also can be an agent that imparts a beneficial attribute to the therapeutic composition e.g., an agent which effects the viscosity of the composition.

It should further be understood that the combinations which are to be included within this invention are those combinations useful for their intended purpose. The agents set forth below are illustrative for purposes and not intended to be limited. The combinations, which are part of this invention, can be the TNFα inhibitors of the present invention and at least one additional agent selected from the lists below. The combination can also include more than one additional agent, e.g., two or three additional agents if the combination is such that the formed composition can perform its intended function.

TNFα inhibitors described herein may be used in combination with additional therapeutic agents such as a Disease Modifying Anti-Rheumatic Drug (DMARD) or a Nonsteroidal Antiinflammatory Drug (NSAID) or a steroid or any combination thereof. Preferred examples of a DMARD are hydroxychloroquine, leflunomide, methotrexate, parenteral gold, oral gold and sulfasalazine. Preferred examples of non-steroidal antiinflammatory drug(s) also referred to as NSAIDS include drugs like ibuprofen. Other preferred combinations are corticosteroids including prednisolone; the well known side effects of steroid use can be reduced or even eliminated by tapering the steroid dose required when treating patients in combination with TNFα inhibitors of this invention.

Preferred combinations of therapeutic agents may interfere at different points in the autoimmune and subsequent inflammatory cascade; preferred examples include TNF antagonists such as soluble p55 or p75 TNF receptors, derivatives, thereof, (p75TNFR1gG (Enbrel™) or p55TNFR1gG (Lenercept), chimeric, humanized or human TNF antibodies, or a fragment thereof, including infliximab (Remicade^{®}, Johnson and Johnson; described in U.S. Patent No. 5,656,272, incorporated by reference herein), CDP571 (a humanized monoclonal anti-TNF-alpha IgG4 antibody), CDP 870 (a humanized monoclonal anti-TNF-alpha antibody fragment), an anti-TNF dAb (Peptech), CNTO 148 (golimumab; Medarex and Centocor, see WO 02/12502), and adalimumab (HUMIRA®^{®} Abbott Laboratories, a human anti-TNF mAb, described in US 6,090,382 as D2E7). Additional TNF antibodies which can be used in the invention are described in U.S. Patent Nos. 6,593,458; 6,498,237; 6,451,983; and 6,448,380, each of which is incorporated by reference herein. Other combinations including TNFα converting enzyme (TACE) inhibitors; IL-1 inhibitors (Interleukin-1-converting enzyme inhibitors, IL-1RA etc.) may be effective for the same reason. Other preferred combinations include Interleukin 11. Yet another preferred combination are other key players of the autoimmune response which may act parallel to, dependent on or in concert with TNFα inhibitors function; especially preferred are IL-18 antagonists including IL-18 antibodies or soluble IL-18 receptors, or IL-18 binding proteins. Yet another preferred combination are non-depleting anti-CD4 inhibitors. Yet other preferred combinations include antagonists of the co-stimulatory pathway CD80 (B7.1) or CD86 (B7.2) including antibodies, soluble receptors or antagonistic ligands.

The TNFα inhibitors used in the invention may also be combined with agents, such as methotrexate, 6-MP, azathioprine sulphasalazine, mesalazine, olsalazine chloroquinine/hydroxychloroquine, pencillamine, aurothiomalate (intramuscular and oral), azathioprine, cochicine, corticosteroids (oral, inhaled and local injection), beta-2 adrenoreceptor agonists (salbutamol, terbutaline, salmeteral), xanthines (theophylline, aminophylline), cromoglycate, nedocromil, ketotifen, ipratropium and oxitropium, cyclosporin, FK506, rapamycin, mycophenolate mofetil, leflunomide, NSAIDs, for example, ibuprofen, corticosteroids such as prednisolone, phosphodiesterase inhibitors, adensosine agonists, antithrombotic agents, complement inhibitors, adrenergic agents, agents which interfere with signalling by proinflammatory cytokines such as TNFα or IL-1 (e.g. IRAK, NIK, IKK, p38 or MAP kinase inhibitors), IL-1β converting enzyme inhibitors, TNFα converting enzyme (TACE) inhibitors, T-cell signalling inhibitors such as kinase inhibitors, metalloproteinase inhibitors, sulfasalazine, azathioprine, 6-mercaptopurines, angiotensin converting enzyme inhibitors, soluble cytokine receptors and derivatives thereof (e.g. soluble p55 or p75 TNF receptors and the derivatives p75TNFRIgG (Enbrel™ and p55TNFRIgG (Lenercept)), sIL-1RI, sIL-1RII, sIL-6R), antiinflammatory cytokines (e.g. IL-4, IL-10, IL-11, IL-13 and TGFβ), celecoxib, folic acid, hydroxychloroquine sulfate, rofecoxib, etanercept, infliximab, naproxen, valdecoxib, sulfasalazine, methylprednisolone, meloxicam, methylprednisolone acetate, gold sodium thiomalate, aspirin, triamcinolone acetonide, propoxyphene napsylate/apap, folate, nabumetone, diclofenac, piroxicam, etodolac, diclofenac sodium, oxaprozin, oxycodone hcl, hydrocodone bitartrate/apap, diclofenac sodium/misoprostol, fentanyl, anakinra, human recombinant, tramadol hcl, salsalate, sulindac, cyanocobalamin/fa/pyridoxine, acetaminophen, alendronate sodium, prednisolone, morphine sulfate, lidocaine hydrochloride, indomethacin, glucosamine sulf/chondroitin, amitriptyline hcl, sulfadiazine, oxycodone hcl/acetaminophen, olopatadine hcl, misoprostol, naproxen sodium, omeprazole, cyclophosphamide, rituximab, IL-1 TRAP, MRA, CTLA4-IG, IL-18 BP, anti-IL-18, Anti-IL15, BIRB-796, SCIO-469, VX-702, AMG-548, VX-740, Roflumilast, IC-485, CDC-801, and Mesopram.

Non-limiting examples of therapeutic agents for Crohn's disease with which TNFα inhibitor of the invention can be combined include the following: budenoside; epidermal growth factor; corticosteroids; cyclosporin, sulfasalazine; aminosalicylates; 6-mercaptopurine; azathioprine; metronidazole; lipoxygenase inhibitors; mesalamine; olsalazine; balsalazide; antioxidants; thromboxane inhibitors; IL-1 receptor antagonists; anti-IL-1β monoclonal antibodies; anti-IL-6 monoclonal antibodies; growth factors; elastase inhibitors; pyridinyl-imidazole compounds; antibodies to or antagonists of other human cytokines or growth factors, for example, TNF, LT, IL-1, IL-2, IL-6 (including Actemra (tocilizumab), IL-7, JL-8, IL-15, IL-16, IL-17, IL-18, EMAP-II, GM-CSF, FGF, and PDGF. Antibodies of the invention, or antigen binding portions thereof, can be combined with antibodies to cell surface molecules such as CD2, CD3, CD4, CD8, CD25, CD28, CD30, CD40, CD45, CD69, CD90 or their ligands. The antibodies of the invention, or antigen binding portions thereof, may also be combined with agents, such as methotrexate, cyclosporin, FK506, rapamycin, mycophenolate mofetil, laflunomide, NSAIDs, for example, ibuprofen, corticosteroids such as prednisolone, phosphodiesterase inhibitors, adenosine agonists, antithrombotic agents, complement inhibitors, adrenergic agents, agents which interfere with signalling by proinflammatory cytokines such as TNFα or IL-1 (e.g. IRAK, NIK, IKK, p38 or MAP kinase inhibitors), IL-1β converting enzyme inhibitors, TNFα converting enzyme inhibitors, T-cell signalling inhibitors such as kinase inhibitors, metalloproteinase inhibitors, sulfasalazine, azathioprine, 6-mercaptopurines, angiotensin converting enzyme inhibitors, soluble cytokine receptors and derivatives thereof (e.g. soluble p55 or p75 TNF receptors, sIL-1KI, sIL-1RII, sIL-6R) and antiinflammatory cytokines (e.g. IL-4, IL-10, IL-11, IL-13 and TGFβ).

Additional examples of therapeutic agents for Crohn's disease in which a TNFα inhibitor can be combined include the following: combinations of TNF antagonists, for example, anti-TNF antibodies, D2E7 (PCT Publication No. WO 97/29131; HUMIRA®), CA2 (REMICADE), CDP 571, TNFR-Ig constructs, (p75TNFRIgG (ENBREL) and p55TNFRIgG (LENERCEPT)) inhibitors and PDE4 inhibitors. TNFα inhibitors of the invention can be combined with corticosteroids, for example, budenoside and dexamethasone. TNFα inhibitors of the invention may also be combined with agents such as sulfasalazine, 5-aminosalicylic acid and olsalazine, and agents which interfere with synthesis or action of proinflammatory cytokines such as IL-1, for example, IL-1β converting enzyme inhibitors and IL-1ra. TNFα inhibitors may also be used with T cell signaling inhibitors, for example, tyrosine kinase inhibitors 6-anercaptopurines. TNFα inhibitors can be combined with IL-11. TNFα inhibitors can be combined with mesalamine, prednisone, azathioprine, mercaptopurine, infliximab, methylprednisolone sodium succinate, diphenoxylate/atrop sulfate, loperamide hydrochloride, methotrexate, omeprazole, folate, ciprofloxacin/dextrose-water, hydrocodone bitartrate/apap, tetracycline hydrochloride, fluocinonide, metronidazole, thimerosal/boric acid, cholestyramine/sucrose, ciprofloxacin hydrochloride, hyoscyamine sulfate, meperidine hydrochloride, midazolam hydrochloride, oxycodone hcl/acetaminophen, promethazine hydrochloride, sodium phosphate, sulfamethoxazole/trimethoprim, celecoxib, polycarbophil, propoxyphene napsylate, hydrocortisone, multivitamins, balsalazide disodium, codeine phosphate/apap, colesevelam hcl, cyanocobalamin, folic acid, levofloxacin, methylprednisolone, natalizumab and interferon-gamma

The TNFα inhibitors may also be combined with agents, such as alemtuzumab, dronabinol, Unimed, daclizumab, mitoxantrone, xaliproden hydrochloride, fampridine, glatiramer acetate, natalizumab, sinnabidol, a-immunokine NNSO3, ABR-215062, AnergiX.MS, chemokine receptor antagonists, BBR-2778, calagualine, CPI-1189, LEM (liposome encapsulated mitoxantrone), THC.CBD (cannabinoid agonist) MBP-8298, mesopram (PDE4 inhibitor), MNA-715, anti-IL-6 receptor antibody, neurovax, pirfenidone allotrap 1258 (RDP-1258), sTNF-R1, talampanel, teriflunomide,TGF-beta2, tiplimotide, VLA-4 antagonists (for example, TR-14035, VLA4 Ultrahaler, Antegran-ELAN/Biogen), interferon gamma antagonists, IL-4 agonists, and the humanized IL-6 antibody tocilizumab.

In yet another embodiment, the invention includes an article of manufacture or a method comprising the combination of a TNF inhibitor and an antibiotic or antiinfective agent. Antiinfective agents include those agents known in the art to treat viral, fungal, parasitic or bacterial infections. The term, "antibiotic," as used herein, refers to a chemical substance that inhibits the growth of, or kills, microorganisms. Encompassed by this term are antibiotic produced by a microorganism, as well as synthetic antibiotics (*e.g.*, analogs) known in the art. Antibiotics include, but are not limited to, clarithromycin (Biaxin^{®}), ciprofloxacin (Cipro^{®}), and metronidazole (Flagyl^{®}).

Any one of the above-mentioned therapeutic agents, alone or in combination therewith, can be administered to a subject suffering from a TNFα-related disorder in which TNFα is detrimental, in combination with the TNFα antibody using a multiple variable dose treatment regimen. In one embodiment, any one of the above-mentioned therapeutic agents, alone or in combination therewith, can be administered to a subject suffering from an intestinal disorder in addition to a TNFα antibody to treat another TNFα-related disorder, such as rheumatoid arthritis. It should be understood that the additional therapeutic agents can be used in combination therapy as described above, but also may be used in other indications described herein wherein a beneficial effect is desired.

The combination of agents used within the methods and pharmaceutical compositions described herein may have a therapeutic additive or synergistic effect on the condition(s) or disease(s) targeted for treatment. The combination of agents used within the methods or pharmaceutical compositions described herein also may reduce a detrimental effect associated with at least one of the agents when administered alone or without the other agent(s) of the particular pharmaceutical composition. For example, the toxicity of side effects of one agent may be attenuated by another agent of the composition, thus allowing a higher dosage, improving patient compliance, and improving therapeutic outcome. The additive or synergistic effects, benefits, and advantages of the compositions apply to classes of therapeutic agents, either structural or functional classes, or to individual compounds themselves.

### IV. Efficacy of TNFα inhibitor

The invention also provides methods for determining whether a TNFα inhibitor is effective at treating Crohn's disease in a subject. Such methods may be used to determine the efficacy of a TNFα inhibitor, including those which are unknown or unconfirmed to have such efficacy. Using the methods described herein, effective TNFα inhibitors may be determined or confirmed, and, subsequently, used in the method of treating Crohn's disease. Further methods for determining whether a TNFα inhibitor is effective at treating Crohn's disease in a subject are described in U.S. Provisional Application Nos. 60/849967 (filed October 6, 2006), 60/904626 (filed March 1, 2007) and 60/849967 (filed March 14, 2007), each of which are incorporated herein by reference.

In one embodiment, the invention provides a method for determining the efficacy of a TNFα inhibitor, including a human TNFα antibody, for maintaining remission of Crohn's disease in a subject, using the Crohn's Disease Activity Index (CDAI). The CDAI was developed to provide a single index of degree of illness in Crohn's disease, where index values of 150 and below are associated with quiescent disease; values above 150 indicate active disease, and values above 450 are seen with extremely severe disease (see Best et al. Gastroenterology. 1976 Mar;70(3):439-44). The CDAI may be used as an index for measuring efficacy of a TNFα inhibitor in a patient population having Crohn's disease, where attaining a certain percentage of patients within a population who were administered the TNFα inhibitor and who maintain clinical remission, i.e. CDAI < 150, indicates that the TNFα inhibitor is effective for maintaining remission of Crohn's disease. In one embodiment, the invention provides a method for determining whether a human TNFα antibody is effective for maintaining remission of Crohn's disease.

The efficacy of a TNFα inhibitor for maintaining remission of Crohn's disease in a patient population who has achieved remission, i.e., CDAI < 150 (also referred to herein as a CDAI / CDAI score of less than 150), may be evaluated by determining the percentage of the patient population in whom remission of Crohn's disease has been induced following administration of the TNFα inhibitor.

In one embodiment, the invention provides a method of determining the efficacy of a TNFα inhibitor for maintaining remission of Crohn's disease in a subject comprising determining a Crohn's Disease Activity Index (CDAI) score of a patient population having Crohn's disease and who was administered the TNFα inhibitor, wherein a CDAI score of less than 150 maintained in at least about 49% of the patient population indicates that the TNFα inhibitor is an effective TNFα inhibitor for the treatment of Crohn's disease in a subject, including, but not limited to, maintenance of remission of Crohn's disease. In one embodiment, the method further comprises administering the effective TNFα inhibitor to a subject to maintain remission of Crohn's disease. The invention provides a method of maintaining remission of Crohn's disease in a subject comprising administering an effective amount of a TNFα inhibitor to the subject such that remission of Crohn's disease is maintained, wherein the effective amount of the TNFα inhibitor was previously identified as maintaining a CDAI score of less than 150 in at least about 49% of a patient population having Crohn's disease.

In one embodiment the invention provides a method of determining the efficacy of a TNFα inhibitor for maintaining remission of Crohn's disease in a subject comprising determining a Crohn's Disease Activity Index (CDAI) score of a patient population having Crohn's disease and who was administered more than one maintenance dose the TNFα inhibitor, wherein a CDAI score of less than 150 maintained in at least about 47% of the patient population indicates that the TNFα inhibitor is an effective TNFα inhibitor for the treatment of Crohn's disease in a subject.

In one embodiment the invention provides a method of determining the efficacy of a TNFα inhibitor for maintaining remission of Crohn's disease in a subject comprising determining a Crohn's Disease Activity Index (CDAI) score of a patient population having Crohn's disease and who was administered a human TNFα antibody, or antigen-binding portion thereof, wherein a CDAI score of less than 150 maintained in at least about 32% of the patient population indicates that the TNFα inhibitor is an effective TNFα inhibitor for the treatment of Crohn's disease in a subject.

In one embodiment, a CDAI score of less than 150 maintained in at least about 32% of the patient population indicates that the TNFα inhibitor is an effective TNFα inhibitor for the treatment of Crohn's disease in a subject. In one embodiment, a CDAI score of less than 150 maintained in at least about 36% of the patient population indicates that the TNFα inhibitor is an effective TNFα inhibitor for the treatment of Crohn's disease in a subject. In one embodiment, a CDAI score of less than 150 maintained in at least about 40% of the patient population indicates that the TNFα inhibitor is an effective TNFα inhibitor for the treatment of Crohn's disease in a subject. In one embodiment, a CDAI score of less than 150 maintained in at least about 41% of the patient population indicates that the TNFα inhibitor is an effective TNFα inhibitor for the treatment of Crohn's disease in a subject. In one embodiment, a CDAI score of less than 150 maintained in at least about 46% of the patient population indicates that the TNFα inhibitor is an effective TNFα inhibitor for the treatment of Crohn's disease in a subject. In one embodiment, a CDAI score of less than 150 maintained in at least about 47% of the patient population indicates that the TNFα inhibitor is an effective TNFα inhibitor for the treatment of Crohn's disease in a subject. In one embodiment, a CDAI score of less than 150 maintained in at least about 50% of the patient population indicates that the TNFα inhibitor is an effective TNFα inhibitor for the treatment of Crohn's disease in a subject. In one embodiment, a CDAI score of less than 150 maintained in at least about 60% of the patient population indicates that the TNFα inhibitor is an effective TNFα inhibitor for the treatment of Crohn's disease in a subject. In one embodiment, a CDAI score of less than 150 maintained in at least about 67% of the patient population indicates that the TNFα inhibitor is an effective TNFα inhibitor for the treatment of Crohn's disease in a subject. In one embodiment, a CDAI score of less than 150 maintained in at least about 70% of the patient population indicates that the TNFα inhibitor is an effective TNFα inhibitor for the treatment of Crohn's disease in a subject. In one embodiment, a CDAI score of less than 150 maintained in at least about 74% of the patient population indicates that the TNFα inhibitor is an effective TNFα inhibitor for the treatment of Crohn's disease in a subject. In one embodiment, a CDAI score of less than 150 maintained in at least about 79% of the patient population indicates that the TNFα inhibitor is an effective TNFα inhibitor for the treatment of Crohn's disease in a subject. In one embodiment, a CDAI score of less than 150 maintained in at least about 80% of the patient population indicates that the TNFα inhibitor is an effective TNFα inhibitor for the treatment of Crohn's disease in a subject. In one embodiment, a CDAI score of less than 150 maintained in at least about 83% of the patient population indicates that the TNFα inhibitor is an effective TNFα inhibitor for the treatment of Crohn's disease in a subject. In one embodiment, a CDAI score of less than 150 maintained in at least about 84% of the patient population indicates that the TNFα inhibitor is an effective TNFα inhibitor for the treatment of Crohn's disease in a subject. In one embodiment, a CDAI score of less than 150 maintained in at least about 85% of the patient population indicates that the TNFα inhibitor is an effective TNFα inhibitor for the treatment of Crohn's disease in a subject. In one embodiment, a CDAI score of less than 150 maintained in at least about 90% of the patient population indicates that the TNFα inhibitor is an effective TNFα inhibitor for the treatment of Crohn's disease in a subject. In one embodiment, a CDAI score of less than 150 maintained in at least about 94% of the patient population indicates that the TNFα inhibitor is an effective TNFα inhibitor for the treatment of Crohn's disease in a subject. In one embodiment, the CDAI improvement and/or maintenance of remission indicated by a CDAI score of less than 150 is found in at least about 32% of the patient population. In another embodiment, at least about 36%. In another embodiment, at least about 40%. In another embodiment, at least about 41%. In another embodiment, at least about 46%. In another embodiment, at least about 47%. In another embodiment, at least about 50%. In another embodiment, at least about 60%. In another embodiment, at least about 67%. In another embodiment, at least about 70%. In another embodiment, at least about 74%. In another embodiment, at least about 79%. In another embodiment, at least about 80%. In another embodiment, at least about 83%. In another embodiment, at least about 84%. In another embodiment, at least about 85%. In another embodiment, at least about 90%. In another embodiment, at least about 94% .

Numbers intermediate to the above recited percentages, *e.g.,* 37%, 38%, 39%. 43%, 44%, 45%, 48%, 49%, 52%, 53%, 54%, 55%, 56%, 57%, 58%, 59%, 61%, 62%, 63%, 64%, 65%, 66%, 68%, 69%, 71%, 72%, 73%, 75%, 76%, 77%, 78%, 81%, 82%, 86%, 87%, 88%, 89%, 91%, 92%, 93%, as well as all other numbers recited herein, are also intended to be part of this invention. Ranges of values using a combination of any of the above recited values as upper and/or lower limits are intended to be included in the scope of the invention. For example, in one embodiment a CDAI score of less than 150 maintained in at least between 47% and 79% of the patient population indicates that the TNFα inhibitor is an effective TNFα inhibitor for the treatment of Crohn's disease in a subject.

In one embodiment, the invention provides a method of determining the efficacy of TNFα inhibitor, e.g., a human TNFα antibody, or antigen-binding portion thereof, for achieving a clinical response in Crohn's disease in a subject comprising determining a Crohn's Disease Activity Index (CDAI) score of a patient population having Crohn's disease and who was administered the TNFα inhibitor, wherein a decrease of at least 100 in the CDAI score of at least about 47% of the patient population indicates that the TNFα inhibitor is an effective TNFα inhibitor for achieving a clinical response in Crohn's disease in a subject. In one embodiment, the method further comprises administering the effective TNFα inhibitor to a subject to achieve a clinical response in Crohn's disease. The invention also provides a method of achieving a clinical response in Crohn's disease in a subject comprising administering an effective amount of a TNFα inhibitor to the subject such that a clinical response in Crohn's disease is achieved, wherein the effective amount of the TNFα inhibitor was previously identified as decreasing a CDAI score by at least 100 in at least about 47% of a patient population having Crohn's disease.

The invention also provides a method of determining the efficacy of a TNFα inhibitor, e.g., a human TNFα antibody, or antigen-binding portion thereof, for maintaining remission of Crohn's disease comprising determining a Crohn's Disease Activity Index (CDAI) score of a patient population having Crohn's disease and who was administered the TNFα inhibitor, wherein Δ 100, i.e., a decrease of at least 100 in the CDAI score, in at least about 41 % of the patient population indicates that the TNFα inhibitor is effective for maintaining remission of Crohn's disease.

In one embodiment, the invention provides a method of determining the efficacy of a TNFα inhibitor for achieving a clinical response in Crohn's disease in a subject comprising determining a Crohn's Disease Activity Index (CDAI) score of a patient population having Crohn's disease and who was administered the TNFα inhibitor, wherein a decrease of at least 100 in the CDAI score of at least about 47% of the patient population indicates that the TNFα inhibitor is an effective TNFα inhibitor for achieving a clinical response in Crohn's disease in a subject.

In one embodiment, the invention provides a method of determining the efficacy of a TNFα inhibitor for achieving a clinical response in Crohn's disease in a subject who has not received infliximab comprising determining a Crohn's Disease Activity Index (CDAI) score of a patient population having Crohn's disease and who was administered the TNFα inhibitor, wherein a decrease of at least 100 in the CDAI score of at least about 41% of the patient population indicates that the TNFα inhibitor is an effective TNFα inhibitor for achieving a clinical response in Crohn's disease in a subject in a subject who has net received infliximab.

In one embodiment, a decrease of at least 100 in the CDAI score of at least about 41% of the patient population indicates that the TNFα inhibitor is an effective TNFα inhibitor for achieving a clinical response in Crohn's disease in a subject. In one embodiment, a decrease of at least 100 in the CDAI score of at least about 48% of the patient population indicates that the TNFα inhibitor is an effective TNFα inhibitor for achieving a clinical response in Crohn's disease in a subject. In one embodiment, a decrease of at least 100 in the CDAI score of at least about 50% of the patient population indicates that the TNFα inhibitor is an effective TNFα inhibitor for achieving a clinical response in Crohn's disease in a subject. In one embodiment, a decrease of at least 100 in the CDAI score of at least about 52% of the patient population indicates that the TNFα inhibitor is an effective TNFα inhibitor for achieving a clinical response in Crohn's disease in a subject. In one embodiment, a decrease of at least 100 in the CDAI score of at least about 60% of the patient population indicates that the TNFα inhibitor is an effective TNFα inhibitor for achieving a clinical response in Crohn's disease in a subject. In one embodiment, a decrease of at least 100 in the CDAI score of at least about 64% of the patient population indicates that the TNFα inhibitor is an effective TNFα inhibitor for achieving a clinical response in Crohn's disease in a subject. In one embodiment, a decrease of at least 100 in the CDAI score of at least about 67% of the patient population indicates that the TNFα inhibitor is an effective TNFα inhibitor for achieving a clinical response in Crohn's disease in a subject. In one embodiment, a decrease of at least 100 in the CDAI score of at least about 70% of the patient population indicates that the TNFα inhibitor is an effective TNFα inhibitor for achieving a clinical response in Crohn's disease in a subject. In one embodiment, a decrease of at least 100 in the CDAI score of at least about 74% of the patient population indicates that the TNFα inhibitor is an effective TNFα inhibitor for achieving a clinical response in Crohn's disease in a subject. In one embodiment, a decrease of at least 100 in the CDAI score of at least about 79% of the patient population indicates that the TNFα inhibitor is an effective TNFα inhibitor for achieving a clinical response in Crohn's disease in a subject. In one embodiment, a decrease of at least 100 in the CDAI score of at least about 80% of the patient population indicates that the TNFα inhibitor is an effective TNFα inhibitor for achieving a clinical response in Crohn's disease in a subject. In one embodiment, a decrease of at least 100 in the CDAI score of at least about 83% of the patient population indicates that the TNFα inhibitor is an effective TNFα inhibitor for achieving a clinical response in Crohn's disease in a subject. In one embodiment, a decrease of at least 100 in the CDAI score of at least about 84% of the patient population indicates that the TNFα inhibitor is an effective TNFα inhibitor for achieving a clinical response in Crohn's disease in a subject. In one embodiment, a decrease of at least 100 in the CDAI score of at least about 85% of the patient population indicates that the TNFα inhibitor is an effective TNFα inhibitor for achieving a clinical response in Crohn's disease in a subject. In one embodiment, a decrease of at least 100 in the CDAI score of at least about 89% of the patient population indicates that the TNFα inhibitor is an effective TNFα inhibitor for achieving a clinical response in Crohn's disease in a subject. In one embodiment, a decrease of at least 100 in the CDAI score of at least about 90% of the patient population indicates that the TNFα inhibitor is an effective TNFα inhibitor for achieving a clinical response in Crohn's disease in a subject. In one embodiment, a decrease of at least 100 in the CDAI score of at least about 94% of the patient population indicates that the TNFα inhibitor is an effective TNFα inhibitor for achieving a clinical response in Crohn's disease in a subject. In one embodiment, a decrease of at least 100 in the CDAI score of at least about 41% of the patient population indicates that the TNFα inhibitor is an effective TNFα inhibitor for achieving a clinical response in Crohn's disease in a subject. In another embodiment, the percentage is at least about 45%. In another embodiment, at least about 48%. In another embodiment, at least about 50%. In another embodiment, at least about 52%. In another embodiment, at least about 60%. In another embodiment, at least about 64%. In another embodiment, at least about 67%. In another embodiment, at least about 70%. In another embodiment, at least about 74%. In another embodiment, at least about 79%, In another embodiment, at least about 80 %. In another embodiment, at least about 89%. In another embodiment, at least about 90%. In another embodiment, at least about 95%. In another embodiment, at least about 100%.

Numbers intermediate to the above recited percentages, *e.g.,* 42%, 43%, 44%, 45%, 46%, 47%, 49%, 51%, 53%, 54%, 55%, 56%, 57%, 58%, 59%, 61%, 62%, 63%, 65%, 66%, 68%, 69%, 70%, 71%, 72%, 73%, 75%, 76%, 77%, 78%, 81%, 82%, 86%, 87%, 88%, 91%, 92%, 93% are also intended to be part ofthis invention. Ranges of values using a combination of any of the above recited values as upper and/or lower limits are intended to be included in the scope of the invention. For example in one embodiment a decrease of at least 100 in the CDAI score of at least between 52% - 74% of the patient population indicates that the TNFα inhibitor is an effective TNFα inhibitor for achieving a clinical response in Crohn's disease in a subject.

In one embodiment, the invention provides a method of determining the efficacy of a TNFα inhibitor, *e.g.*, a human TNFα antibody, or antigen-binding portion thereof, for achieving a clinical response in Crohn's disease in a subject comprising determining a Crohn's Disease Activity Index (CDAI) score of a patient population having Crohn's disease and who was administered the TNFα inhibitor, wherein a decrease of at least 70 in the CDAI score of at least about 43% of the patient population indicates that the TNFα inhibitor is an effective TNFα inhibitor for achieving a clinical response in Crohn's disease in a subject. In one embodiment, the invention further comprises administering the effective TNFα inhibitor to a subject in need thereof. In one embodiment, the invention provides a method of achieving a clinical response in Crohn's disease in a subject comprising administering an effective amount of a TNFα inhibitor to the subject such that a clinical response in Crohn's disease is achieved, wherein the effective amount of the TNFα inhibitor was previously identified as decreasing a CDAI score by at least 70 in at least about 43% of a patient population having Crohn's disease.

In one embodiment, a decrease of at least 70 in the CDAI score of at least about 49% of the patient population indicates that the TNFα inhibitor is an effective TNFα inhibitor for achieving a clinical response in Crohn's disease in a subject. In one embodiment, a decrease of at least 70 in the CDAI score of at least about 50% of the patient population indicates that the TNFα inhibitor is an effective TNFα inhibitor for achieving a clinical response in Crohn's disease in a subject. In one embodiment, a decrease of at least 70 in the CDAI score of at least about 54% of the patient population indicates that the TNFα inhibitor is an effective TNFα inhibitor for achieving a clinical response in Crohn's disease in a subject. In one embodiment, a decrease of at least 70 in the CDAI score of at least about 56% of the patient population indicates that the TNFα inhibitor is an effective TNFα inhibitor for achieving a clinical response in Crohn's disease in a subject. In one embodiment, a decrease of at least 70 in the CDAI score of at least about 58% of the patient population indicates that the TNFα inhibitor is an effective TNFα inhibitor for achieving a clinical response in Crohn's disease in a subject. In one embodiment, a decrease of at least 70 in the CDAI score of at least about 60% of the patient population indicates that the TNFα inhibitor is an effective TNFα inhibitor for achieving a clinical response in Crohn's disease in a subject. In one embodiment, a decrease of at least 70 in the CDAI score of at least about 70% of the patient population indicates that the TNFα inhibitor is an effective TNFα inhibitor for achieving a clinical response in Crohn's disease in a subject. In one embodiment, a decrease of at least 70 in the CDAI score of at least about 80% of the patient population indicates that the TNFα inhibitor is an effective TNFα inhibitor for achieving a clinical response in Crohn's disease in a subject. In one embodiment, a decrease of at least 70 in the CDAI score of at least about 90% of the patient population indicates that the TNFα inhibitor is an effective TNFα inhibitor for achieving a clinical response in Crohn's disease in a subject. In one embodiment, a decrease of at least 70 in the CDAI score of at least about 43% of the patient population indicates that the TNFα inhibitor is an effective TNFα inhibitor for achieving a clinical response in Crohn's disease in a subject. In another embodiment, the percent of the patient population is about 49%. In another embodiment, at least about 49%, In another embodiment, at least about 54%. In another embodiment, at least about 56%. In another embodiment, at least about 58%.

Numbers intermediate to the above recited percentages, e.g., 44%, 45%, 46%, 47%, 49%, 51%, 52%, 53%, 55%, 57%, 59%, 61%, 62%, 63%. 64%, 65%, 66%, 67%, 68%, 69%, 71%, 72%, 73%, 74%, 75%, 76%, 77%, 78%, 79%, 81%, 82%, 83%, 84%, 85%, 86%, 87%, 88%, 89% are also intended to be part of this invention. Ranges of values using a combination of any of the above recited values as upper and/or lower limits are intended to be included in the scope of the invention. For example in one embodiment a decrease of at least 70 in the CDAI score of at least between 56% - 70% of the patient population indicates that the TNFα inhibitor is an effective TNFα inhibitor for achieving a clinical response in Crohn's disease in a subject.

The invention also provides a method of determining the efficacy of a TNFα inhibitor for treating Crohn's disease using an Inflammatory Bowel Disease Questionnaire (IBDQ) score of a patient population having Crohn's disease. The IBDQ, a 32-item questionnaire, was developed to provide a measure of health status for clinical trials in inflammatory bowel disease (see Guyatt et al. Gastroenterology. 1989; 96:804-10, the contents of which are expressly incorporated herein by reference). It evaluates quality of life with respect to bowel function (*e.g*. loose stools and abdominal pain), systemic symptoms (fatigue and altered sleep pattern), social function (work attendance and the need to cancel social events) and emotional status (angry, depressed, or irritable). The score ranges from 32 to 224, with higher scores indicating a better quality of life. In one embodiment, the methods further comprises administering the effective TNFα inhibitor to a subject having Crohn's disease.

In one embodiment, the invention provides a method of determining the efficacy of a TNFα inhibitor to maintain remission of Crohn's disease in a subject comprising determining an Inflammatory Bowel Disease Questionnaire (IBDQ) score of a patient population having Crohn's disease who was administered the TNFα inhibitor, wherein an IBDQ score greater than 170 in at least about 74% of the patient population indicates that the TNFα inhibitor is an effective TNFα inhibitor for maintaining remission of Crohn's disease in a subject.

The invention includes a method ofmaintaining remission of Crohn's disease in a subject comprising administering an effective amount of a TNFα inhibitor to the subject, such that remission of Crohn's disease is maintained, wherein the effective amount of the TNFα inhibitor was previously identified as maintaining an IBDQ score greater than 170 in at least about 74% of a patient population having Crohn's disease.

In one embodiment, an IBDQ score greater than 170 in at least about 40% of the patient population indicates that the TNFα inhibitor is an effective TNFα inhibitor for maintaining remission of Crohn's disease in a subject. In one embodiment, an IBDQ score greater than 170 in at least about 50% of the patient population indicates that the TNFα inhibitor is an effective TNFα inhibitor for maintaining remission of Crohn's disease in a subject. In one embodiment, an IBDQ score greater than 170 in at least about 60% of the patient population indicates that the TNFα inhibitor is an effective TNFα inhibitor for maintaining remission of Crohn's disease in a subject. In one embodiment, an IBDQ score greater than 170 in at least about 70% of the patient population indicates that the TNFα inhibitor is an effective TNFα inhibitor for maintaining remission of Crohn's disease in a subject. In one embodiment, an IBDQ score greater than 170 in at least about 80% of the patient population indicates that the TNFα inhibitor is an effective TNFα inhibitor for maintaining remission of Crohn's disease in a subject. In one embodiment, an IBDQ score greater than 170 in at least about 83% of the patient population indicates that the TNFα inhibitor is an effective TNFα inhibitor for maintaining remission of Crohn's disease in a subject. In one embodiment, an IBDQ score greater than 170 in at least about 74% indicates that the TNFα inhibitor is an effective TNFα inhibitor for maintaining remission of Crohn's disease in a subject. In another embodiment, at least about 76%. In another embodiment, at least about 78%. In another embodiment, at least about 80%, In another embodiment, at least about 83%.

Numbers intermediate to the above recited percentages, e.g., 41%, 42%, 44%, 46%, 47%, 48%, 49%, 51%, 52%, 53%, 54%, 55%, 56%, 57%, 58%, 59%, 61%, 62%, 63%. 64%, 65%, 66%, 67%, 68%, 69%, 71%, 72%, 73%, 74%, 75%, 76%, 77%, 78%, 79%, 81%, 82% are also intended to be part of this invention. Ranges of values using a combination of any of the above recited values as upper and/or lower limits are intended to be included in the scope of the invention. For example, in one embodiment an IBDQ score greater than 170 in at least between about 50%-83% of the patient population indicates that the TNFα inhibitor is an effective TNFα inhibitor for maintaining remission of Crohn's disease in a subject.

The invention includes a method of determining the efficacy of a human TNFα antibody, or antigen-binding portion thereof, for achieving a clinical response in Crohn's disease in a subject comprising determining the percentage complete fistula closing of a patient population having Crohn's disease and who was administered the human TNFα antibody, or antigen-binding portion thereof, wherein complete fistula closing observed in at least about 28% of the patient population indicates that the human TNFα antibody, or antigen-binding portion thereof, is an effective human TNFα antibody, or antigen-binding portion thereof, for achieving a clinical response in Crohn's disease in a subject. In one embodiment, the percentage complete fistula closing observed in a patient population is at least about 28%. In anther embodiment, at least about 30%. In another embodiment, at least about 33%. In another embodiment, at least about 35%. In anther embodiment, at least about 37% of the patient population.

It should be noted that the Examples provided herein represent different methods of determining the efficacy of a TNFα inhibitor, such as a human TNFα antibody, or antigen-binding portion thereof. As such, data and results described in the Examples section which shows efficacy of a TNFα. inhibitor, *e.g*, ability to maintain remission of Crohn's, are included in the methods of determining efficacy of the invention.

Time points for determining efficacy will be understood by those of skill in the art to depend on the type of efficacy being determined, *e.g,*, maintenance of remission. In one embodiment, measurements in scores, *e.g.,* a decrease in the CDAI score of a subject, may be measured against a subject's baseline score. Generally, a baseline refers to a measurement or score of a patient before treatment, *i.e.* week 0. Other time points may also be included as a starting point in determining efficacy, however, For example, in determining the efficacy of a TNFα inhibitor for maintaining remission of Crohn's disease in a patient population, a determination of the percentage of the patient population who maintained remission, *i,e*., CDAI score of less than 150, may be determined based on a time point from when remission was induced.

Patient populations described in the methods of the invention are generally selected based on common characteristics, such as, but not limited to, subjects diagnosed with Crohn's disease who are in remission as a result of being on a dosing regimen comprising a TNFα inhibitor. Such a patient population would be appropriate for determining the efficacy of the TNFα inhibitor for maintaining remission in Crohn's disease in the given patient population. In one embodiment, the patient population is an adult population, *.e.g,* older than 17 years of age or older than 18 years of age.

In one embodiment, the methods of the invention for determining whether a TNFα inhibitor is an effective TNFα inhibitor, include determining changes, improvements, measurements, etc., in Crohn's disease using appropriate indices known in the art, *e.g*., (CDAI, IBDQ, status of Crohn's related disorders, etc. from a patient population who has already been administered the TNFα inhibitor. Such a patient population may be pre-selected according to common characteristics, .*e.g*., Crohn's disease, loss of response to infliximab, and may have already been given the TNFα inhibitor. Administration of the TNFα inhibitor may or may not be performed by the same person of ordinary skill who is determining the efficacy of the TNFα inhibitor in accordance with the teachings of the specificaiton,

In one embodiment, the methods of the invention comprise administering the TNFα inhibitor to the subjects of a patient population and determining the efficacy of the TNFα inhibitor by determining changes, improvements, measurements, etc., using Crohn's indices known in the art, in the patient population in comparison to the Examples set forth below. For example, in one embodiment the invention includes a method for determining efficacy of a TNFα inhibitor for the treatment of Crohn's disease comprising administering the TNFα inhibitor to a preselected patient population having Crohn's disease; and determining the effectiveness of the TNFα inhibitor by using a mean baseline Crohn's Disease Activity Index (CDAI) score of the patient population and a mean CDAI score following administration of the TNFα inhibitor, wherein a CDAI<150 achieved in at least about 43% of the patient population indicates that the TNFα inhibitor is effective for the treatment of Crohn's disease. In another embodiment, the invention includes a method for determining efficacy of a TNFα inhibitor for maintaining remission of Crohn's disease comprising administering the TNFα inhibitor to a preselected patient population in remission of Crohn's disease; and determining the effectiveness of the TNFα inhibitor using a mean CDAI score following administration of the TNFα inhibitor on a maintenance therapy, wherein a CDAI<150 achieved in at least about 40% of the patient population indicates that the TNFα inhibitor is effective for the maintaining remission of Crohn's disease,

Methods of the invention relating to determining efficacy, *i.e*., determining whether a TNFα inhibitor is an effective TNFα inhibitor, may also be applied to specific patient populations within the overall patient population who together have specific, common characteristics, *i.e*., a subpopulation. For example, the patient population may comprise patients on concomitant immunosuppressant (IMM) treatment with the TNFα inhibitor. In another example, the patient population may comprises patients not on concomitant IMM treatment.

In addition, while the above methods are described in terms of patient populations, methods of efficacy described herein may also be applied to individual subjects. For example, a method for determining efficacy may comprise determining whether a subject who is in remission from Crohn's disease, and who is on a dosage regimen comprising a human TNFα antibody, is able to maintain a CDAI of less than 150 to determing if the human TNFα antibody is an effective human TNFα antibody. In one embodiment, if the subject is able to maintain remission of Crohn's disease for at least about 26 weeks, then the human TNFα antibody is effective at maintaining remission of Crohn's disease.

The Examples and discoveries described herein are representative of a TNFα inhibitor, *i.e*., adalimumab, which is effective for treating Crohn's disease, including inducing and maintaining remission of Crohn's. As such, the studies and results described in the Examples section herein may be used as a guideline for determining the efficacy of a TNFα inhibitor, *i.e.,* whether a TNFα inhibitor is an effective TNFα inhibitor for the treatment of Crohn's disease. In one embodiment, methods of determining efficacy described herein may be used to determine whether a TNFα inhibitor is bioequivalent to another TNFα inhibitor.

In one embodiment, the article of manufacture of the invention comprises instructions regarding how to determine the efficacy of the TNF inhibitor for the treatment of Crohn's disease.

The present invention is further illustrated by the following examples which should not be construed as limiting in any way.

### Example 1: Adalimumab Therapy in Patients With Crohn's Disease

Study E was a 4-week randomized controlled study of adalimumab in the induction of remission in patients with active Crohn's disease (CD). Immunosuppressant (IMM)-azathioprine, 6-MP, or methotrexate-use was permitted if patients entered the study on a stable IMM dose for 12 weeks prior to screening. IMM use did not influence the response to adalimumab in Study E.

One goal of the following study was to assess the effect of concomitant IMM on the efficacy of adalimumab over 1 year. Study F was also designed to evaluate adalimumab's ability to maintain clinical remission. The study design for Study F is shown in Figure 1.

Inclusion criteria for Study F included a diagnosis of CD for at least 4 months and moderately to severely active CD (CDAI 220-450). Stable doses of CD medications (steroids, immunosuppressive agents, aminosalicylates, antibiotics) were allowed. Patients also had to have completed the previous 4-week adalimumab Study E, in which patients were randomized to 1 of 4 treatments administered subcutaneously (sc) at Week 0 and Week 2. Participants in Study E had no previous exposure to TNF antagonists.

All patients in Study F completed Study E. All patients in Study F received adalimumab 40 mg sc at Weeks 0 (Week 4 of Study E) and 2. Depending on their clinical remission status at weeks 0 and 4, patients were assigned to one of two cohorts: randomize or open-label (see Figure 1). Patients with Crohn's Disease Activity Index (CDAI) ≥ 150 at Weeks 0 and/or 4 (non-remission) received open-label (OL) adalimumab 40 mg sc every other week (eow), weekly dosing was permitted for flare or persistent non-response. Patients with CDAI < 150 at both Weeks 0 and 4 were randomized to receive adalimumab, 40 mg sc eow or weekly, or placebo for up to 1 year. Patients not in remission at both Weeks 0 and 4 and patients who flared during the randomized portion of the study received open-label (OL) 40 mg eow for 52 weeks. CDAI was assessed at each study visit. Information on concomitant IMM use was collected at the start of the study through 56 weeks of treatment,

Patients who did not respond to or who flared with 40 mg eow treatment were treated with 40 mg weekly. Non-response was defined as a decrease in CDAI of less than 70 points at any visit when compared to the patient's CDAI score at Week 4 of Study F. A flare was defined as a recurrence of very active disease, CDAI>220, and an increase in CDAI of 70 or more points at any visit when compared to the patient's CDAI score at Week 4 of Study F. At Week 56, all patients received open-label therapy. An overview of the study design is shown in Figure 1.

Endpoints for this study included independent and combined effects of concomitant IMM and adalimumab on Crohn's Disease Activity Index (CDAI) remission and Δ100 clinical response. Remission was defined as CDAI<150 and clinical response was defined as a decrease in CDAI scores of at least 100 (Δ100) points. Thus, the relationship of IMM and adalimumab on remission (CDAI < 150) and Δ100 CDAI response (decrease in CDAI score ≥ 100 points) and their potential interaction was assessed.

276 of 284 patients who completed Study E entered Study F. Baseline demographic and disease characteristics were similar among treatment groups (shown below in Tables 1 and 2).

**Table 1: Baseline characteristics for Study F randomized and open-label cohorts**

| | Randomized | | | |
|---|---|---|---|---|
| Characteristic | Placebo | 40 mg EOW | 40 mg Weekly | Open-label |
| N | 18 | 19 | 18 | 221 |
| Mean age, yrs | 36 | 34 | 38 | 39 |
| % Male | 33 | 37 | 50 | 47 |
| % Caucasian | 94 | 90 | 83 | 90 |
| Mean weight, kg | 70 | 69 | 72 | 76 |
| Mean CDAI* | 107 | 106 | 88 | 246 |
| Mean IBDQ | 187 | 181 | 192 | 146 |
| % IMM+ | 17 | 21 | 28 | 33 |

| | | | | |
|---|---|---|---|---|
| Potential CDAI range (0 to over 600). Potential IBDQ range (32-224). *CDAI at Week 0 of Study E (range): Placebo group, n=74, mean CDAI=296 (216-437); all adalimumab groups, n=225, mean CDAI=298 (191-450) | | | | |

**Table 2: Patient disposition through week 56: Study F randomized and open-label cohorts**

| | Randomized | | | |
|---|---|---|---|---|
| | Placebo | 40 mg EOW | 40 mg Weekly | Open-label |
| N | 18 | 19 | 18 | 221 |
| Completed 56 weeks, n (%) | 13 (72) | 15 (79) | 16 (89) | 131 (59) |
| Withdrew | 5 | 4 | 2 | 90 |
| Reason for withdrawal | | | | |
| AE | 1 | 1 | 1 | 28 |
| Lost to follow-up | 0 | 1 | 0 | 27 |
| Lack of efficacy | 1 | 0 | 0 | 15 |
| Withdrew consent | 3 | 2 | 1 | 5 |
| Other | 0 | 0 | 0 | 15 |

Of 276 patients enrolled in Study F, 30% received concomitant IMM (IMM+). In the OL cohort (N=221), 46% of IMM+ patients achieved CDAI < 150 and 64% achieved Δ100 CDAI, versus 40% and 60% of those not receiving IMM (IMM-), respectively.

Results in the randomized cohort (N=55) are summarized in Table 3, below. At one year, 83% of the patients taking adalimumab weekly maintained clinical remission, versus 44% for placebo. Of patients taking adalimumab every other week, 74% maintained clinical remission vs. 44% of placebo. As shown below, more than 2/3 of patients treated with adalimumab in the randomized cohort maintained remission (CDAI<150) through Week 56-immunosuppressants had no significant impact on the maintenance of remission. IMM status did not notably influence the efficacy of adalimumab.

**Table 3. Week 56 Maintenance of Remission and Response: Study F Randomized Cohort Therapy**

| Therapy | | CDAI < 150 | | | Δ100 CDAI | | |
|---|---|---|---|---|---|---|---|
| | | Total | IMM+ | IMM- | Total | IMM+ | IMM- |
| Placebo | N | 8/18 | 1/3 | 7/15 | 10/18 | 1/3 | 9/15 |
| | % | 44 | 33 | 47 | 56 | 33 | 60 |
| 40 mg | N | 14/19 | 4/4 | 10/15 | 14/19 | 4/4 | 10/15 |
| eow | % | 74 | 100 | 67 | 74 | 100 | 67 |
| 40 mg | N | 15/18 | 4/5 | 11/13 | 16/18 | 5/5 | 11/13 |
| weekly | % | 83 | 80 | 85 | 89 | 100 | 85 |

Adalimumab was well-tolerated overall, as shown below in Tables 4 and 5:

**Table 4: Adverse events in Study F through week 56**

| | Randomized | | | |
|---|---|---|---|---|
| | Placebo | 40 mg EOW | 40 mg Weekly | Open-label |
| N | 18 | 19 | 18 | 221 |
| Any AE, n (%) AE's at least possibly | 18 (100) | 15 (79) | 13 (72) | 205 (93) |
| related, n (%) AE's leading to | 10 (56) | 8(42) | 3 (17) | 99 (45) |
| withdrawal, n (%) | 2 (11) | 1 (5) | 1 (6) | 36 (16) |

37 patients in the open-label cohort experienced 54 serious adverse events (SAE). 3 patients experienced SAE in the randomized cohort of Study F-2 patients who received placebo and 1 who received adalimumab 40 mg EOW.

**Table 5: Serious adverse events in Study F through week 56**

| Randomized | | | | | |
|---|---|---|---|---|---|
| | | Placebo | 40 mg EOW | 40 mg Weekly | Open-label |
| | N | 18 | 19 | 18 | 221 |

| Patients with Serious AE | | | | | |
|---|---|---|---|---|---|
| | Serious AE, n | 2 | 1 | 0 | 37 |
| | Deaths, n | 0 | 0 | 0 | 0 |

| Types of Serious AE | | | | | |
|---|---|---|---|---|---|
| | Infection | 0 | 0 | 0 | 12 |
| | Crohn's exacerbation Obstruction/ | 0 | 0 | 0 | 9 |
| | stricture/stenosis | 1 | 0 | 0 | 7 |
| | | | | | |
| | Ovarian cyst | 0 | 0 | 0 | 3* |
| | Other | 1† | 1† | 0 | 23† |
| | Total | 2 | 1 | 0 | 54 |
| *2 events in 1 patient | | | | | |
| †Randomized Cohort: Back pain, non-critical coronary artery disease. Open-label cohort: Disc disease (2), anemia (2), biliary colic, fatigue, vomiting, diaphoresis, dizziness, ankle fracture, spinal fracture, renal calculus, headache NOS, cholecystitis, gastric ulcer, pyloric stenosis, pain NOS, dehydration, esophageal ulcer, diverticulitis, Sphincter of Oddi dysfunction, acute renal failure, cerebrovascular accident. | | | | | |

With respect to immunogenicity, all patients were exposed to adalimumab in the first 4 weeks of Study F. 2 patients were positive for AAA in the randomized cohort: placebo group (1) and 40 mg eow (1). 6 patients were positive for AAA in the open-label cohort: 3 patients terminated early from the study. None of the 6 achieved clinical remission by Week 56

In conclusion, adalimumab consistently improved CDAI outcomes with or without concomitant IMM use, including achieving a clinical response and maintaining clinical remission in subjects with Crohn's disease. Furthermore, long-term administration of adalimumab was well-tolerated in patients with Crohn's disease.

### Examples 2: Adalimumab Maintains Improvement in Inflammatory Bowel Disease Questionnaire (IBDQ) Scores Over 1 Year Following the Initial Attainment of Remission in Patients with Moderately to Severely Active Crohn's Disease

Adalimumab, a fully human anti-TNF monoclonal antibody, is approved for the treatment of rheumatoid arthritis and psoriatic arthritis. IBDQ measures disease-related functional changes in patients with IBD. A Total IBDQ>170 score has been correlated to clinical remission (CDAI<150) (Irvine et al. Gastroenterology 1994; 1 06:287.96).

In Study E, a 4-week randomized trial, the efficacy of adalimumab in the induction of remission in patients with Crohn's disease (CD) was demonstrated, where a mean improvement in patient function and in disease activity were highly correlated (p<0.0001). Patients treated with 160/80 mg or 80/40 mg adalimumab demonstrated statistically significant improvements in mean CDAI and total IBDQ vs. placebo at Week 4. Emotional function, bowel system, and systemic dimensions of IBDQ improved significantly with adalimumab at Week 4 with 160/80 mg or 80/40 mg vs. placebo,

The purpose of the following study (Study F) was to assess the maintenance of improvement in physical function in subjects with active CD who achieved remission (CDAI<150) (measured by the Inflammatory Bowel Disease Questionnaire (IBDQ)) when treated with adalimumab in Study E and maintained it at Week 4 of Study F, an extension trial. Patients were studied over 1 year in the randomized cohort of patients in the Study F clinical trial.

All subjects in Study F completed Study E and received adalimumab 40 mg sc at Weeks 0 (Week 4 of Study E) and 2. Inclusion criteria for Study F is described above in Example 1. Study design of Study F is described above in Example 1, as well as Figure 1. Eligibility for the randomized cohort in Study F included patients in remission (CDAI<150) at both Week 0 and Week 4 of Study F.

Endpoints for this study included maintenance of remission, defined as CDAI<150, and clinical response, defined as a decrease in CDAI scores of at least 100 (Δ100) points. An additional endpoint was maintenance of improved physical function (Total IBDQ>170)

Baseline demographics for the randomized cohort of the patient population are described above in Table 1. In addition, 56%, 53%, and 50% of the patients in the placebo, 40 mg EOW, and 40 mg weekly groups, respectively, used concomitant steroids. 44%, 68%, and 67% of the patients in the placebo, 40 mg EOW, and 40 mg weekly groups, respectively, used concomitant 5-ASAs. Patient disposition is also described above in Example 1.

Patients in remission at both Weeks 0 and 4 were randomized to receive adalimumab, 40 mg sc every other week (eow) or weekly, or placebo for up to 1 year. CDAI and Inflammatory Bowel Disease Questionnaire (IBDQ) scores were assessed at each study visit. IBDQ measures disease-related functional changes in patients with IBD. A Total IBDQ > 170 score has been correlated to clinical remission (CDAI<150) (Irvine et al. (1994) Gastroenterology 106:287).

The mean baseline Study E IBDQ score of 55 patients randomized was 137, consistent with active CD. The mean IBDQ score (186.4) at the start of Study F was consistent with remission. Remission was maintained with adalimumab treatment through Week 56 in 74% and 83% of patients treated with adalimumab 40 mg eow and weekly, respectively, compared to 44% of placebo-treated patients (LOCF). IBDQ scores consistent with clinical remission were maintained through Week 56 in patients treated with adalimumab 40 mg eow or weekly, while IBDQ scores declined rapidly in patients receiving placebo. As shown below, maintenance of remission was significant in patients receiving adalimumab compared with patients receiving placebo. The maintenance of clinical remission (CDAI < 150) in the Study F randomized cohort is described below in Table 6:

**Table 6: Maintenance of clinical remission (CDAI < 150) in the Study F randomized cohort**

| | | 24 Weeks | 56 Weeks |
|---|---|---|---|
| | Placebo (n=18) | 50 | 44 |
| % of patients | 40 mg eow (n=19) | 84* | 79* |
| | 40 mg weekly (n=18) | 94* | 83* |

| | | | |
|---|---|---|---|
| LOCF:ITT population (n-55) *P=0.05 vs. placebo | | | |

Baseline IBDQ≥170 scores were maintained in patients randomized to receive adalimumab through Week 56 compared to patients who received placebo. The change in mean IBDQ score from baseline at week 56 was as follows: -24.8 for placebo; -1.0* for 40 mg EOW (**P*=0.006 versus placebo); and -5.9† (†*P*=0.015 versus placebo) for 40 mg weekly. In addition, patients treated with adalimumab in the randomized cohort of Study F achieved and maintained a ≥100-point decrease in CDAI from baseline Study E scores over the 52-week randomized period (see Table 7).

**Table 7: Δ100 CDAI clinical response in the Study F randomized cohort**

| | | 24 Weeks | 56 Weeks |
|---|---|---|---|
| | Placebo (n=18) | 61 | 56 |
| % of patients | 40 mg eow (n=19) | 84 | 79 |
| | 40 mg weekly (n=18) | 94* | 89 |

| | | | |
|---|---|---|---|
| LOCF:ITT population (n=55) *P=0.05 vs. placebo | | | |

Safety was well-tolerated in the Study F randomized cohort to week 56, as shown above in Tables 4 and 5.

In conclusion, in this randomized cohort, clinical remission and physical function were maintained with adalimumab therapy.

### Example 3: TNFα Antibody Induces and Maintains Clinical Response and Remission in Patients with Active Crohn's Disease

This study examined the efficacy of the TNFα antibody adalimumab to induce and maintain a clinical response and remission of the intestinal disorder Crohn's disease. The objective of this study was to determine the efficacy and safety of adalimumab 40 mg eow vs weekly doses for maintenance of clinical remission in moderate/severe Crohn's disease.

The overall study design was a double-blind, placebo-controlled trial, which included an open label (OL) 4-week induction period. In the OL induction period, patients were administered 80 mg at week 0 (baseline), 40 mg at week 2. All patients (responders and nonresponders) were randomized at week 4, and patients were stratified at Week 4 according to clinical response (CDAI decrease ≥70 points (CR70)). A 52 week blinded phase followed, where all patients (responders and nonresponders) were randomized to 1 of 3 maintenance treatment groups, i.e., 40 mg EOW, 40 mg weekly, PBO. In addition, open label (OL) maintenance included patients who flared/ failed to respond at/after week 12 at 40 mg OL adalimumab EOW or weekly.

Patient inclusion and exclusion criteria included the following parameters. Patients were examined endoscopically or radiographically to confirm diagnosis of Crohn's disease. Moderate to severely active Crohn's disease was defined as 220 ≤ CDAI ≤ 450. Subjects previously exposed to anti-TNF agents allowed in study if anti-TNF had been discontinued at least 12 weeks prior to screening and patient met any of the following criteria:
- a) responded and then stopped the agent
- b) responded and lost their response
- c) responded and became intolerant
- d) did not tolerate the anti-TNF agent.
In addition, concomitant treatment with 5-ASAs, corticosteroids and immunosuppressants (azathioprine, 6-MP, methotrexate) were permitted provided subject was on stable doses.

The study design of Study R is described in Figure 2. The open label segment of the study included an 80 mg induction dose of adalimumab at week 0, followed by a treatment dose of 40 mg at week 2. At week 4, the patients were stratified according to response status and entered the randomized segment of the study. At week 4, patients were randomized to select either 40 mg of ada eow, 40 mg of ada weekly, or a placebo. The endpoints in the study included the following two co-primary endpoints:

In CR70 responders at week 4
■ Remission (CDAI <150) at week 26
■ Remission (CDAI <150) at week 56

The major secondary endpoints included:
- Clinical response (CDAI decrease by 70 and 100 points)
- Discontinuation of steroid use
   ■ Steroid taper permitted at/after week 8 for patients with CDAI decrease of at least 70 points
- Fistula healing
- Remission in TNF experienced patients
Table 8 shows the baseline demographics of the study.

**Table 8: Baseline demographics**

| | All Treated (n=854) |
|---|---|
| Mean Age, years | 37 |
| Males, % | 38 |
| Caucasians, % | 93 |
| Mean Weight, kg | 71 |
| Mean CRP, mg/dl | 2.27 |

| Previous/Concomitant Medications | |
|---|---|
| Previous Anti-TNF | 50% |
| Steroids | 44% |
| Immunosuppressants | 47% |
| 5-ASAs | 39% |

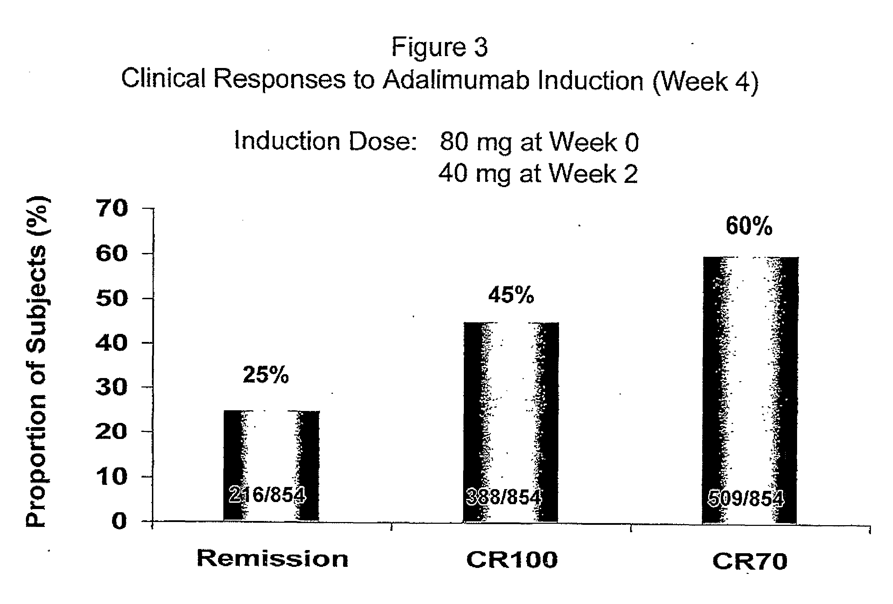

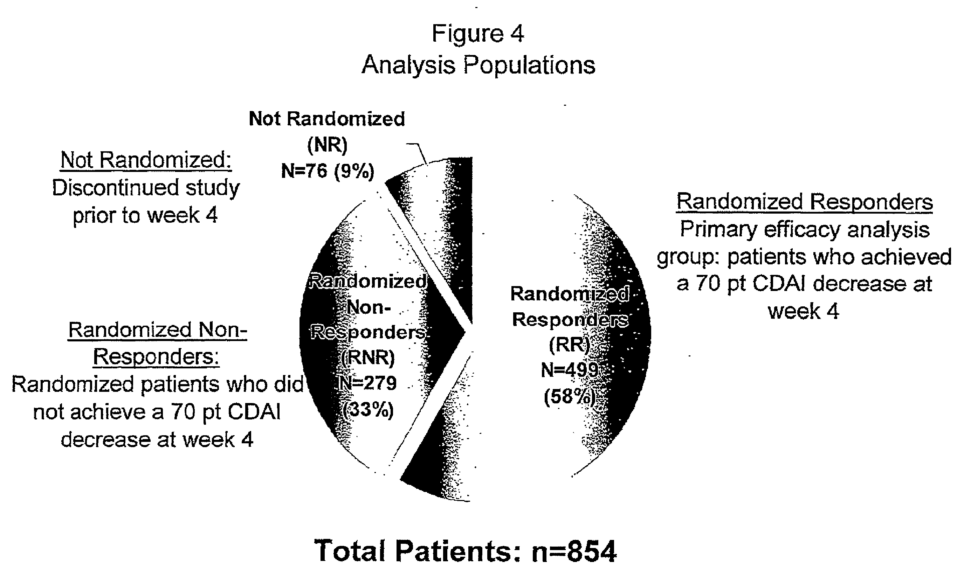

Clinical responses to adalimumab induction at week 4 are shown in Figure 3. Analysis of patient populations is described in Figure 4. Table 9 shows the baseline demographics for the randomized responders (see pie graph in Figure 4 for population).

**Table 9: Baseline Demographics for Randomized Responder Population**

| | Placebo n=170 | 40 mg EOW n=172 | 40 mg weekly n=157 | p-value |
|---|---|---|---|---|
| Mean Age, years | 37 | 36 | 37 | ns |
| Mean Weight, kg | 70 | 70 | 70 | ns |
| Mean CRP, mg/dl | 2.46 | 2.24 | 2.38 | ns |
| Mean CDAI score | 321 | 316 | 313 | |

| Previous/Concomitant Medications | | | | |
|---|---|---|---|---|
| Previous Anti-TNF | 81 (48%) | 86 (50%) | 71 (45%) | ns |
| Steroids | 69 (41%) | 65 (38%) | 76 (48%) | ns |
| Immunosuppressants | 83 (49%) | 78 (45%) | 79 (50%) | ns |

In addition, Table 10 shows the patient disposition for the randomized responder population.

**Table 10: Patient Disposition of Randomized Responders**

| | Placebo n=170 | 40 mg EOW n=172 | 40 mg weekly n=157 |
|---|---|---|---|
| Completed, n (%) | 110 (65) | 115 (67) | 131 (83) |
| Withdrawn, n (%) | 60 (35) | 57 (33) | 26 (17) |
| Adverse Event | 28 | 23 | 14 |
| Protocol Violation | 4 | 0 | 0 |
| Lack of Efficacy | 17 | 19 | 3 |
| Withdrew Consent | 8 | 11 | 7 |
| Other | 0 | 2 | 1 |

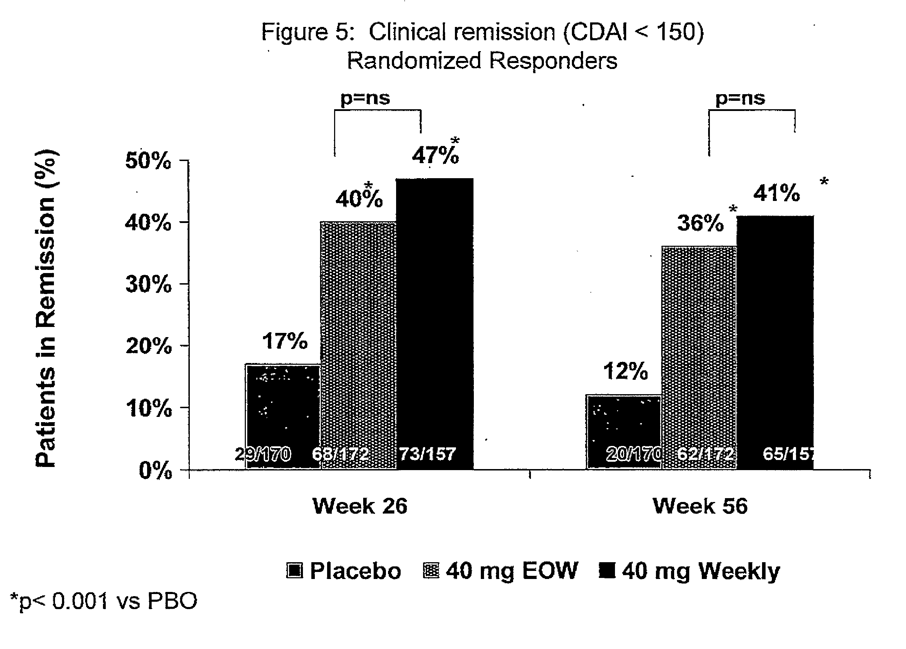

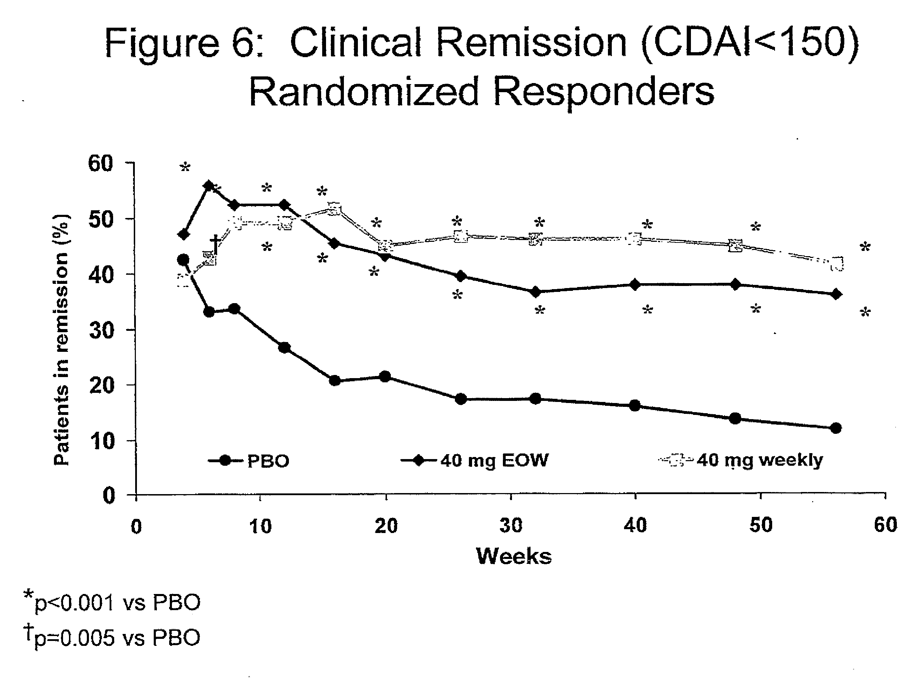

The percentage of patients in clinical remission (CDAI < 150) for the randomized responders at weeks 26 and 56 is shown in Figure 5. At week 56, 36% of the randomized responders who received adalimumab 40 mg eow and 41% of the randomized responders who received adalimumab 40 mg weekly were in clinical remission (CDAI < 150), compared to only 12% of placebo patients. The percentage of patients over the 56 week study who achieved clinical remission (CDAI < 150) in the randomized responder population is also shown in Figure 6.

Patients receiving ada either at 40 eow or weekly showed a clinical response (CDAI Δ100 and Δ70) in the randomized responder population (as shown in Table 11).

**Table 11: Clinical Response (CDAI Δ 100 and Δ70) for Randomized Responder Population**

| | | Δ100 | | Δ70 | |
|---|---|---|---|---|---|
| | | Week 26 | Week 56 | Week 26 | Week 56 |
| Patients in Response (%) | Placebo | 27 | 17 | 28 | 18 |
| | 40 mg eow | 52* | 41* | 54* | 43* |
| | 40 mg weekly | 52* | 48* | 56* | 49* |

| | | | | | |
|---|---|---|---|---|---|
| *p<0.001 ws. placebo | | | | | |

As shown in Table 12, clinical remission in the randomized responder population at weeks 26 and 56 was 32% and 38% for the patients receiving 40 mg eow and 40 mg weekly of ada, respectively, in comparison to 8% for placebo.

**Table 12: Clinical Remission at Weeks 26 and 56 Randomized Responders**

| | | Week 26 | Week 26 *and* 56 |
|---|---|---|---|
| % Patients in Remission | Placebo | 17 (29/170) | 8 (14/170) |
| | 40 mg eow | 40 (68/172) | 32 (55/172) |
| | 40 mg weekly | 47 (73/157) | 38 (59/157) |

A significant percentage of remitters at week 26 receiving either 40 mg eow (81%) (55/68) or 40 mg weekly (81%) (59/73) of ada were also in remission at week 56 in comparison to the placebo treated group (48%) (14/29). Treatment with either 40 mg eow or 40 mg weekly of ada was also able to maintain steroid-free remission for the randomized responders, as shown in Table 13.

**Table 13: Steroid-Free Remission, Randomized Responders**

| | | Off steroids, week 26 | Off steroids, week 56 |
|---|---|---|---|
| % Patients in Remission and Off Steroids | Placebo | 3 (2/66) | 6 (4/66) |
| | 40 mg eow | 35* (20/58) | 29* (17/58) |
| | 40 mg weekly | 30* (22/74) | 23** (17/74) |

| | | | |
|---|---|---|---|
| *p<0.001; **p=0.008 | | | |

Table 14 shows clinical remission by previous anti-TNF use in the randomized responder population.

**Table 14: Clinical Remission on Previous Anti-TNF Use, Randomized Responders**

| | | 26 weeks | | 56 weeks | |
|---|---|---|---|---|---|
| Patients in Remission (%) | | (+) previous TNF | (-) Previous TNF | (+) previous TNF | (-) previous TNF |
| | Placebo | 16 (81ppg) | 18 (89ppg) | 10 (81ppg) | 14 (89ppg) |
| | 40 mg eow | 33 (86 ppg) | 47 (86 ppg) | 31 (86 ppg) | 42 (86 ppg) |
| | 40 mg weekly | 42 (71 ppg) | 50 (86 ppg) | 34 (71 ppg) | 48 (86 ppg) |

| | | | | | |
|---|---|---|---|---|---|
| Ppg = patients per group | | | | | |

In sum, the above study, including Figures 5, 6 and Tables 11-14, show that adalimumab maintained remission in responder patients with active Crohn's disease, and that there was no significant difference between the 40mg eow and 40mg weekly maintenance doses.

A portion of patients receiving ada either at 40 eow or weekly who also had draining fistulas were able to maintain healing of the fistula in contrast to placebo treated patients. With respect to the total randomized patient population, 33% of patients in both ada treatment groups (37% who received 40 mg ada eow (11/30) and 30% who received 40 mg weekly (12/40)) had complete healing of draining fistulas at the last two visits versus only 13% in the placebo treatment group (6/47). Healing for this part of the study was defined as no draining fistulas for more than or equal to the last 2 post-baseline evaluations. Patients with fistulas included draining fistulas at both screening and baseline.

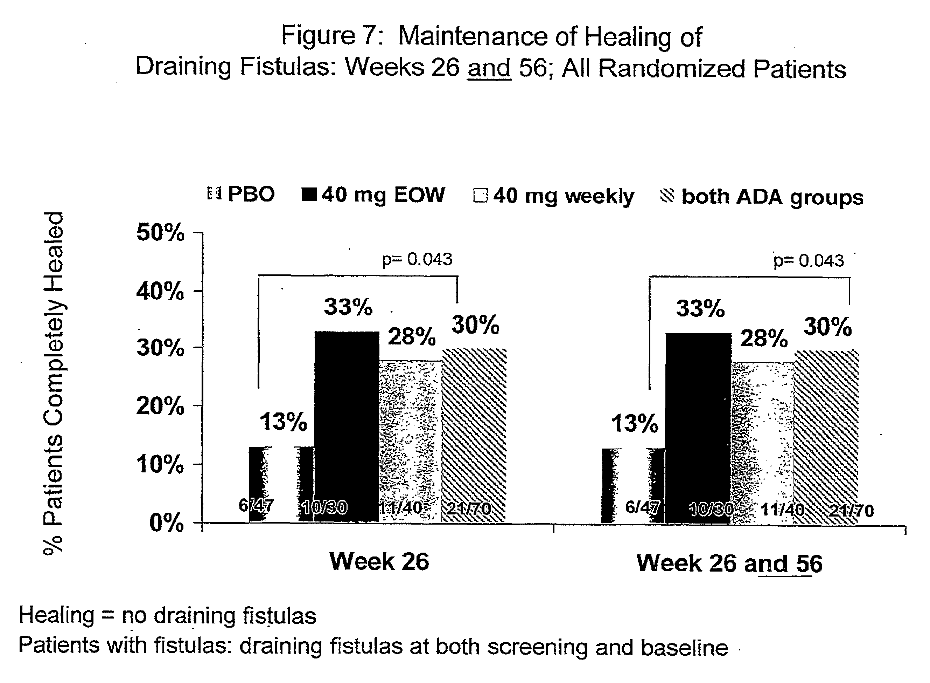

Healed draining fistulas were maintained at weeks 26 and 56 in the total randomized patient population who received ada (30%) versus placebo (13%), as shown in Figure 7.

Table 15 shows the effect of baseline CRP on remission in responder patients at week 56.

**Table 15: Effect of baseline CRP on remission: week 56**

| | | CRP < 1 mg/dl | CRP ≥ 1 mg/dl |
|---|---|---|---|
| % Patients in Rem ission at week 56 | Placebo | 13 (11/85) | 11 (9/85) |
| | 40 mg eow | 36 (34/95) | 37 (28/76) |
| | 40 mg weekly | 33 (27/82) | 51 (38/75) |
| | Both ada | 35 (61/177) | 44 (66/151) |

Table 16 shows the overall adverse events during the 4 week open label (OL) induction portion of the study for the entire patient population.

**Table 16: Patients With Adverse Events During Open Label Induction (4 wks)**

| | |
|---|---|
| All patients (n=854) | N (%) |
| Any AE | 508 (59.5%) |
| AEs leading to drug withdrawal | 54 (6%) |
| Infectious AE | 130 (15%) |
| Any SAE | 45 (5%) |
| Infectious SAE | 10 (1%) |
| Deaths (death was after 2^{nd} dose of ada due to Pulmonary embolus) | 1 (0.1%) |

Table 17 shows the adverse events according to the therapy received during the double-blind period only.

**Table 17: Adverse Events By Therapy Received, Double-Blind Period Only**

| | Placebo n=261 | 40 mg EOW n=260 | 40 mg weekly n=257 |
|---|---|---|---|
| Any AE, n (%) | 221 (85) | 231 (89) | 220 (86) |
| AE's leading to drug withdrawal, n (%) | 35 (13) | 18 (7)* | 12 (5)* |
| Infectious AE, n (%) | 96 (37) | 120 (46)* | 114 (44) |
| Any SAE, n (%) | 40 (15) | 24 (9)* | 21 (8)* |
| Infectious SAE. n (%) | 9 (3) | 7 (3) | 7 (3) |

| | | | |
|---|---|---|---|
| * p<0.05 vs PBO | | | |

The SAEs of interest for all ada treated patients are shown in Table 18 below.

**Table 18: SAEs Of Interest For All Ada Treated Patients**

| | 4 week OL N=854 | Post Randomization (weeks 4-56) | | |
|---|---|---|---|---|
| | | Placebo n=261 | 40 mg EOW n=535 | 40 mg weekly n=410 |
| Infections | 11 (1.3) | 9 (3.4) | 19 (3.6) | 11 (2.7) |
| Abscess, n (%) | 5 (0.6) | 3 (1.1) | 3 (0.6) | 4 (1.0) |
| TB | 0 (0.0) | 0 (0.0) | 1 (0.2) | 1 (0.2) |
| Other Opportunistic Infections | 0 (0.0) | 1 (0.4) | 1 (0.2) | 0 (0.0) |
| Wound infection/ septicemia | 3 (0.4) | 1 (0.4) | 1 (0.2) | 0 (0.0) |
| Pneumonia, chest infection | 0 (0.0) | 0 (0.0) | 1 (0.2) | 2 (0.5) |
| Cancer | 0 (0.0) | 1 (0.4) | 0 (0.0) | 0 (0.0) |
| MS | 1 (0.1) | 0 (0.0) | 0 (0.0) | 0 (0.0) |
| Serum Sickness | 1 (0.1) | 0 (0.0) | 0 (0.0) | 0 (0.0) |
| Death | 1 (0.1)a | 0 (0.0) | 0 (0.0) | 0 (0.0) |

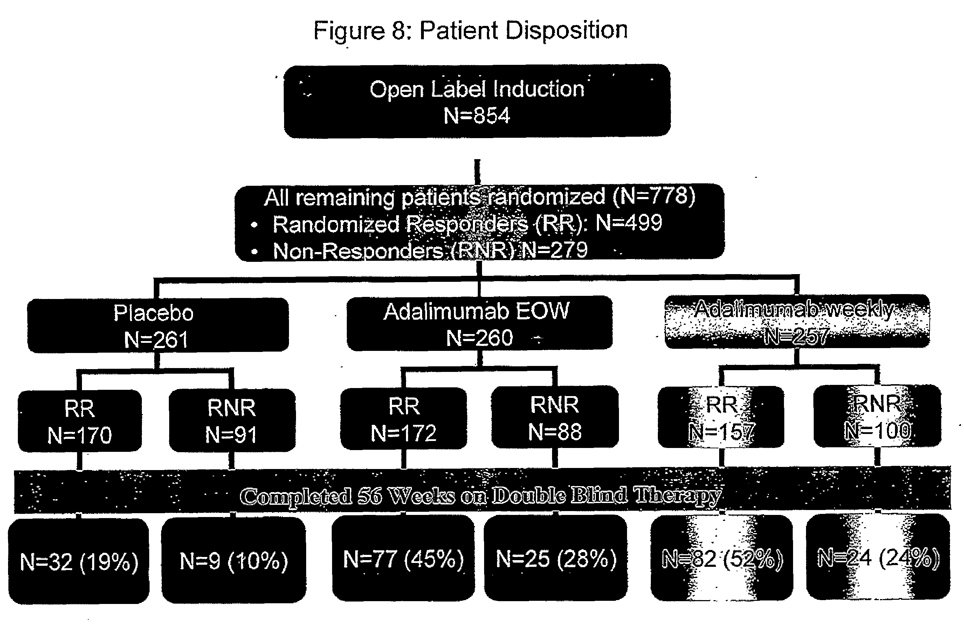

Overall patient disposition is shown in Figure 8.

The above results show that adalimumab maintained remission in patients with moderately to severely active Crohn's disease. There was no significant difference between the 40mg eow and 40mg weekly maintenance doses, and adalimumab was effective regardless of previous anti-TNF exposure. Furthermore, patients treated with adalimumab discontinued steroids and remained in remission more frequently than patients receiving placebo. Adalimumab treatment significantly increased the proportion of patients with complete healing of draining fistulas.

In addition, the results show that adalimumab was well tolerated. There was a significantly lower rate of SAEs with adalimumab maintenance compared to placebo, and no new safety concerns compared to experience in RA and previous Crohn's studies. A summary of the adverse events observed in this study is shown in Table 19.

**Table 19: Adverse Events Summary**

| | | Induction n=854 | PBO n=261 | 40 mg EOW n=260 | 40 mg W n=257 |
|---|---|---|---|---|---|
| Any AE, n (%) | | 508 (59.5) | 221 (84.7) | 231 (88.8) | 220 (85.6) |
| AEs of interest | | | | | |
| Infections Selected injection site reactions | | 130 (15.2) | 96 (36.8) | 120 (46.2)* | 114 (44.4) |
| Bruising | | 1 (0.1) | 2 (0.8) | 6 (2.3) | 2(0.8) |
| Erythema | | 7 (0.8) | 0 (0.0) | 7 (2.7)* | 3 (1.2) |
| Hemorrhage | | 4 (0.5) | 2 (0.8) | 5 (1.9) | 0 (0.0) |
| Induration | | 0 (0.0) | 0 (0.0) | 3 (1.2) | 1 (0.4) |
| Irritation | | 39 (4.6) | 2 (0.8) | 10 (3.8)* | 7 (2.7) |
| | Pain | 41 (4.8) | 2 (0.8) | 5 (1.9) | 4 (1.6) |
| | Pruritus | 2 (0.2) | 0 (0.0) | 3 (1.2) | 2 (0.8) |
| | Reaction | 17 (2.0) | 1 (0.4) | 11 (4.2)* | 15 (5,8)* |

| | | | | | |
|---|---|---|---|---|---|
| *p<0.05 vs. PBO | | | | | |

Based on the results described above for Study R, as well Examples described herein relating to Study F, an initial loading dose regimen of adalimumab 160 mg followed in 2 weeks by 80 mg for induction and a maintenance dose regimen of 40 mg every other week would be an optimal adalimumab strategy for patients with Crohn's disease, *e.g.,* moderately to severely active Crohn's disease.

### Example 4: Pharmacokinetics of Adalimumab in a Long-Term Investigation of the Induction and Maintenance of Remission in Patients with Crohn's Disease

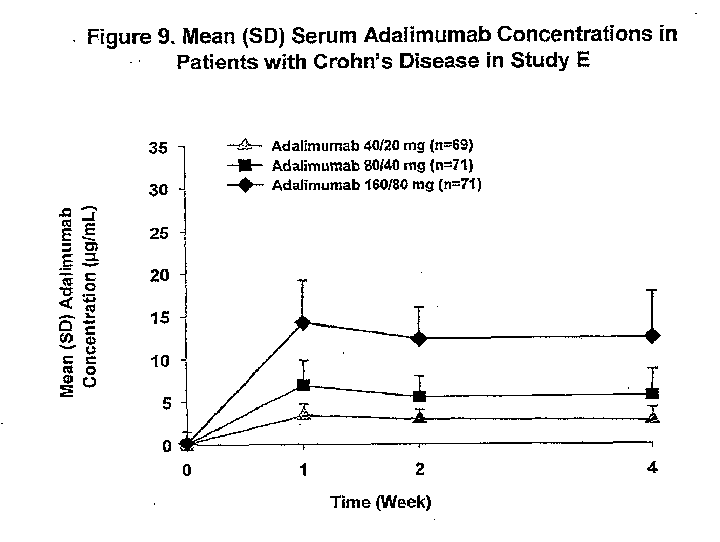

Adalimumab, a fully human anti-TNF monoclonal antibody, is approved for the treatment of rheumatoid arthritis and psoriatic arthritis. Study E was a 4-week randomized controlled study of induction of remission in patients with Crohn's disease (CD), serum adalimumab concentrations were dose-proportional. Additionally, adalimumab concentrations were sustained during the study period as a result of the initial loading dose (Paulson *et al,* DDW 2005). Figure 9 shows the mean (SD) serum adalimumab concentration in patients with Crohn's disease from Study E. In patients with Crohn's disease, the loading dose of 160 mg of adalimumab on week 0 followed by 80 mg adalimumab on week 2 achieves mean serum adalimumab trough levels of approximately 12 µg/mL at week 2 and week 4.

The purpose of the study (referred to herein as Study F) was to assess the pharmacokinetics (PK) and immunogenicity of adalimumab following long-term administration in patients with CD. Figure 1 shows an overview of the study design of Study F.

Study F, an extension of Study E, included the following patient population parameters:
■ CD for at least 4 months prior to screening
■ Moderately to severely active CD (CDAI 220-450) at the start of Study E
■ On stable doses of CD mediations (AZA, MTX, or 6-MP)
■ No previous exposure to TNF antagonists
■ Patients who completed Study E were eligible to enroll in Study F

In Study F, an extension study of Study E, all patients received adalimumab 40 mg subcutaneously (sc) at Week 0 (Week 4 of Study E) and Week 2. The study design included the following parameters:
■ In Study F, patients received adalimumab 40 mg at Week 0 (which corresponded to Week 4 of Study E) and Week 2, (Figure 2)
   ◆ Patients in remission at both Week 0 and Week 4 → 52-wk randomized cohort
   ◆ Patients not in remission at both Week 0 and Week 4 → 52-wk 40 mg eow OL cohort
■ Patients with disease flare or who persistently had no response in the randomized cohort were allowed to switch to OL 40 mg eow
   ◆ Flare was defined as recurrence of very active disease; specifically an increase in CDAI when compared to their Study F Week 4 value of 70 or more points and a CDAI>220
■ Dosage escalation to 40 mg weekly was allowed if a patient experienced a flare or persistently had no response while receiving OL 40 mg eow
■ Blood samples were collected for the evaluation of adalimumab and AAA concentrations at Weeks 4, 24, and 56

Thus, patients in remission (CDAI<150) at both Weeks 0 and 4 of Study F were randomized to receive adalimumab, 40 mg sc every other week (eow) or weekly, or placebo for up to 1 year. Patients not in remission at both Weeks 0 and 4 of Study F received open-label (OL) adalimumab, 40 mg sc eow. Dose escalation to 40 mg/week was allowed for flare or persistent non-response. Trough serum samples for adalimumab and anti-adalimumab antibody (AAA) assays were obtained at Weeks 4, 24, and 56 to determine serum concentrations using validated ELISA methods. Population PK analyses were performed using the NONMEM software to estimate adalimumab apparent clearance (CL/F) combining the data from both Studies E and F. A one-compartment model was used to describe adalimumab PK.

Outcomes were measured as follows:
■ Clinical remission: CDAI<150
■ Clinical response: CDAI decrease of at least 70 or 100 points from baseline of Study E
■ Serum adalimumab and AAA concentrations, determined using validated ELISA methods

Statistical analyses were determined as follows:
■ Descriptive statistics for serum adalimumab concentration data were calculated
■ Pharmacokinetic models were built using the NONMEM software. Combined data from Study E and Study F were analyzed

A total of 276 patients entered into Study F and 176 (64%) completed the 56-week period (Table 20)

**Table 20: Disposition of Patients in Study F**

| All Patients | | | Randomized N=55 | Open-Label N=221 |
|---|---|---|---|---|
| Completed 56 weeks | | | 45 (82%) | 131 (59%) |
| Withdrawn | | | 10 (18%) | 90 (41%) |
| Reasons for withdrawal (n) | | | | |
| | Adverse events (AE) | | 3 | 28 |
| | Lack of efficacy | | 1 | 27 |
| | Withdrew consent | | 5 | 15 |
| | Lost to follow-up | | 1 | 5 |
| | Other | | - | 15 |

Demographic characteristics were similar across randomized double-blind (DB) and open-label (OL) treatment groups (Table 21)

**Table 21: Summary Demographics of Patients by Treatment Group**

| | All Patients (N=276) | Treatment Groups in Study F* | | | |
|---|---|---|---|---|---|
| | | DB Placebo (N=18) | DB 40 mg eow (N=19) | DB 40 mg weekly (N=18) | OL 40 mg eow (N=221) |
| Age (yrs)# | 39 | 36 | 34 | 38 | 39 |
| Range | 18-74 | 20-68 | 20-58 | 23-60 | 18-74 |
| Weight(kg)# | 75 | 70 | 69 | 72 | 76 |
| Range | 41-134 | 50-95 | 45-109 | 52-134 | 41-128 |
| % Male | 45 | 33 | 37 | 50 | 47 |
| % Caucasian | 90 | 94 | 90 | 83 | 90 |

| | | | | | |
|---|---|---|---|---|---|
| *The treatment assigned to a patient at Week 4. All patients received 40 mg adalimumab at Baseline and Week 2. #Mean values. Mean (range) of CDAI at Week 0 of Study E (n=299): 298 (191-450). | | | | | |

Of 276 patients entering Study F, 55 were randomized to receive adalimumab, 40 mg sc eow (n=19) or weekly (n=18), or placebo (n=18). The remaining 221 patients received OL adalimumab, 40 mg sc eow. The median CL/F was about 15 mL/hr (14.9 mL/hr).

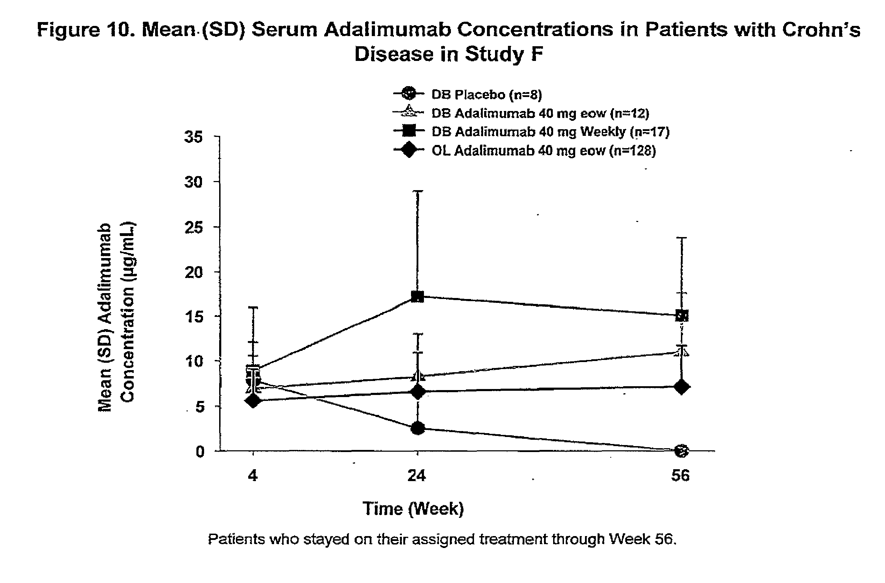

Serum adalimumab concentration results are shown in Table 22 and Figure 10. Mean steady-state trough levels of approximately 7 µg/mL were observed at week 24 and 233k 56 in Crohn's disease patients after receiving a maintenance dose of 40 mg adalimumab every other week. 128 out of 221 patients stayed on OL 40 mg eaw therapy and mean adalimumab trough concentration at Week 56 was 7.2 µg/mL. Furthermore, 17 out of 18 patients stayed on DB 40 mg weekly therapy and mean adalimumab trough concentration at Week 56 was 15.0 µg/mL

**Table 22: Mean + SD Adalimumab Through Concentrations (µg/mL)**

| Treatment* | Week 4 | Week 24 | Week 56 |
|---|---|---|---|
| DB Placebo (N=8) | 7.8 ± 4.2 (n=8) | 2.6 ± 5.8 (n=6) | 0.0 ± 0.0 (n=6) |
| DB 40 mg eow (N=12) | 6.9 ± 3.6 (n=12) | 8.2 ± 4.7 (n=10) | 10.9 ± 6.6 (n=10) |
| DB 40 mg wkly (N=17) | 8.8 ± 7.1 (n=17) | 17.0 ± 11.9 (n=16) | 15.0 ± 8.7 (n=14) |
| OL 40 mg eow (N=128) | 5.6 ± 3.4 (n=112) | 6.6 ± 4.3 (n=82) | 7.2 ± 4.6 (n=71) |

| | | | |
|---|---|---|---|
| * The treatment assigned to a subject at Week 4. All patients received 40 mg adalimumab at Baseline and Week 2. All the patients listed in this table stayed on their assigned treatment through Week 56. | | | |

A population pharmacokinetic model was used. A one-compartment model expressing V/F in terms of body weight, with inter-individual error terms on both V/F and CL/F, and with a proportional residual error term. Goodness-of-fit plots demonstrate the adequacy of the fitting of the model to the data. The median CL/F of adalimumab was 14.9 mL/hr, and was 38% lower than that in RA patients without concomitant MTX (23.9 mL/hr) The median CL/F of adalimumab was comparable to that in RA patients treated with MTX (12.0 to 14.6 mL/hr). Furthermore, the median V/F was 8.7 L, indicating that adalimumab mainly resides in the extracellular space

Concomitant therapy with either the immunosuppressant 6 mercaptopurine (n=23) or azathioprine (n=36) slightly lowered or had no impact on adalimumab CL/F. Concomitant azathioprine or 6-mercaptopurine slightly decreased adalimumab clearance (10% and 18% lower, respectively), but.the differences did not reach statistical significance (*P*>0.09). The results also show that concomitant AZA or 6-MP slightly decreased adalimumab clearance, but the decrease was not statistically significant (*P*>0.09). The number of patients with concomitant MTX was too small (n=6) for adequate assessment.

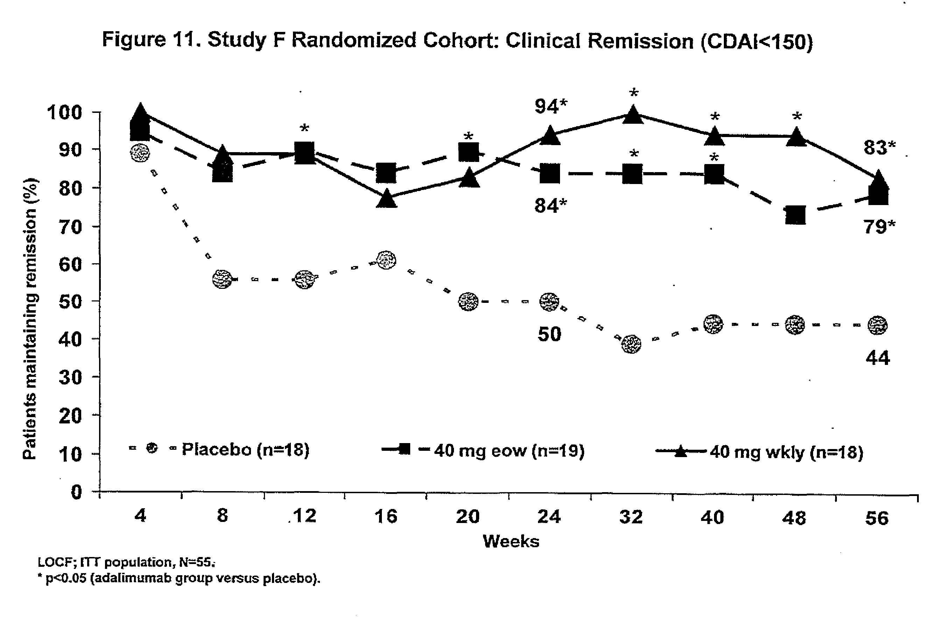

With respect to immunogenicity, the incidence of positive AAA was 2.6% (7 of 269 patients), including 1 of 18 patients in the DB placebo* group (* All patients received OL 40 mg adalimumab at Weeks 0 and 2); 1 of 18 patients in the DB adalimumab 40 mg eow group; and 5 of 215 patients in the OL adalimumab 40 mg eow group. All 7 AAA+ patients stayed on their original treatment. No patients tested AAA+ in the adalimumab 40 mg weekly group. Among the 7 AAA+ patients, 3 (43%) were in clinical remission at Week 24 and 2 (29%) sustained clinical remission at Week 56. Thus, the effect of concomitant methotrexate alone on adalimumab CL/F was inconclusive (n=6). The overall incidence of positive AAA in Study F was about 3% (2.6%; 7/269). Among the 7 AAA+ patients, 3 (43%) were in remission (CDAI<150) at Week 24 and 2 (29%) were in remission at Week 56. Adalimumab administration led to long-lasting improvements in clinical response and remission, as described above in Examples 1-3 and in Figure 11.

In conclusion, the pharmacokinetics of adalimumab in patients with CD remained constant over time. The median CL/F of adalimumab in patients with CD was 14.9 mL/hr. Concomitant immunosuppressants (AZA or 6-MP) did not have a statistically significant effect on adalimumab clearance. The effect of concomitant methotrexate alone on adalimumab CL/F was inconclusive (n=6). The overall incidence of positive AAA was low (2.6%). Adalimumab administration led to long-lasting improvements in clinical response and remission in patients with CD. Adalimumab was well-tolerated.

### Example 5: Efficacy of HUMIRA® (Adalimumab) for Treatment of Patients with Crohn's Disease

The following example provides a collective overview of the clinical studies, including Study E, Study F, Study R, and Study 2, described herein.

The safety and efficacy of multiple doses of HUMIRA^{®} were assessed in adult patients with moderately to severely active Crohn's disease (Crohn's Disease Activity Index (CDAI) ≥220 and ≤450) in randomized, double-blind, placebo-controlled studies. Concomitant stable doses of aminosalicylates, corticosteroids, and/or immunomodulatory agents were permitted, and 79% of patients continued to receive at least one of these medications.

Induction of clinical remission (defined as CDAI <150) was evaluated in two studies. In Study E, 299 TNF-blocker naïve patients were randomized to one of four treatment groups: the placebo group received placebo at weeks 0 and 2, the 160/80 group received 160 mg HUMIRA^{®} at week 0 and 80 mg at week 2, the 80/40 group received 80 mg at week 0 and 40 mg at week 2, and the 40/20 group received 40 mg at week 0 and 20 mg at week 2. Clinical results were assessed at week 4.

In the second induction study, Study 2, 325 patients who had lost response to, or were intolerant to, previous infliximab were randomized to receive either 160 mg HUMIRA® at week 0 and 80 mg at week 2, or placebo at weeks 0 and 2. Clinical results were assessed at week 4.

Maintenance of clinical remission was evaluated in Study R. In this study, 854 patients with active disease received open-label HUMIRA®, 80 mg at week 0 and 40 mg at week 2. Patients were then randomized at week 4 to 40 mg HUMIRA® every other week, 40 mg HUMIRA® every week, or placebo. The total study duration was 56 weeks. Patients in clinical response (decrease in CDAI ≥70) at week 4 were stratified and analyzed separately from those not in clinical response at week 4.

### Induction of Clinical Remission

A greater percentage of the patients treated with 160/80 mg HUMIRA® achieved induction of clinical remission versus placebo at week 4 regardless of whether the patients were TNF blocker naïve (Study E) or had lost response to or were intolerant to infliximab (Study 2) (see Table 23).

**Table 23: Induction of Clinical Remission in Studies E and 2 (Percent of Patients)**

| | Study E | | Study 2 | |
|---|---|---|---|---|
| | Placebo N=74 | HUMIRA® 160/80 mg N=76 | Placebo N=166 | HUMIRA® 160/80 mg N=159 |
| Week 4 | | | | |
| Clinical remission | 12% | 36%** | 7% | 21%** |
| Clinical response | 34% | 58%* | 34% | 52%* |

| | | | | |
|---|---|---|---|---|
| Clinical remission is CDAI score <150; clinical response is decrease in CDAI of at least 70 points. * p<0.001 for HUMIRA® *vs*. placebo pairwise comparison of proportions ** p<0.01 for HUMIRA® *vs.* placebo pairwise comparison of proportions | | | | |

Additional details regarding Study 2 are provided in Example 6 below.

### Maintenance of clinical Remission

In Study R at week 4, 58% (499/854) of patients were in clinical response and were assessed in the primary analysis. At weeks 26 and 56, greater proportions of patients who were in clinical response at week 4 achieved clinical remission in the HUMIRA® 40 mg every other week maintenance group compared to patients in the placebo maintenance group (see Table 24). The group that received HUMIRA® therapy every week did not demonstrate significantly higher remission rates compared to the group that received HUMIRA® every other week.

**Table 24: Maintenance of Clinical Remission in Study R (Percent of Patients)**

| | Placebo N=170 | 40 mg HUMIRA® every other week N=172 |
|---|---|---|
| *Week 26* | | |
| Clinical remission | 17% | 40%^{*} |
| Clinical remission (CR-70) | 28% | 54%^{*} |

| *Week 56* | | |
|---|---|---|
| Clinical remission | 12% | 36%^{*} |
| Clinical remission (CR-70) | 18% | 43%^{*} |

| | | |
|---|---|---|
| Clinical remission is CDAI score <150; clinical response is decrease in CDAI of at least 70 points. * p<0.001 for HUMIRA® *vs.* placebo pairwise comparisons of proportions | | |

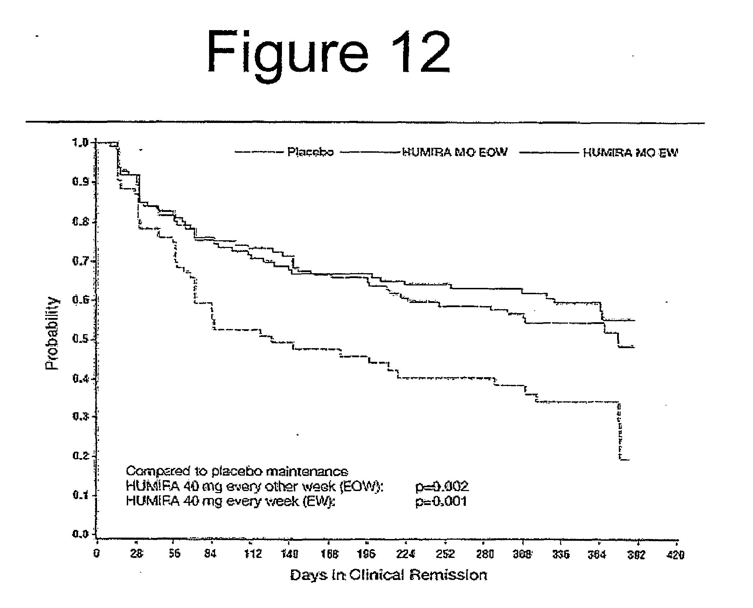

Of those in response at week 4 who attained remission during the study, patients in the HUMIRA® every other week group maintained remission for a longer time than patients in the placebo maintenance group (see Figure 12). Among patients who were not in response by week 12, therapy continued beyond 12 weeks did not result in significantly more responses.

### Patient Reported Outcomes

In Study E and Study 2, statistically significant improvement in the disease-specific inflammatory bowel disease questionnaire (IBDQ) total score was achieved at week 4 in patients randomized to HUMIRA® 160/80 mg compared to placebo. Statistically significant improvement from baseline in IBDQ total scores was seen at weeks 26 and 56 in Study R among the adalimumab treatment groups compared to the placebo group.

### Example 6: Adalimumab Induction Therapy for Crohn's Disease with Lost Response to or Intolerance of Infliximab

The following study describes a double-blind, randomized, placebo-controlled 4-week trial conducted in which patients with moderate to severe Crohn's disease who had previously responded to infliximab and then lost response or who became intolerant received induction therapy with adalimumab 160 mg followed by 80 mg or placebo at weeks 0 and 2, and were followed through week 4.

### Methods

### Patients

This study, Study 2 was a randomized, double-blind, placebo-controlled trial conducted at 52 centers. Eligible patients included men and women 18 to 75 years of age with Crohn's disease for at least 4 months that was moderately to severely active at baseline as defined by a Crohn's Disease Activity Index (CDAI) score of 220 to 450 points (scores range from 0 to 600, with higher scores indicating more severe disease activity). Radiologic or endoscopic evidence was required to confirm the presence of Crohn's disease. Patients must have previously responded to infliximab and then lost response or become intolerant (see detailed definition, which follows). Those who had a primary nonresponse to infliximab as defined by the investigator, those who had received infliximab or another TNF antagonist within 8 weeks, and those who had previously received adalimumab (HUMIRA®®, Abbott Laboratories, Abbott Park, Illinois) or participated in an adalimumab clinical trial were excluded. Concurrent therapies, including stable dosages of 5-aminosalicylates, prednisone (40 mg per day or less), budesonide (9 mg per day or less), azathioprine, 6-mercaptopurine, methotrexate, and antibiotics, were permitted. Patients with short bowel syndrome, a stricture with obstructive symptoms, or bowel resection within 6 months; patients who had undergone ostomy or ileoanal pouch; patients receiving total parenteral nutrition; patients who had received antibiotic treatment within 3 weeks of the study for non-Crohn's disease-related infections; patients with untreated tuberculosis; and patients with demyelinating disorders were excluded.

### Infliximab Loss of Response or Intolerance

Patients were classified as having loss of response if they had a history of an initial response to infliximab, as defined by the investigator; had received at least 2 doses of infliximab of at least 5 mg per kilogram body weight; and had lack of improvement or worsening in at least 1 of the following Crohn's-disease-related signs or symptoms at least 2 weeks after the last dose of infliximab: stool frequency, daily abdominal pain, fever, recurring drainage from a previously nondraining fistula or development of a new draining fistula, rectal bleeding, or change in usage of antidiarrheal medication.

Patients were classified as having intolerance to infliximab if they had a history of infliximab discontinuation as a result of a significant acute or delayed infusion reaction. A significant acute infusion reaction was defined as an adverse reaction that 1) occurred during or within 24 hours of an infliximab infusion, 2) was considered related to the infusion by the physician, and 3) was manifested through 1 or more of the following signs or symptoms: fever greater than 100 degrees Fahrenheit; chills or rigors; itching; rash; flushing; urticaria or angioedema; breathing difficulties (dyspnea, chest pain or tightness, shortness of breath, wheezing, stridor); and clinical hypotension (pallor, diaphoresis, faintness, syncope), or blood pressure less than 90 over 60 millimeters of mercury, or orthostatic drop in systolic blood pressure of greater than 20 millimeters of mercury. A significant delayed infusion reaction was defined as an adverse reaction that occurred more than 24 hours and fewer than 15 days after infusion of infliximab; was considered related to the infusion by the physician; and was manifested through 1 or more of the following signs or symptoms: myalgias, arthralgias, fever greater than 100 degrees Fahrenheit, malaise, and rash.

### study Design

Eligible patients were randomly assigned 1:1 to receive subcutaneous injections of adalimumab 160 mg at week 0 and 80 mg at week 2 or placebo at weeks 0 and 2, and were followed through week 4.

The primary efficacy endpoint of the trial was the proportion of patients with remission at week 4. Remission was defined as a CDAI score of less than 150 points. 12 Response was defined as a decrease in CDAI score of at least 70 points (70-point response) or of at least 100 points (100-point response) at week 0.

The trial was centrally randomized. Patients and investigators were unaware of treatment assignments. The dosages of all concomitant medications remained constant.

### Efficacy and Safety Evaluations

Patients were assessed 2 weeks before randomized treatment began, on day 0, and at 1, 2, and 4 weeks. At each visit, the CDAI score was determined, adverse events and concomitant medications were recorded, and samples were collected for laboratory evaluations. The Inflammatory Bowel Disease Questionnaire (IBDQ) was administered to assess patient-reported outcomes at weeks 0 and 4 (total scores range from 32 to 224, with higher scores indicating better patient function and quality of life). 13 Safety evaluations included vital signs and physical examinations. Laboratory evaluations included hematologic analysis; serum biochemical analysis; urinalysis; and determination of concentrations of C-reactive protein, adalimumab and antibodies to adalimumab, and infliximab and antibodies to infliximab.

### Statistical Analysis

It is estimated that a minimum of 300 patients would be needed to have 80 percent power to detect an absolute difference in remission rates of 15 percent between the adalimumab and placebo groups, assuming a 35 percent rate of remission with adalimumab, a 20 percent rate of remission with placebo, and a 5 percent rate of patients who could not be evaluated, and a two-sided alpha-level of 5 percent.

The primary efficacy endpoint was remission at week 4. Prespecified secondary analyses included the proportions of patients attaining clinical response (70-point response and 100-point response) at week 4 in the adalimumab and placebo groups; changes from baseline in CDAI total score, IBDQ total score, and C-reactive protein concentration; improvement in the number of draining fistulas at week 4 (defined as a ' decrease of at least 50 percent in the number of draining fistulas at weeks 2 and 4 as compared with baseline); and fistula remission at week 4 (defined as closure of all fistulas at weeks 2 and 4 that were draining at screening and baseline).

To compare the proportions of patients achieving remission, 70-point response, 100-point response, fistula improvement, and fistula remission between treatment groups, a 2-sided Pearson's chi-square test was performed. Continuous response parameters including the CDAI total score, the IBDQ total score, and the C-reactive protein concentration were compared using ANCOVA. Fisher's exact test was used for analysis of adverse events. Patients for whom data were missing were counted as treatment failures. In addition, analyses were performed to explore the effect of adalimumab in subpopulations with elevated C-reactive protein concentration (greater than 1.0 mg per deciliter at baseline), those intolerant of infliximab; those who lost response to infliximab, those positive for antibodies to infliximab, and those using immunosuppressive agents.

### Results

### Characteristics of the Patients

The following is a summary of the disposition of patients. 325 patients underwent randomization at week 0 of the trial. 166 patients received placebo at weeks 0 and 2, of which 10 withdrew prematurely (4 due to adverse events; 1 withdrawal of consent; and 5 for protocol violation). 159 patients received adalimumab at 160 mg at week 0 and 80 mg at week 2, of which 4 withdrew (2 adverse events, 1 withdrawal of consent, and 1 protocol violation). Thus, 156 patients who received placebo completed the trial, and 155 patients who received adalimumab completed the trial.

The baseline characteristics of the patients were generally similar in the two groups (Table 25).

**Table 25, Baseline Characteristics of the Patients**

| | | Placebo | Adalimumab 160mg/80mg | P-value |
|---|---|---|---|---|
| Characteristic | | n=166 | n=159 | |
| Male sex - no. (%) | | 65 (39) | 50(31) | 0.146 |
| Age-mean years (SD) | | 37 (12) | 39 (12) | 0.149 |
| Weight-mean kg (SD) | | 72 (19) | 72 (19) | 0.934 |
| Intestinal area involved - no. (%)* | | | | |
| | Colonic | 113 (68) | 10 (66) | 0.724 |
| | Ileal | 124 (75) | 112 (70) | 0.455 |
| | Rectum | 37 (22) | 36 (23) | 1.000 |
| | Perianal/anal | 31 (19) | 27 (17) | 0.772 |
| | Gastroduodenum | 16 (10) | 5 (3) | 0.023 |
| | Jejunum | 4 (2) | 6 (4) | 0.535 |
| | Other | 6 (4) | 5 (3) | 1.000 |
| Abdominal or perianal fistula at baseline - no. (%) | | 25 (15) | 20 (13) | 0.526 |
| CDAI - mean score (SD) | | 313 (66) | 313 (58) | 0.857 |
| Baseline IBDQ - mean score (SD)^{t} | | 124 (28) | 120 (27) | 0.228 |
| CRP-mg/decililter^{‡} | | | | |
| | Mean (SD) | 2.0 (3.7) | 1.9 (2.5) | 0.819 |
| | Median (range) | 0.7 (0.0-23.5) | 0.9 (0.0-11.5) | 0.242 |
| CRP concentration ≥ 1.0^{§} - no. (%) | | 68 (41) | 77 (48) | 0.176 |
| Prior loss of response to infliximab - no. (%)* | | 87 (52)^{∥} | 77 (48) | 0.506 |
| Prior intolerance to inflixiniab -no. (%)* | | 95 (57) | 95 (60) | 0.654 |
| | Acute reaction | 63 (38) | 68 (43) | 0.429 |
| | Delayed reaction | 52 (31) | 43 (27) | 0.464 |
| | Acute and delayed reaction | 20 (12) | 15 (9) | 0.479 |
| Prior loss of response and intolerance to infliximab -no. (%)* | | 21 (13)^{∥} | 19 (12) | 0.868 |
| Antibodies to infliximab-no. (%)^{¶} | | | | |
| | Absent | 91/159 (57) | 88/150 (59) | 0.818 |
| | Indeterminant^{#} | 8/159 (6)** | 12/150 (8) | 0.357 |
| | Present | 60/159 (38)** | 50/150 (33) | 0.476 |
| | Corticosteroids^{††} | 73 (44) | 55 (35) | 0.083 |
| | Any immunosuppressive agent | 85 (51) | 73 (46) | 0.340 |
| | 5-aminosalicylates^{‡‡} | 60 (36) | 45 (28) | 0.131 |
| Current smoker-no. (%) | | 56 (34) | 55 (35) | 0.866 |

| | | | | |
|---|---|---|---|---|
| * Percentages total more than 100 percent (patient could have been counted in more than one category). ^{†}Scores for the IBDQ can range from 32 to 224; higher scores indicate a better quality of life. ^{‡}To convert to SI units, multiply mg/deciliter by 10 to result in mg/liter. ^{§}High sensitivity cardiology assay for CRP; normal range is <0.283 mg/dL (2.83 mg/L). ^{∥}Missing data for 1 patient in the placebo group. ^{¶}M¡ssing data for 7 patients in the placebo group and 6 patients in the adalimumab group. ^{#}Results for antibodies to infliximab indeterminant because of the presence of infliximab in 9 patients in the placebo group and 12 patients in the adalimumab group **One patient in the placebo group had both a measurable infliximab concentration and antibody to infliximab. ^{††}Budesonide, betamethasone, dexamethasone, deflazacort, cortisone, cloprednol, corticosteroids, fluocortolone, glucocorticoids, glucocorticosteroids, hydrocortisone, methylprednisolone, prednisolone, prednisone, paramethasone, or prednylidene. ^{‡‡}Mesalamine, sulfasalazine, olsalazine, and balsalazide. CDA1, Crohn's Disease Activity Index; CRP, C-reactive protein; IBDQ, Inflammatory Bowel Disease Questionnaire; kg, kilogram; mg, milligram; SD, standard deviation | | | | |

### Efficacy

At week 1, 6% of patients in the adalimumab group were in remission compared with 4% of placebo (P=0.004). At week 2, 21 % of patients in the adalimumab group were in remission compared with 6% of placebo (P<0.001). At week 4, 21 percent of patients in the adalimumab group were in remission (34 of 159), as compared with 7 percent of placebo (12 of 166, P=0.001). The difference between the adalimumab and placebo groups was already significant at week 1 for the more sensitive measure of response, defined as a decrease of at least 70 points in the CDAI score. The percentages of 70 Point Response were: week 1: 21% placebo, 35% adalimumab (P=0.004); week 2= 33% placebo, 52% adalimumab (P<0.001); week 4 = 34% placebo, 52% adalimumab (P=0.001). The percentages of 100 Point Response were: week 1: 12% placebo, 20% adalimumab (P=0.065); week 2= 18% placebo, 37% adalimumab (P<0,001); week 4 = 25% placebo, 38% adalimumab (P=0.008). Thus, the rates of 70-point response, 100-point response, and remission were significantly greater for the adalimumab group at weeks 2 and 4.

The efficacy of adalimumab was demonstrated in subgroups of patients stratified for concomitant immunosuppressive therapy (azathioprine, 6-mercaptopurine, methotrexate), concomitant corticosteroid therapy, prior loss of response to infliximab, prior intolerance to infliximab, prior loss of response to and intolerance to infliximab, presence of antibodies to infliximab at screening, and week-0 C-reactive protein concentration ≥1.0 mg per deciliter (10 mg per liter) (Table 26). Statistical analysis failed to demonstrate a relationship between any of these variables and induction of remission at week 4.

**Table 26. Summary of Subgroup Efficacy Results**

| | Placebo | Adalimumab 160 mg/80 mg | P-value |
|---|---|---|---|
| Remission in patients not receiving immunosuppressive agents at baseline -no. (%) | 6/81 (7) | 18/86 (21) | 0.015 |
| Remission in patients receiving immunosuppressive agents at baseline - no. (%) | 6/85 (7) | 16/73 (22) | 0.010 |
| Remission in patients not receiving corticosteroids at baseline-no. (%) | 9/93 (10) | 16/104 (15) | 0.286 |
| Remission in patients receiving corticosteroids at baseline - no. (%) | 3/73 (4) | 18/55 (33) | <0.001 |
| Remission in patients with prior loss of response to infliximab-no. (%)* | 7/87 (8) | 15177 (20) | 0.039 |
| Remission in patients with prior intolerance to infliximab - no. (%) | 5/95 (5) | 21/95 (22) | 0.001 |
| Remission in patients with prior loss of response to and intolerance to infliximab - no. (%)* | 0/21 (0) | 3/19 (16) | 0.098 |
| Remission in patients who are negative for antibodies to infliximab - no. (%)^{†} | 7/91 (8) | 19/88 (22) | 0.010 |
| Remission in patients who are indeterminate for antibodies to infliximab-no. (%)^{†} | 2/8 (25) | 2/12 (17) | 1.000 |
| Remission in patients who are positive for antibodies to infliximab - no. (%)^{†} | 2/60 (3) | 11/50 (22) | 0.003 |
| Remission in patients with baseline CRP concentration <1.0 mg/dL (10 mg/L) - no. (%) | 7/98 (7) | 15/82 (18) | 0.038 |
| Remission in patients with baseline CRP concentration ≥1.0 mg/dL (10 mg/L) - no. (%) | 5/68 (7) | 19/77 (25) | 0.007 |
| Enterocutaneous or perianal fistula improvement^{‡} - no. (%) | 5/25 (20) | 3/20 (15) | 0.716 |
| Enterocutaneous or perianal fistula remission^{§} - no. (%) | 2/25 (8) | 1/20 (5) | 1.000 |

| | | | |
|---|---|---|---|
| *Missing data for I patient in the placebo group. ^{†}missing data for 7 patients in the placebo group and 6 patients in the adalimumab group. ^{‡}Fistula improvement defined as a decrease of at least 50 percent in the number of draining fistulas at weeks 2 and 4 as compared with baseline. ^{§}Fistula remission defined as closure of all fistulas at weeks 2 and 4 that were draining at screening and baseline. | | | |

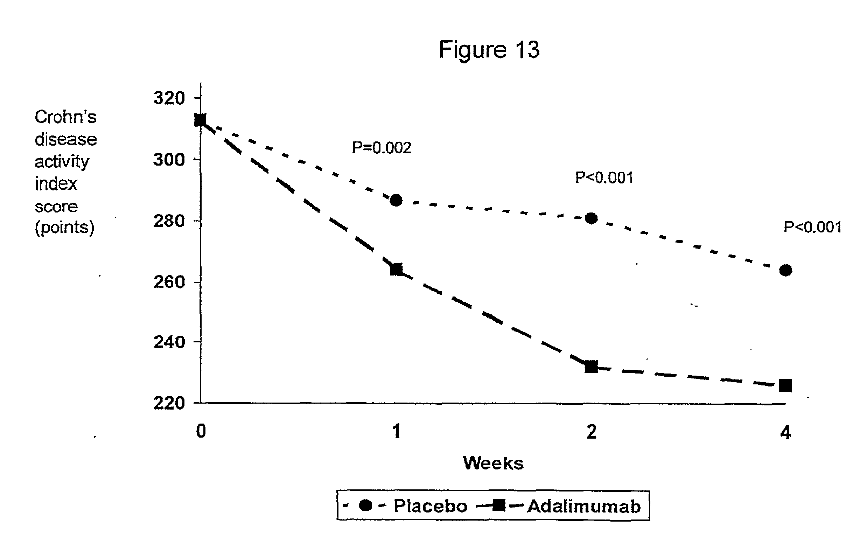

Patients in the adalimumab group had significantly lower mean CDAI total scores at weeks 1, 2, and 4 (Figure 13), as compared with patients in the placebo group.

Disease-related patient outcomes were assessed by self-administration of the Inflammatory Bowel Disease Questionnaire (IBDQ) at baseline and Week 4. The 32-item IBDQ ranges from 32-224, with higher scores indicating better patient function. Statistical analysis was conducted to compare mean change in IBDQ total scores at Week 4 for each treatment group vs. placebo, and four IBDQ dimensional scores - systemic, bowel system, emotional function, and social function. The mean IBDQ total scores at week 4 were 150 in the adalimumab group and 139 in the placebo group, P<0.001. At Week 4, patients randomized to adalimumab had a greater mean change in total IBDQ score from baseline than placebo, 30.2 adalimumab vs.15.1 placebo. In an ANCOVA model with treatment as a factor and baseline value as a covariate, the least square mean difference between adalimumab and placebo was 14.16 (p<0.001).

A significantly greater number of patients achieved clinically relevant improvements in all aspects of health-related quality of life, demonstrated in the IBDQ Total score, with adalimumab compared to placebo. The proportion of patients with clinically meaningful IBDQ total change score of ≥ 16 units was: 42% placebo patients and 62% adalimumab patients.

Scores for each IBDQ domain score (social function, systemic system, emotional function, and bowel symptoms) in the adalimumab group were all significantly greater than placebo (p<0.001). The least square mean different in IBDQ domain scores from placebo at week 4 included: 5.3 bowel symptoms, 2.0 systemic, 4.1 emotional function, and 2.7 social function. In other words, all aspects of quality of life had a better outcome with active therapy. Within 4 weeks of treatment initiation, adalimumab improved all aspects of quality of life measured by the IBDQ in patients with active CD who had failed infliximab therapy.

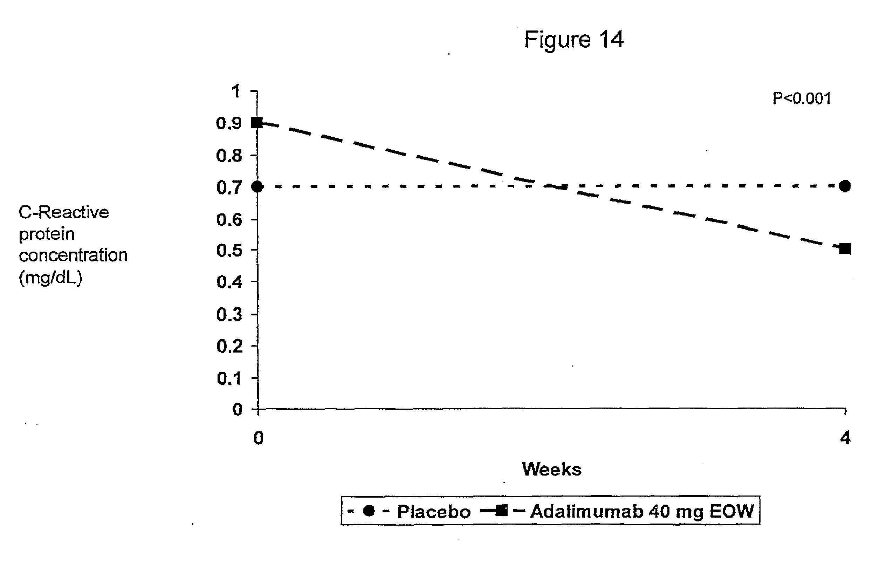

Patients in the adalimumab group also had a significantly lower median C-reactive protein concentration at week 4 as compared with patients in the placebo group (Figure 14). Fourteen percent of the randomized patients (45 of 325) had draining enterocutaneous or perianal fistulas at baseline. The rates of fistula improvement and remission at week 4 were similar for both treatment groups (Table 26).

### Safety

Adverse events were reported more frequently by patients receiving placebo than by patients receiving adalimumab (Table 27). Exacerbation of Crohn's disease was significantly greater in the placebo group. Otherwise, there were no significant differences between the two groups (Table 27). There was no significant difference in the number of patients who discontinued treatment because of an adverse event, 1 percent in the adalimumab group (2 of 159) and 2 percent in the placebo group (4 of 166) (Table 27). Serious adverse events occurred in 1 percent of patients in the adalimumab group (2 of 159), as compared with 5 percent of patients in the placebo group (8 of 166) (Table 27). No solid tumors or hematologic cancers occurred during the study, none of the patients developed clinical symptoms consistent with lupus or lupus-like disease, and there were no deaths.

**Table 27. Summary of Safety and Immunogenicity Analyses for All Patients Who Underwent Randomization**

| Variable | | | Placebo | Adalimumab 160 mg/80 mg | P-value |
|---|---|---|---|---|---|
| | | | n=166 | N=159 | |
| Adverse events - no. of patients (%) | | | 121 (73) | 91 (57) | 0.004 |
| Adverse events occurring in ≥5% of any treatment group - no. of patients (%) | | | | | |
| | Abdominal pain | | 12 (7) | 9(6) | 0.654 |
| | Arthralgia | | 3 (2) | 9 (6) | 0.081 |
| | Headache | | 12 (7) | 8 (5) | 0.492 |
| | Injection site irritation | | 7(4) | 8 (5) | 0.795 |
| | Fatigue | | 9 (5) | 7 (4) | 0.799 |
| | Crohn's disease | | 15 (9) | 2 (1) | 0.002 |
| Patients with any type of injection site reactions - no. (%) | | | 17 (10) | 17 (11) | 1^{.}000 |
| | Specific types of injection site reactions - no. (%) | | | | |
| | | Bruising | 1 (0.6) | 3 (2) | 0.362 |
| | | Erythema | 0 (0) | 1 (0.6) | 0.489 |
| | | Hemorrhage | 0 (0) | 1 (0.6) | 0.489 |
| | | Irritation | 7 (4) | 8 (5) | 0.795 |
| | | Pain | 4(2) | 1 (0.6) | 0.372 |
| | | Pruritus | 0 (0) | 1 (0.6) | 0.489 |
| | | Injection-site reaction | 6 (4) | 5 (3) | 1.000 |
| Adverse events leading to discontinuation of study drug - no. of patients (%) | | | 4(2) | 2 (1) | 0.685 |
| Serious adverse events - no. of patients (%) | | | 8 (5) | 2 (1) | 0.105 |
| Infections-no. of patients (%) | | | 39 (24) | 26 (16) | 0.127 |
| Serious infections-no. of patients (%) | | | 4 (2) | 0 (0) | 0.123 |
| | Abdominal abscess | | 1 (0.6) | 0 (0) | 1.000 |
| | Pelvic abscess | | 1 (0.6) | 0 (0) | 1.000 |
| | Perianal abscess | | 1 (0.6) | 0 (0) | 1.000 |
| | Staphylococcal sepsis | | 1 (0.6) | 0 (0) | 1.000 |
| Antibodies against adalimumab during the study - no. of patients/total no. (%) | | | 0 (0) | 0 (0) | 1.000 |

There were no clinically significant changes in laboratory values in either treatment group. The incidence of infectious adverse events in the adalimumab group (16 percent [26 of 159]) and the placebo group (24 percent [39 of 166]) was similar (Table 27). Serious infectious adverse events occurred in none of the 159 patients in the adalimumab group and 2 percent of patients in the placebo group (4 of 166). The specific types of serious infections observed in each treatment group are shown in Table 27. No patients in either group developed tuberculosis or opportunistic infections. Injection-site reactions occurred in 11 percent of patients in the adalimumab group (17 of 159), as compared with 10 percent of patients in the placebo group (17 of 166) (Table 27).

### Adalimumab Concentrations and Immunogenicity

The mean (± SD) adalimumab concentration at week 4 in the adalimumab group was 12.6 ± 6.0 µg/mL. None of 159 patients treated with adalimumab were positive for anti-adalimumab antibodies at week 4.

### Discussion

Induction therapy with adalimumab was superior to placebo for inducing remission and response in patients with moderate to severe Crohn's disease who had previously responded to infliximab and then became intolerant or lost response. Patients who received adalimumab were 3 times more likely to achieve remission and approximately 1.5 times more likely to achieve 100-point response and 70-point response at 4 weeks as compared with patients who received placebo. Patients who received adalimumab had significantly greater decreases in disease activity as measured by changes in mean CDAI scores, mean IBDQ total scores, and median C-reactive protein concentrations compared with patients who received placebo. Statistically significant responses in some measures (70-point response and mean CDAI score) were observed as early as week 1. Subgroup analyses demonstrated consistent benefit of adalimumab when the results were stratified for corticosteroid therapy, immunosuppressive therapy, baseline C-reactive protein concentration, prior loss of response to infliximab, and prior intolerance to infliximab.

The above study was the first randomized, double-blind, placebo-controlled trial in any immune-mediated disease in which TNF plays a central role to evaluate the efficacy of administering a second TNF inhibitor to patients who had failed a first TNF antagonist.

The types and frequencies of adverse events in this study were comparable to those previously reported for patients naïve to anti-TNF therapy. The overall rates of any type of injection-site reaction were 10 percent in the placebo group and 11 percent in the adalimumab group. Injection-site irritation and non-specific injection-site reactions were the most commonly reported of these reactions, none of which led to withdrawal. The rates of serious adverse events and serious infections in patients treated with adalimumab were similar to placebo. No patients developed opportunistic infections, lupus or lupus-like disease, neurologic diseases, lymphoma, or solid tumor malignancies; no patients died. It should be acknowledged that this was only a 4-week trial. In patients with rheumatoid arthritis treated with adalimumab, serious and opportunistic infections, lymphoma, demyelination, congestive heart failure, and lupus-like syndrome have all been reported previously. Rates of infections in placebo-controlled trials of adalimumab in patients with rheumatoid arthritis were 1.0 per patient-year in adalimumab-treated patients and 0.9 per patient-year in placebo-treated patients. Serious infections occurred at a rate of 0.04 per patient-year in adalimumab-treated patients and 0.02 per patient-year in placebo-treated patients. Pneumonia, tuberculosis, histoplasmosis, aspergillosis, and nocardia were all observed (Schiff et al. Safety analyses of adalimumab (HUMIRA®) in global clinical trials and US postmarketing surveillance of patients with rheumatoid arthritis. Ann Rheum Dis 2006;65(7):889-94)

None of the 159 patients who received adalimumab in this study developed antibodies against adalimumab. These results are similar to those reported previously for adalimumab in patients with Crohn's disease in which the frequency of antibodies to adalimumab ranged from 0.4 to 3.6 percent (Hanauer et al. Gastroenterology 2006; 130:323-33; and Sandborn et al. Am J Gastroenterol 2005;100(9 Suppl):S311).

In conclusion, patients with moderate to severe Crohn's disease who had previously responded to infliximab and then lost response or became intolerant were more likely to be in remission at week 4 if they had received induction therapy with adalimumab than if they had received treatment with placebo. Patients with moderate to severe Crohn's disease who had previously responded to infliximab and then lost response or became intolerant were more likely to be in remission at week 4 if they had received adalimumab induction therapy at weeks 0 and 2 than if they had received placebo. Adalimumab induction therapy rapidly improved the health-related quality of life measured by the IBDQ in patients with moderately to severely active Crohn's disease who had lost response and/or had adverse reactions to infliximab. The differences were both statistically significant and clinically relevant.

### Example 7: Early Crohn's Disease Shows High Levels of Remission to Therapy with Adalimumab

The objective of this study was to investigate the large population of the placebo (PBO)-controlled, randomized trial for a relationship between disease duration and response to 12-month ADA therapy. The following describes a subanalysis of Study R.

### Methods

The study design is shown in Figure 2. Double-blind, placebo-controlled trial with a 4-week open-label induction period.

Of 854 pts enrolled in the study, 778 were randomized at Week (Wk) 4 to either PBO, 40 mg ADA every-other-week (EOW) or 40 mg weekly (W) through Wk 56. 499 were responders at Wk 4 (CDAI decrease by ≥70 points [CR-70]) and formed the primary efficacy population of randomized responders (RR). RR pts were divided into groups by disease duration: <2 years, 2 to <5 years, and ≥5 years (post-hoc). Remission (CDAI<150) was determined by disease duration groupings. Effect of disease duration on remission was studied by logistic regression model controlling for potential confounders: sex, age, baseline CRP, concomitant therapies (steroids, immunosuppression), treatment, and presence of fistulae.

Analysis of populations was stratified by clinical response (CDAI decrease ≥70 points from baseline (CR-70]) at Week 4. Not randomized: discontinued prior to Week 4. Randomized non-responders: did not,achieve CR-70 at Week 4. Randomized responders (primary analysis group): achieved CR-70 at Week 4.

Flare was defined as recurrence of very active disease (CDAI increase ≥70 points from Week 4 and a CDAI>220). Patients who flared at or after Week 12 in the randomized arm received open-label 40 mg eow. Patients who flared on open-label 40 mg eow received open-label 40 mg weekly. Patients who did not respond to open-label weekly were withdrawn from the study. Patients who did not meet the definition of flare, but were consistent non-responders (did not attain CR-70) at or after Week 12, also received open-label 40 mg eow

Inclusion criteria included the following:
- Moderately to severely active Crohn's disease (220≤CDAI≤450)
- Concomitant treatment with stable doses of aminosalicylates, corticosteroids and immunosuppressants (azathioprine, 6-MP, methotrexate) was permitted
- History of previous anti-TNF therapies, if any, was permissible only if:
   o Prior anti-TNF therapy had been discontinued ≥12 weeks prior to screening
   o Patient met any of the following criteria:
      ■ Responded and then stopped the agent, lost response, or became intolerant
      ■ Did not tolerate the anti-TNF agent

Endpoints in the study included the following:
o Major secondary endpoint
   o Clinical response (CDAI decrease by ≥70 and 100 points)
o Co-primary endpoints in patients with CR-70 response at Week 4
   (randomized responders)
   o Remission (CDAI<150) at Week 26
   o Remission (CDAI<150) at Week 56

### Results

At Week 4, of 854 patients who received induction doses of adalimumab: 216 patients (25%) achieved remission; 388 patients (45%) achieved CR-100; and 509 patients (60%) achieved CR-70. Analysis of patient populations found the following: Not randomized: N=76 (9%); Randomized non-responders: N=279 (33%); and Randomized responders: N=499 (58%). Baseline demographic characteristics of randomized responders were similar among treatment groups (see Table 28).

**Table 28: Baseline Demographics: Randomized Responders**

| Characteristics | | Placebo n = 170 | 40 mg eow n = 172 | 40 mg weekly n= 157 |
|---|---|---|---|---|
| Age, years* | | 37 | 36 | 37 |
| Weight, Kg* | | 70 | 70 | 70 |
| CRP, mg/dL* | | 2.46 | 2.24 | 2.38 |
| CDAI score* | | 321 | 316 | 313 |

| Previous/ Concomitant medications, n (%) | | | | |
|---|---|---|---|---|
| | Previous anti-TNF | 81 (48) | 86 (50) | 71 (45) |
| | Steroids | 69 (41) | 65 (38) | 76 (48) |
| | Immunosuppressants | 83 (49) | 78 (45) | 79 (50) |

| | | | | |
|---|---|---|---|---|
| *Mean Values cow, every other week. No statistical significance observed. | | | | |

No significant differences were noted in baseline demographics.

A significant influence of disease duration on remission rates was found. At Wk 56, remission rates for <2 years disease duration were 52% ADA EOW (p<0.05), 50% ADA W, vs 17% PBO; for≥5 years duration, rates were 33% ADA EOW (p<0.05), 38% ADA W (p<0.05), vs 11% PBO. The logistic regression model confirmed disease duration had a significant effect on remission when controlling for confounders (0.96 point estimate, 0.94-0.99 95% CI, p=0.002).

The percentages of randomized responders in clinical remission at Week 56 were significantly higher in adalimumab treatment groups vs. placebo, as shown in Table 29. A logistic regression model confirmed a benefit of early treatment with adalimumab for the long-term maintenance of remission when controlling for confounders (0.96 point estimate, 0.94-0.99, 95% CI, p=0.002).

**Table 29: Clinical Remission at Week 56: Randomized Responders**

| | | < 2 years | 2 to < 5 years | ≥ 5 years |
|---|---|---|---|---|
| % of Patients | Placebo | 17 (4/23) | 11 (4/36) | 11 (12/111) |
| | 40 mg eow | 52* (13/25) | 35* (9/26) | 33* (40/121) |
| | 40 mg weekly | 50 (7/14) | 52† (16/31) | 38† (42/112) |

| | | | | |
|---|---|---|---|---|
| *p<0.05 † p<0.001, both vs. placebo | | | | |

Adalimumab-treated patients maintained significantly greater rates of clinical response (CR-100) through Week 56 compared with placebo-treated patients, regardless of disease duration group, as described below in Table 30.

**Table 30: Clinical Response (CR-100) At Week 56: Randomized Responders.**

| | | < 2 years | 2 to < 5 years | ≥ 5 years |
|---|---|---|---|---|
| % of Patients | Placebo | 22 (5/23) | 17 (6/36) | 15 (17/111) |
| | 40 mg eow | 56* (14/25) | 42* (11/26) | 38† (46/121) |
| | 40 mg weekly | 50** (7/14) | 55† (17/31) | 46† (51/112) |

| | | | | |
|---|---|---|---|---|
| *p<0.05 **p<0.002 † p<0.001, all vs. placebo | | | | |

Adalimumab-treated patients maintained significantly greater rates of clinical response (CR-70) through Week 56 compared with placebo-treated patients, regardless of disease duration group. Results are described below in Table 31.

**Table 31: Clinical Response (CR-70) At Week 56: Randomized Responders.**

| | | < 2 years | 2 to < 5 years | ≥ 5 years |
|---|---|---|---|---|
| % of Patients | Placebo | 22 (5/23) | 19 (7/36) | 16 (18/111) |
| | 40 mg eow | 56* (14/25) | 42 (11/26) | 41† (49/121) |
| | 40 mg weekly | 50 (7/14) | 58** (18/31) | 46† (52/112) |

| | | | | |
|---|---|---|---|---|
| *p<0.05 **p<0.002 † p<0.001, all vs. placebo | | | | |

Induction and maintenance therapy with adalimumab was generally well-tolerated, with low rates of discontinuation due to any adverse event (AE) observed (Table 32). The incidence of AEs during the double-blind treatment period was similar for adalimumab and placebo groups. Serious adverse events (SAEs) were significantly fewer with adalimumab maintenance compared with placebo. The safety profile of adalimumab was consistent with previously reported studies in RA and Crohn's disease.

**Table 32: Adverse Events: All Adalimumab-Treated Patients**

| | | Post Randomization (Double-blind Period from Weeks 4-56) | | |
|---|---|---|---|---|
| Adverse Event, n (%) | 4-week OL N=854 | Placebo N = 26 1 | 40 mg eow N = 260 | 40 mg weekly n = 257 |
| Any AE | 508 (59.5) | 221 (85) | 231(89) | 220(86) |
| AEs leading to drug withdrawal | 54 (6) | 35 (13) | 18 (7)* | 12 (5)* |
| Infectious AE | 130 (15) | 96 (37) | 120 (46)* | 114 (44) |
| Any SAE | 45 (5) | 40 (15) | 24 (9)* | 21 (8)* |
| Infectious SAE | 10 (1) | 9 (3) | 7 (3) | 7 (3) |
| Deaths | 1 (0.1)^ | 0 (0) | 0 (0) | 0 (0) |

| | | | | |
|---|---|---|---|---|
| * p<0.05 vs. placebo. ^ Death after 2^{nd} dose of adalimumab due to a pulmonary embolus. | | | | |

### Conclusion

ADA therapy led to sustained remission in approximately half of CD RR pts on ADA with CD duration of <2 years. The large remission rates in early CD suggest a disease modification by early use of anti-TNF therapy with ADA.

Adalimumab maintained remission through Week 56 in patients with moderately to severely active Crohn's disease, regardless of disease duration. There was no significant difference between eow and weekly maintenance dosages. Maintenance of clinical remission and response through Week 56 was greater in adalimumab-treated patients with early Crohn's disease (disease duration <2 years). Long-term maintenance of remission rates in this study suggests that early disease modification with adalimumab would be beneficial to early Crohn's disease patients.

### EQUIVALENTS

Those skilled in the art will recognize, or be able to ascertain using no more than routine experimentation, many equivalents to the specific embodiments of the invention described herein. Such equivalents are intended to be encompassed by the following claims. The contents of all references, patents and published patent applications cited throughout this application are incorporated herein by reference.

In addition, the present invention further relates to the following items:
1. Use of a TNFα inhibitor in the manufacture of a medicament for the treatment of early Crohn's disease in a subject who has early Crohn's disease.
2. The use of item 1, wherein the subject has had Crohn's disease for less than 2 years.
3. A method of inducing and maintaining remission of Crohn's disease in a subject comprising
   administering an initial loading dose of a TNFα inhibitor to the subject at week 0,
   administering a second dose of the TNFα inhibitor to the subject, wherein the second dose is about half the dose amount of the loading dose, and
   administering at least one a maintenance dose to the subject, wherein the maintenance dose is about half the dose amount of the second dose,
   such that remission of Crohn's disease is induced and maintained.
4. The method of item3, wherein the initial dose is given in its entirety on one day or is divided over 2 days.
5. The method of item 3 or 4, wherein the second dose is administered to the subject about two weeks after the first dose.
6. The method of any one of items3-5, wherein the maintenance dose is administered to the subject about two weeks after the second dose.
7. The method of any one of items 3-6, wherein the maintenance dose is administered on a biweekly dosing regimen.
8. A method of determining the efficacy of a TNFα inhibitor for maintaining remission of Crohn's disease in a subject comprising
   determining a Crohn's Disease Activity Index (CDAI) score of a patient population having Crohn's disease and who was administered the TNFα inhibitor,
   wherein a CDAI score of less than 150 maintained in at least about 49% of the patient population indicates that the TNFα inhibitor is an effective TNFα inhibitor for the treatment of Crohn's disease in a subject.
9. The method of item 8, further comprising administering the effective TNFα inhibitor to a subject to maintain remission of Crohn's disease.
10. The method of item 8 or 9, wherein a CDAI score of less than 150 maintained in at least about 50% of the patient population indicates that the TNFα inhibitor is an effective TNFα inhibitor for the treatment of Crohn's disease in a subject.
11. The method of item 8 or 9, wherein a CDAI score of less than 150 maintained in at least about 60% of the patient population indicates that the TNFα inhibitor is an effective TNFα inhibitor for the treatment of Crohn's disease in a subject.
12. The method of item 8 or 9, wherein a CDAI score of less than 150 maintained in at least about 70% of the patient population indicates that the TNFα inhibitor is an effective TNFα inhibitor for the treatment of Crohn's disease in a subject.
13. The method of item8 or 9, wherein a CDAI score of less than 150 maintained in at least about 80% of the patient population indicates that the TNFα inhibitor is an effective TNFα inhibitor for the treatment of Crohn's disease in a subject.
14. The method of item 8 or 9, wherein a CDAI score of less than 150 maintained in at least about 90% of the patient population indicates that the TNFα inhibitor is an effective TNFα inhibitor for the treatment of Crohn's disease in a subject.
15. The method of item 8 or 9, wherein a CDAI score of less than 150 maintained in at least about 94% of the patient population indicates that the TNFα inhibitor is an effective TNFα inhibitor for the treatment of Crohn's disease in a subject.
16. A method of maintaining remission of Crohn's disease in a subject comprising administering an effective TNFα inhibitor to the subject such that remission of Crohn's disease is maintained, wherein the effective TNFα inhibitor was previously identified as maintaining a CDAI score of less than 150 in at least about 49% of a patient population having Crohn's disease.
17. The method of item 16, wherein the effective TNFa inhibitor was previously identified as maintaining a CDAI score of less than 150 in at least about 50% of a patient population having Crohn's disease.
18. The method of item 16, wherein the effective TNFα inhibitor was previously identified as maintaining a CDAI score of less than 150 in at least about 60% of a patient population having Crohn's disease.
19. The method of item 16, wherein the effective TNFα inhibitor was previously identified as maintaining a CDAI score of less than 150 in at least about 70% of a patient population having Crohn's disease.
20. The method of item 16, wherein the effective TNFα inhibitor was previously identified as maintaining a CDAI score of less than 150 in at least about 80% of a patient population having Crohn's disease.
21. The method of item 16, wherein the effective TNFα inhibitor was previously identified as maintaining a CDAI score of less than 150 in at least about 90% of a patient population having Crohn's disease.
22. The method of item 16, wherein the effective TNFα inhibitor was previously identified as maintaining a CDAI score of less than 150 in at least about 94% of a patient population having Crohn's disease.
23. A method of determining the efficacy of a TNFα inhibitor for achieving a clinical response in Crohn's disease in a subject comprising
   determining a Crohn's Disease Activity Index (CDAI) score of a patient population having Crohn's disease and who was administered the TNFα inhibitor,
   wherein a decrease of at least 100 in the CDAI score of at least about 47% of the patient population indicates that the TNFα inhibitor is an effective TNFα inhibitor for achieving a clinical response in Crohn's disease in a subject.
24. The method of item 23, further comprising administering the effective TNFα inhibitor to a subject to achieve a clinical response in Crohn's disease.
25. The method of item 23 or 24, wherein a decrease of at least 100 in the CDAI score of at least about 50% of the patient population indicates that the TNFα inhibitor is an effective TNFα inhibitor for achieving a clinical response in Crohn's disease in a subject.
26. The method of item23 or 24, wherein a decrease of at least 100 in the CDAI score of at least about 60% of the patient population indicates that the TNFα inhibitor is an effective TNFα inhibitor for achieving a clinical response in Crohn's disease in a subject.
27. The method of item 23 or 24, wherein a decrease of at least 100 in the CDAI score of at least about 70% of the patient population indicates that the TNFα inhibitor is an effective TNFα inhibitor for achieving a clinical response in Crohn's disease in a subject.
28. The method of item 23 or 24, wherein a decrease of at least 100 in the CDAI score of at least about 80% of the patient population indicates that the TNFα inhibitor is an effective TNFα inhibitor for achieving a clinical response in Crohn's disease in a subject.
29. The method of item 23 or 24, wherein a decrease of at least 100 in the CDAI score of at least about 90% of the patient population indicates that the TNFα inhibitor is an effective TNFα inhibitor for achieving a clinical response in Crohn's disease in a subject.
30. A method of achieving a clinical response in Crohn's disease in a subject comprising administering an effective TNFα inhibitor to the subject such that a clinical response in Crohn's disease is achieved, wherein the effective TNFα inhibitor was previously identified as decreasing a CDAI score by at least 100 in at least about 47% of a patient population having Crohn's disease.
31. The method of item 30, wherein the effective TNFα inhibitor was previously identified as decreasing a CDAI score by at least 100 in at least about 50% of a patient population having Crohn's disease.
32. The method of item 30, wherein the effective TNFα inhibitor was previously identified as decreasing a CDAI score by at least 100 in at least about 60% of a patient population having Crohn's disease.
33. The method of item 30, wherein the effective TNFα inhibitor was previously identified as decreasing a CDAI score by at least 100 in at least about 70% of a patient population having Crohn's disease.
34. The method of item 30, wherein the effective TNFα inhibitor was previously identified as decreasing a CDAI score by at least 100 in at least about 80% of a patient population having Crohn's disease.
35. The method of item 30, wherein the effective TNFα inhibitor was previously identified as decreasing a CDAI score by at least 100 in at least about 90% of a patient population having Crohn's disease.
36. A method of determining the efficacy of a human TNFα antibody, or antigen-binding portion thereof, for achieving a clinical response in Crohn's disease in a subject comprising
   determining a Crohn's Disease Activity Index (CDAI) score of a patient population having Crohn's disease and who was administered the human TNFα antibody, or antigen-binding portion thereof,
   wherein a decrease of at least 70 in the CDAI score of at least about 43% of the patient population indicates that the human TNFα antibody, or antigen-binding portion thereof, is an effective human TNFα antibody, or antigen-binding portion thereof,
   for achieving a clinical response in Crohn's disease in a subject.
37. The method of item 36, further comprising administering the effective human TNFα antibody, or antigen-binding portion thereof, to a subject.
38. The method of item 36 or 37, wherein a decrease of at least 70 in the CDAI score of at least about 50% of the patient population indicates that the human TNFα antibody, or antigen-binding portion thereof, is an effective human TNFα antibody, or antigen-binding portion thereof, for achieving a clinical response in Crohn's disease in a subject.
39. The method of item 36 or 37, wherein a decrease of at least 70 in the CDAI score of at least about 60% of the patient population indicates that the human TNFα antibody, or antigen-binding portion thereof, is an effective human TNFα antibody, or antigen-binding portion thereof, for achieving a clinical response in Crohn's disease in a subject.
40. The method of item 36 or 37, wherein a decrease of at least 70 in the CDAI score of at least about 70% of the patient population indicates that the human TNFα antibody, or antigen-binding portion thereof, is an effective human TNFα antibody, or antigen-binding portion thereof, for achieving a clinical response in Crohn's disease in a subject.
41. The method of item 36 or 37, wherein a decrease of at least 70 in the CDAI score of at least about 80% of the patient population indicates that the human TNFα antibody, or antigen-binding portion thereof, is an effective human TNFα antibody, or antigen-binding portion thereof, for achieving a clinical response in Crohn's disease in a subject.
42. The method of item 36 or 37, wherein a decrease of at least 70 in the CDAI score of at least about 90% of the patient population indicates that the human TNFα antibody, or antigen-binding portion thereof, is an effective human TNFα antibody, or antigen-binding portion thereof, for achieving a clinical response in Crohn's disease in a subject.
43. A method of determining the efficacy of a TNFα inhibitor to maintain remission of Crohn's disease in a subject comprising
   determining an Inflammatory Bowel Disease Questionnaire (IBDQ) score of a patient population having Crohn's disease who was administered the TNFα inhibitor,
   wherein an IBDQ score greater than 170 in at least about 74% of the patient population indicates that the TNFα inhibitor is an effective TNFα inhibitor for maintaining remission of Crohn's disease in a subject.
44. The method of item43, further comprising administering the effective TNFα inhibitor to a subject having Crohn's disease.
45. The method of item43 or 44, wherein a IBDQ score greater than 170 in at least about 80% of the patient population indicates that the TNFα inhibitor is an effective TNFα inhibitor for maintaining remission of Crohn's disease in a subject.
46. A method of maintaining remission of Crohn's disease in a subject comprising administering an effective amount of a TNFα inhibitor to the subject, such that remission of Crohn's disease is maintained, wherein the effective amount of the TNFα inhibitor was previously identified as maintaining an IBDQ score greater than 170 in at least about 74% of a patient population having Crohn's disease.
47. The method of item 46, wherein the effective amount of the TNFα inhibitor was previously identified as maintaining an IBDQ score greater than 170 in at least about 80% of a patient population having Crohn's disease.
48. A method of maintaining remission of Crohn's disease in a subject who has achieved remission of Crohn's disease comprising administering a maintenance dose of the TNFα inhibitor to the subject, wherein the maintenance dose provides a mean serum trough level of about 7 µg/mL of the TNFα inhibitor.
49. A method of inducing and maintaining remission of Crohn's disease in a subject in need thereof comprising
   administering a loading dose of a TNFα inhibitor to the subject, wherein the loading dose provides a mean serum TNFα inhibitor trough level of about 12 µg/mL, and
   administering a maintenance dose of the TNFα inhibitor to the subject to maintain remission of Crohn's disease, wherein the maintenance dose provides a mean serum trough level of about 7 µg/mL of the TNFα inhibitor.
50. The method of any one of items 1-49, wherein the TNFα inhibitor is a TNFα antibody, or antigen-binding portion thereof, or a TNFα fusion protein.
51. The method of item 50, wherein the TNFα fusion protein is etanercept.
52. The method of item 50, wherein the TNFα antibody, or antigen-binding portion thereof, is an antibody selected from the group consisting of a humanized antibody, a chimeric antibody, a human antibody, and a multivalent antibody.
53. The method of item 50, wherein the TNFα antibody, or antigen-binding portion thereof, is infliximab or golimumab.
54. The method of item 50, wherein the human TNFα antibody, or an antigen-binding portion thereof, dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of I x 10⁻⁷ M or less.
55. The method of item 50, wherein the human TNFα antibody, or an antigen-binding portion thereof, has the following characteristics:
   a) dissociates from human TNFα with a K_{off} rate constant of 1 x 10⁻³ s⁻¹ or less, as determined by surface plasmon resonance;
   b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
   c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.
56. The method of item 50, wherein the human TNFα antibody, or an antigen-binding portion thereof, comprises a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and comprises a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11.
57. The method of item 50, wherein the human TNFα antibody, or an antigen-binding portion thereof, comprises a light chain variable region (LCVR) comprising the amino acid sequence of SEQ ID NO: 1 and a heavy chain variable region (HCVR) comprising the amino acid sequence of SEQ ID NO: 2.
58. The method of item 50, wherein the human TNFα antibody, or an antigen-binding portion thereof, is adalimumab.
59. The method of any one of items 54-58, wherein the human TNFα antibody, or an antigen-binding portion thereof, was administered to the patient population on a maintenance therapy comprising a biweekly dosing regimen.
60. The method of item 59, wherein the human TNFα antibody, or an antigen-binding portion thereof, was administered in a dose of about 40 mg.
61. A method of achieving a clinical response in Crohn's disease in a subject comprising administering an effective human TNFα antibody, or antigen-binding portion thereof, to the subject such that a clinical response in Crohn's disease is achieved, wherein the effective human TNFα antibody, or antigen-binding portion thereof, was previously identified as decreasing a CDAI score by at least 70 in at least about 43% of a patient population having Crohn's disease.
62. The method of item61, wherein the effective human TNFα antibody, or antigen-binding portion thereof, was previously identified as decreasing a CDAI score by at least 70 in at least about 50% of a patient population having Crohn's disease.
63. The method of item61, wherein the effective human TNFα antibody, or antigen-binding portion thereof, was previously identified as decreasing a CDAI score by at least 70 in at least about 60% of a patient population having Crohn's disease.
64. The method of item61, wherein the effective human TNFα antibody, or antigen-binding portion thereof, was previously identified as decreasing a CDAI score by at least 70 in at least about 70% of a patient population having Crohn's disease.
65. The method of item61, wherein the effective human TNFα antibody, or antigen-binding portion thereof, was previously identified as decreasing a CDAI score by at least 70 in at least about 80% of a patient population having Crohn's disease.
66. The method of item 61, wherein the effective human TNFα antibody, or antigen-binding portion thereof, was previously identified as decreasing a CDAI score by at least 70 in at least about 90% of a patient population having Crohn's disease.
67. Use of a human TNFα antibody, or antigen binding portion thereof, in the manufacture of a medicament for maintaining remission of a Crohn's-related fistula in a subject.
68. Use of a human TNFα antibody, or antigen-binding portion thereof, in the manufacture of a medicament for maintaining remission of Crohn's disease in a subject who has achieved remission.
69. The use of item 68, wherein the medicament is for administration to the subject on a maintenance dose regimen.
70. The use of item 68 or 69, wherein the remission of Crohn's disease is a CDAI of < 150.
71. A method of inducing and maintaining remission of Crohn's disease in a subject comprising
   administering an initial loading dose of a human TNFα antibody or antigen-binding portion thereof, to the subject at week 0,
   administering a second dose of the human TNFα antibody or antigen-binding portion thereof, to the subject, wherein the second dose is about half the dose amount of the loading dose, and
   administering at least one maintenance dose of the human TNFα antibody or antigen-binding portion thereof, to the subject, wherein the maintenance dose is about half the dose amount of the second dose,
   such that remission of Crohn's disease is induced and maintained.
72. The method of item71, wherein the initial dose is given in its entirety on one day or is divided over 2 days.
73. The method of item 71 or 72, wherein the second dose is administered to the subject about two weeks after the first dose.
74. The method of any one of items71-73, wherein the maintenance dose is administered to the subject about two weeks after the second dose.
75. The method of any one of items71-74, wherein the maintenance dose is administered on a biweekly dosing regimen.
76. The method of any one of items61-75, wherein the human TNFα antibody, or an antigen-binding portion thereof, dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less.
77. The method of any one of items61-75, wherein the human TNFα antibody, or an antigen-binding portion thereof, has the following characteristics:
   a) dissociates from human TNFα with a K_{off} rate constant of 1 x 10⁻³ s⁻¹ or less, as determined by surface plasmon resonance;
   b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
   c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.
78. The method of any one of items61-75, wherein the human TNFα antibody, or an antigen-binding portion thereof, comprises a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and comprises a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11.
79. The method of any one of items 61-75, wherein the human TNFα antibody, or an antigen-binding portion thereof, comprises a light chain variable region (LCVR) comprising the amino acid sequence of SEQ ID NO: 1 and a heavy chain variable region (HCVR) comprising the amino acid sequence of SEQ ID NO: 2.
80. The method of any one of items61-75, wherein the human TNFα antibody, or an antigen-binding portion thereof, is adalimumab.
81. An article of manufacture comprising a TNFα inhibitor and a package insert, wherein the package insert indicates the recommended TNFα inhibitor dose regimen for adult patients with Crohn's disease is 160 mg at week 0, 80 mg at week 2, followed by 40 mg every other week beginning at week 4.
82. The article of manufacture of item82, wherein the package insert indicates that the week 0 dose can be administered as four injections in one day or as two injections per day for two consecutive days.
83. An article of manufacture comprising a TNFα inhibitor and a package insert, wherein the package insert indicates that in patients with Crohn's disease who have been administered the TNFα inhibitor, the loading dose on week 0 followed by a second dose on week 2 achieves serum adalimumab trough concentrations of approximately 12 µg/mL.
84. An article of manufacture comprising a TNFα inhibitor and a package insert, wherein the package insert indicates that in patients with Crohn's disease who have been administered the TNFα inhibitor, the mean steady-state trough levels of approximately 7 µg/mL were observed in Crohn's disease patients who received a maintenance dose of the TNFα inhibitor every other week.
85. The article of any one of items 81-84, wherein the TNFα inhibitor is a TNFα antibody, or an antigen-binding portion thereof.
86. The article of item 85, wherein the TNFα antibody, or an antigen-binding portion thereof, is human.
87. The article of item 86, wherein the human TNFα antibody, or an antigen-binding portion thereof, dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less.
88. The article of item 87, wherein the human TNFα antibody, or an antigen-binding portion thereof, comprises a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and comprises a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11.
89. The article of item 86, wherein the human TNFα antibody, or an antigen-binding portion thereof, is adalimumab.
90. An article of manufacture which comprising adalimumab and a package insert, wherein the package insert indicates that the adalimumab may be used to treat Crohn's disease in patients who have had an inadequate response to conventional therapy and/or who have lost response to or are intolerant to infliximab.
91. An article of manufacture comprising
   a) a packaging material;
   b) a human TNFα antibody, or antigen-binding portion thereof; and
   c) a package insert contained within the packaging material indicating that aminosalicylates, corticosteroids, and/or immunomodulatory agent, e.g., 6-mercaptopurine and azathioprine, may be continued during treatment with the TNFα antibody, or antigen-binding portion thereof.

## Claims

1. An isolated human anti-TNFα antibody, or antigen binding portion thereof, for use in maintaining remission of Crohn's disease in a subject,
wherein 40 mg of the anti-TNFα antibody, or antigen binding portion thereof, is administered to the subject for 26 weeks,
wherein the human anti-TNFα antibody, or antigen-binding portion thereof, is a human anti-TNFα antibody, or an antigen-binding portion thereof, that comprises a light chain variable region (LCVR) having a CDR1 domain comprising the amino acid sequence of SEQ ID NO: 7, a CDR2 domain comprising the amino acid sequence of SEQ ID NO: 5, and a CDR3 domain comprising the amino acid sequence of SEQ ID NO: 3; and a heavy chain variable region (HCVR) having a CDR1 domain comprising the amino acid sequence of SEQ ID NO: 8, a CDR2 domain comprising the amino acid sequence of SEQ ID NO: 6, and a CDR3 domain comprising the amino acid sequence of SEQ ID NO: 4.

2. The anti-TNFα antibody, or antigen binding portion thereof, of claim 1, wherein the anti-TNFα antibody, or antigen binding portion thereof, is administered to the subject for 56 weeks.

3. The anti-TNFα antibody, or antigen binding portion thereof, of claim 1 or 2, wherein the anti-TNFα antibody, or antigen binding portion thereof, is co-administered with a steroid that is discontinued over time.

4. An isolated human anti-TNFα antibody, or antigen binding portion thereof, for use in treating early Crohn's disease in a subject who has early Crohn's disease,
wherein the human anti-TNFα antibody, or antigen-binding portion thereof, is a human anti-TNFα antibody, or an antigen-binding portion thereof, that comprises a light chain variable region (LCVR) having a CDR1 domain comprising the amino acid sequence of SEQ ID NO: 7, a CDR2 domain comprising the amino acid sequence of SEQ ID NO: 5, and a CDR3 domain comprising the amino acid sequence of SEQ ID NO: 3; and a heavy chain variable region (HCVR) having a CDR1 domain comprising the amino acid sequence of SEQ ID NO: 8, a CDR2 domain comprising the amino acid sequence of SEQ ID NO: 6, and a CDR3 domain comprising the amino acid sequence of SEQ ID NO: 4.

5. The anti-TNFα antibody, or antigen binding portion thereof, of claim 4, where in the subject has had Crohn's disease for less than 2 years.

6. The anti-TNFα antibody, or antigen binding portion thereof, of claim 4, wherein the anti-TNFα antibody, or antigen binding portion thereof, is administered at a 80 mg dose.

7. The anti-TNFα antibody, or antigen binding portion thereof, of any one of claims 1-5, where in the anti-TNFα antibody, or antigen binding portion thereof, is administered at a 40 mg dose.

8. The anti-TNFα antibody, or antigen binding portion thereof, of any one of claims 1-7, wherein the anti-TNFα antibody, or antigen binding portion thereof, is administered subcutaneously every other week.

9. The anti-TNFα antibody, or antigen binding portion thereof, of any one of claims 1-8, wherein the anti-TNFα antibody, or antigen binding portion thereof, is a human anti-TNFα antibody, or an antigen-binding portion thereof, that comprises a light chain variable region (LCVR) comprising the amino acid sequence of SEQ ID NO: 1 and a heavy chain variable region (HCVR) comprising the amino acid sequence of SEQ ID NO: 2.

10. The anti-TNFα antibody, or antigen binding portion thereof, of any one of claims 1-8, wherein the anti-TNFα antibody is adalimumab, or an antigen binding portion thereof.
